# EUROPEAN PATENT APPLICATION

(11) **EP 2 460 828 A2**
(43) Date of publication of application: **06.06.2012**
(21) Application number: 12153900.1
(22) Date of filing: 09.11.2007
(51) Int. Cl.: C07K 16/22

(54) **Antibodies and diagnostics**

(30) Priority: 10.11.2006 US 857870 P
(62) Divisional of application: 07874263.2
(71) Applicant: UCB Pharma, S.A., 1070 Brussels (BE)
(72) Inventor: Latham, John, Seattle, WA 98119 (US); Winkler, David, Arlington, MA 02476 (US)
(74) Representative: Campbell, Patrick John Henry

(57) **Abstract**

Compositions and methods relating to sclerostin binding agents, such as antibodies and polypeptides capable of binding to sclerostin, are provided.

SEQUENCE LISTING

<110> UCB PHARMA S.A.

<120> Antibodies and Diagnostics

<130> 31173/40010

<160> 255

<170> FastSEQ for Windows Version 4.0

<210> 1
<211> 190
<212> PRT
<213> Homo sapiens

<400> 1
Gln Gly Trp Gln Ala Phe Lys Asn Asp Ala Thr Glu Ile Ile Pro Glu
 1               5                  10                  15
Leu Gly Glu Tyr Pro Glu Pro Pro Pro Glu Leu Glu Asn Asn Lys Thr
            20                  25                  30
Met Asn Arg Ala Glu Asn Gly Gly Arg Pro Pro His His Pro Phe Glu
        35                  40                  45
Thr Lys Asp Val Ser Glu Tyr Ser Cys Arg Glu Leu His Phe Thr Arg
    50                  55                  60
Tyr Val Thr Asp Gly Pro Cys Arg Ser Ala Lys Pro Val Thr Glu Leu
65                  70                  75                  80
Val Cys Ser Gly Gln Cys Gly Pro Ala Arg Leu Leu Pro Asn Ala Ile
                85                  90                  95
Gly Arg Gly Lys Trp Trp Arg Pro Ser Gly Pro Asp Phe Arg Cys Ile
            100                 105                 110
Pro Asp Arg Tyr Arg Ala Gln Arg Val Gln Leu Leu Cys Pro Gly Gly
        115                 120                 125
Glu Ala Pro Arg Ala Arg Lys Val Arg Leu Val Ala Ser Cys Lys Cys
    130                 135                 140
Lys Arg Leu Thr Arg Phe His Asn Gln Ser Glu Leu Lys Asp Phe Gly
145                 150                 155                 160
Thr Glu Ala Ala Arg Pro Gln Lys Gly Arg Lys Pro Arg Pro Arg Ala
                165                 170                 175
Arg Ser Ala Lys Ala Asn Gln Ala Glu Leu Glu Asn Ala Tyr
            180                 185                 190

<210> 2
<211> 134
<212> PRT
<213> Oryctolagus cuniculus

<400> 2
Met Asp Thr Arg Ala Pro Thr Gln Leu Leu Gly Leu Leu Leu Leu Trp
 1               5                  10                  15
Leu Pro Gly Ala Thr Phe Ala Ala Val Leu Thr Gln Thr Pro Ser Pro
            20                  25                  30
Val Ser Ala Gly Val Gly Gly Thr Val Thr Ile Lys Cys Gln Ser Ser
        35                  40                  45
Gln Arg Val Phe Asn Asn Asn Glu Leu Ser Trp Tyr Gln Gln Lys Pro
    50                  55                  60
Gly Gln Pro Pro Lys Arg Leu Ile Tyr Glu Ala Ser Thr Leu Ala Ser
65                  70                  75                  80
Gly Val Pro Asp Arg Phe Ser Gly Ser Gly Ser Gly Thr Gln Phe Thr
                85                  90                  95
Leu Thr Ile Ser Gly Val Gln Cys Asp Asp Ala Ala Thr Tyr Tyr Cys

                100                 105                 110
Leu Gly Gly Tyr Asp Asp Asp Gly Asp Asn Ala Phe Gly Gly Gly Thr
        115                 120                 125
Glu Val Val Val Lys Arg
    130

<210> 3
<211> 402
<212> DNA
<213> Oryctolagus cuniculus

<400> 3
atggacacga gggcccccac tcagctgctg gggctcctgc tgctctggct cccaggtgcc 60
acatttgcag ccgtgctgac ccagacacca tcgcccgtgt ctgcaggtgt gggaggcaca 120
gtcaccatca agtgccagtc cagtcagagg gtttttaata acaacgaatt atcctggtat 180
cagcagaaac cagggcagcc gcccaaacgc ctgatctatg aagcatccac actggcatct 240
ggggtcccag ataggttcag cggcagtgga tctgggacac agttcactct caccatcagc 300
ggcgtgcagt gtgacgatgc tgccacttac tactgtctag gcggttatga tgatgatggt 360
gataatgctt tcggcggagg gaccgaggtg gtggtcaaac gt                    402

<210> 4
<211> 143
<212> PRT
<213> Oryctolagus cuniculus

<400> 4
Met Glu Thr Gly Leu Arg Trp Leu Leu Leu Val Ala Val Leu Lys Gly
 1               5                  10                  15
Val Gln Cys Gln Glu Gln Leu Lys Glu Ser Gly Gly Arg Leu Val Thr
            20                  25                  30
Pro Gly Thr Pro Leu Thr Leu Thr Cys Thr Ala Ser Gly Phe Ser Leu
        35                  40                  45
Asn Asn Tyr Tyr Met Thr Trp Val Arg Gln Ala Pro Gly Lys Gly Leu
    50                  55                  60
Glu Trp Ile Gly Leu Ile Tyr Pro Asp Gly Ser Thr Phe Tyr Ala Asn
65                  70                  75                  80
Trp Ala Glu Gly Arg Phe Thr Ser Ser Lys Thr Ser Thr Thr Val Thr
                85                  90                  95
Leu Lys Met Thr Ser Pro Thr Thr Glu Asp Thr Ala Thr Tyr Phe Cys
            100                 105                 110
Ala Arg Glu Gly Gly Ala Gly Asp Asn Thr Gly Thr Glu Tyr Tyr Tyr
        115                 120                 125
Gly Val Asp Leu Trp Gly Gln Gly Thr Leu Val Thr Val Ser Ser
    130                 135                 140

<210> 5
<211> 429
<212> DNA
<213> Oryctolagus cuniculus

<400> 5
atggagactg ggctgcgctg gcttctcctg gtcgctgtgc tcaaaggtgt ccagtgtcag 60
gagcagctga aggagtccgg gggtcgcctg gtcacgcctg ggacacccct gacactcacc 120
tgcacagcct ctggattctc cctcaataat tactatatga cctgggtccg ccaggctcca 180
gggaagggac tggaatggat cggactcatt tatcctgatg gtagcacatt ctacgcgaac 240
tgggcggaag gccgattcac cagctccaag acctcgacca cggtgactct gaagatgacc 300
agtccgacaa ccgaggacac ggccacctat ttctgtgcca gagaaggtgg tgctggtgat 360
aatactggta ccgaatatta ctacggcgtg gacctctggg gccagggcac cctcgtcacc 420
gtctcgagc                                                         429

<210> 6

<211> 135
<212> PRT
<213> Oryctolagus cuniculus

<400> 6
Met Asp Met Arg Ala Pro Thr Gln Leu Leu Gly Leu Leu Leu Leu Trp
1               5                   10                  15
Leu Pro Gly Ala Thr Phe Ala Ile Val Met Thr Gln Thr Pro Ser Ser
            20                  25                  30
Val Ser Ala Ala Val Gly Asp Thr Val Thr Ile Asn Cys Gln Ala Ser
        35                  40                  45
Glu Asn Val Tyr Asp Lys Ser Ala Leu Ser Trp Tyr Gln Gln Lys Pro
    50                  55                  60
Gly Gln Pro Pro Lys Leu Leu Ile Tyr Leu Ala Ser Thr Leu Ala Ser
65                  70                  75                  80
Gly Val Pro Ser Arg Phe Lys Gly Ser Gly Ser Gly Thr Gln Phe Thr
                85                  90                  95
Leu Thr Ile Ser Asp Val Val Cys Ala Asp Ala Ala Thr Tyr Tyr Cys
            100                 105                 110
Ala Gly Tyr Lys Ser Ser Ile Ile Asp Gly Thr Ala Phe Gly Gly Gly
        115                 120                 125
Thr Asp Val Val Val Lys Gly
    130                 135

<210> 7
<211> 405
<212> DNA
<213> Oryctolagus cuniculus

<400> 7
atggacatga gggcccccac tcagctgctg gggctcctgc tgctctggct cccaggtgcc 60
acatttgcca tcgtgatgac ccagactcca tcttccgtgt ctgcagctgt gggagataca 120
gtcaccatca attgccaggc cagtgagaac gtttatgata aaagcgcctt atcctggtat 180
cagcagaaac cagggcagcc tcccaagctc ctgatctatc tggcatccac tctggcatct 240
ggggtcccat cgcggttcaa aggcagtgga tctgggacac agttcactct caccatcagc 300
gatgtggtgt gtgcggatgc tgccacttac tactgtgcag gatataaaag tagtattatt 360
gatggtactg ctttcggcgg agggaccgac gtggtggtca aagga                 405

<210> 8
<211> 132
<212> PRT
<213> Oryctolagus cuniculus

<400> 8
Met Glu Thr Gly Leu Arg Trp Leu Leu Leu Val Ala Val Leu Lys Gly
1               5                   10                  15
Val Gln Cys Gln Ser Val Glu Glu Ser Gly Gly Arg Leu Val Thr Pro
            20                  25                  30
Gly Thr Pro Leu Thr Leu Thr Cys Thr Val Ser Gly Phe Ser Leu Ser
        35                  40                  45
Asn Asn Ala Leu Ser Trp Val Arg Gln Ala Pro Gly Lys Gly Leu Glu
    50                  55                  60
Trp Ile Gly Ala Ile Gly Ala Gly Gly Asn Thr Tyr Tyr Ala Ser Trp
65                  70                  75                  80
Ala Lys Gly Arg Phe Thr Ile Ser Lys Thr Ser Ser Thr Thr Val Asp
                85                  90                  95
Leu Arg Met Thr Ser Pro Thr Thr Glu Asp Thr Ala Thr Tyr Phe Cys
            100                 105                 110
Ala Asn Gly Asp Leu Pro Gly Gly Ile Trp Gly Pro Gly Thr Leu Val
        115                 120                 125
Thr Val Ser Leu
    130

<210> 9
<211> 396
<212> DNA
<213> Oryctolagus cuniculus

<400> 9
atggagacag gcctgcgctg gcttctcctg gtcgctgtgc tcaaaggtgt ccagtgtcag 60
tcggtggagg agtccggggg tcgcctggtc acgcctggga cacccctgac actcacctgc 120
acagtctctg gattctccct cagtaacaat gcactgagct gggtccgcca ggctccaggg 180
aaggggctgg aatggatcgg ggccattggt gctggtggta acacatacta tgcgagctgg 240
gcgaaaggcc gattcaccat ctccaaaacc tcgtcgacca cggtggatct gagaatgacc 300
agtccgacaa ccgaggacac ggccacctat ttctgtgcca acggggacct gccaggtggc 360
atctggggcc caggcaccct ggtcaccgtc tccata 396

<210> 10
<211> 136
<212> PRT
<213> Oryctolagus cuniculus

<400> 10
Met Asp Ile Arg Ala Pro Thr Gln Leu Leu Gly Leu Leu Leu Leu Trp
1               5                   10                  15
Leu Pro Gly Ala Arg Cys Ala Asp Ile Val Met Thr Gln Thr Ala Ser
            20                  25                  30
Pro Val Ser Ala Ala Val Gly Gly Thr Val Thr Ile Asn Cys Gln Ala
        35                  40                  45
Ser Gln Ser Ile Ser Pro Ala Leu Ala Trp Tyr Gln Gln Lys Pro Gly
    50                  55                  60
Gln Arg Pro Lys Leu Leu Ile Tyr Asp Val Ser Lys Leu Ala Ser Gly
65                  70                  75                  80
Val Pro Ser Arg Phe Ser Gly Ser Gly Ser Gly Thr Gln Phe Thr Leu
                85                  90                  95
Thr Ile Ser Asp Leu Glu Cys Ala Asp Gly Ala Thr Tyr Tyr Cys Gln
            100                 105                 110
Ser Tyr Tyr Gly Ile Asn Ser Asn Ser Tyr Gly Asn Ile Phe Gly Gly
        115                 120                 125
Gly Thr Glu Val Val Val Lys Gly
    130                 135

<210> 11
<211> 408
<212> DNA
<213> Oryctolagus cuniculus

<400> 11
atggacataa gggcccccac tcagctgctg gggctcctac tgctctggct cccaggtgcc 60
agatgtgctg acattgtgat gacccagact gcatcccccg tgtctgcagc tgtgggaggc 120
acagtcacca tcaattgcca ggccagtcag agcattagcc ctgcattagc ctggtatcag 180
cagaaaccag ggcagcgtcc caagctcctg atctacgatg tatcgaaact ggcatctggg 240
gtcccatcgc ggttcagtgg cagtggatct gggacacagt tcactctcac catcagcgac 300
ctggagtgtg ccgatggtgc cacttactac tgtcaaagct attatggtat taatagtaat 360
agttatggta atattttcgg cggagggacc gaggtggtgg tcaaagga 408

<210> 12
<211> 130
<212> PRT
<213> Oryctolagus cuniculus

<400> 12
Met Glu Thr Gly Leu Arg Trp Leu Leu Leu Val Ala Val Leu Lys Gly
1               5                   10                  15

Val Gln Cys Gln Ser Val Glu Glu Ser Gly Gly Arg Leu Val Thr Pro
            20                  25                  30
Gly Thr Pro Leu Thr Leu Thr Cys Thr Val Ser Gly Phe Ser Leu Ser
        35                  40                  45
Ser Tyr Ala Met Gly Trp Phe Arg Gln Ala Pro Gly Lys Gly Leu Glu
    50                  55                  60
Trp Ile Gly Tyr Ile Tyr Gly Asn Tyr Asn Lys Tyr Tyr Ala Ser Trp
65                  70                  75                  80
Ala Lys Gly Arg Phe Thr Ile Ser Lys Thr Ser Thr Met Val Asp Leu
                85                  90                  95
Lys Met Thr Ser Leu Thr Thr Glu Asp Thr Ala Thr Tyr Phe Cys Ala
            100                 105                 110
Arg Gly Gly Ala Met Asp Val Trp Gly Gln Gly Thr Leu Val Thr Val
        115                 120                 125
Ser Ser
    130

<210> 13
<211> 390
<212> DNA
<213> Oryctolagus cuniculus

<400> 13
atggagacag gcctgcgctg gcttctcctg gtcgctgtgc tcaaaggtgt ccagtgtcag 60
tcggtggagg agtccggggg tcgcctggtc acgcctggga cacccctgac actcacctgc 120
acagtctctg gattctccct cagtagctat gcaatgggct ggttccgcca ggctccaggg 180
aaggggctgg agtggatcgg ctacatttat ggtaattata ataaatacta cgcgagctgg 240
gcgaaaggcc gattcaccat ctccaaaacc tcgaccatgg tggatctgaa gatgactagt 300
ctgacaaccg aggacacggc cacctatttc tgtgccagag ggggtgctat ggatgtctgg 360
ggccagggca ccctggtcac cgtctccagc                                  390

<210> 14
<211> 133
<212> PRT
<213> Oryctolagus cuniculus

<400> 14
Met Asp Thr Arg Ala Pro Thr Gln Leu Leu Gly Leu Leu Leu Leu Trp
 1               5                  10                  15
Leu Pro Gly Ala Arg Cys Ala Tyr Asp Met Thr Gln Thr Pro Ala Ser
            20                  25                  30
Val Glu Val Ala Val Gly Gly Thr Val Thr Ile Lys Cys Gln Ala Ser
        35                  40                  45
Glu Ser Ile Ser Thr Trp Leu Ala Trp Tyr Gln Gln Lys Pro Gly Gln
    50                  55                  60
Pro Pro Lys Leu Leu Ile Tyr Arg Ala Ser Thr Leu Ala Ser Gly Val
65                  70                  75                  80
Ser Ser Arg Phe Lys Gly Ser Gly Tyr Gly Thr Gln Phe Thr Leu Thr
                85                  90                  95
Ile Ser Gly Val Glu Cys Ala Asp Ala Ala Thr Tyr Tyr Cys Gln Gln
            100                 105                 110
Gly Trp Asn Ile Asn Asn Ile Asp Asn Ile Phe Gly Gly Gly Thr Glu
        115                 120                 125
Val Val Val Lys Gly
    130

<210> 15
<211> 399
<212> DNA
<213> Oryctolagus cuniculus

<400> 15
atggacacca gggcccccac tcagctgctg gggctcctgc tgctctggct cccaggtgcc 60
agatgcgcct atgatatgac ccagactcca gcctctgtgg aggtagctgt gggaggcaca 120
gtcaccatca agtgccaggc cagtgagagc attagcactt ggttagcctg gtatcagcag 180
aaaccagggc agcctcccaa gctcctgatc tacagggcat ccactctggc atctggggtc 240
tcatcgcggt tcaaaggcag tggatatggg acacagttca ctctcaccat cagcggcgtg 300
gagtgtgccg atgctgccac ttactactgt caacagggtt ggaatattaa taatattgat 360
aatattttcg gcggagggac cgaggtggtg gtcaaagga 399

<210> 16
<211> 137
<212> PRT
<213> Oryctolagus cuniculus

<400> 16
Met Glu Thr Gly Leu Arg Trp Leu Leu Leu Val Ala Val Leu Lys Gly
1               5                   10                  15
Val Gln Cys Gln Ser Val Glu Glu Ser Gly Gly Arg Leu Val Thr Pro
            20                  25                  30
Gly Thr Pro Leu Thr Leu Thr Cys Thr Val Ser Gly Phe Ser Leu Ser
        35                  40                  45
Ser Tyr Ser Met Tyr Trp Val Arg Gln Ala Pro Gly Lys Gly Leu Glu
    50                  55                  60
Tyr Ile Gly Phe Ile Leu Ser Ala Thr Ala Val Ser Tyr Ala Thr Trp
65                  70                  75                  80
Ala Lys Gly Arg Phe Thr Ile Ser Arg Thr Ser Thr Thr Val Asp Leu
                85                  90                  95
Lys Ile Thr Ser Pro Thr Thr Glu Asp Thr Ala Thr Tyr Phe Cys Ala
            100                 105                 110
Arg Asp Arg Asp Gly Gly Thr Thr Leu Asp Gly Phe Asp Pro Trp Gly
        115                 120                 125
Pro Gly Thr Leu Val Thr Val Ser Ser
    130                 135

<210> 17
<211> 411
<212> DNA
<213> Oryctolagus cuniculus

<400> 17
atggagacag gcctgcgctg gcttctcctg gtcgctgtgc tcaaaggtgt ccagtgtcag 60
tcggtggagg agtccggggg tcgcctggtc acgcctggga cacccctgac actcacctgc 120
acagtctctg gattctccct cagtagctat tcaatgtatt gggtccgcca ggctccaggg 180
aaggggctgg aatacatcgg atttattctt agtgctactg ccgtatccta cgcgacctgg 240
gcgaaaggcc gattcaccat ctccagaacc tcgaccacgg tggatctgaa aatcaccagt 300
ccgacaaccg aggacacggc cacctatttc tgtgccagag atagagatgg tggtactaca 360
ctagatggtt ttgatccctg gggcccaggc accctggtca ccgtctccag c 411

<210> 18
<211> 135
<212> PRT
<213> Oryctolagus cuniculus

<400> 18
Met Asp Ile Arg Ala Pro Thr Gln Leu Leu Gly Leu Leu Leu Leu Trp
1               5                   10                  15
Leu Pro Gly Ala Thr Phe Ala Gln Leu Leu Thr Gln Thr Pro Pro Ser
            20                  25                  30
Val Ser Ala Ala Val Gly Gly Thr Val Thr Ile Ser Cys Gln Ser Ser
        35                  40                  45
Gln Ser Val Val Asn Asn Asn Asn Leu Ala Trp Tyr Gln Gln Lys Pro
    50                  55                  60

Gly Gln Pro Pro Lys Leu Leu Ile Tyr Phe Ala Ser Thr Leu Ala Ser
65                  70                  75                  80
Gly Val Pro Ser Arg Phe Lys Gly Ser Gly Ser Gly Thr Gln Phe Thr
                85                  90                  95
Leu Thr Ile Ser Asp Val Gln Cys Asp Asp Ala Ala Thr Tyr Tyr Cys
            100                 105                 110
Gln Gly Thr Tyr Leu Ser Asp Asp Trp Ser Asp Ala Phe Gly Gly Gly
        115                 120                 125
Thr Glu Val Val Val Lys Gly
    130                 135

<210> 19
<211> 405
<212> DNA
<213> Oryctolagus cuniculus

<400> 19
atggacatca gggcccccac tcagctgctg gggctcctgc tgctctggct cccaggtgcc 60
acatttgccc aactgctgac ccagactcca ccctcggtgt ctgcagctgt gggaggcaca 120
gtcaccatca gttgccagtc cagtcagagt gttgttaata acaacaactt agcctggtat 180
cagcagaaac cagggcagcc tcccaagctc ctgatctatt ttgcctccac tctggcatct 240
ggggtcccat cgcggttcaa aggcagtgga tctgggacac agttcactct caccatcagc 300
gacgtgcagt gtgacgatgc tgccacttac tactgtcaag gcacttatct tagtgatgat 360
tggtccgatg ctttcggcgg agggaccgag gtggtggtca aagga                 405

<210> 20
<211> 136
<212> PRT
<213> Oryctolagus cuniculus

<400> 20
Met Glu Thr Gly Leu Arg Trp Leu Leu Leu Val Ala Val Leu Lys Gly
 1               5                  10                  15
Val Gln Cys Gln Ser Val Glu Glu Ser Gly Gly Arg Leu Val Thr Pro
            20                  25                  30
Gly Thr Pro Leu Thr Leu Thr Cys Lys Val Ser Gly Phe Ser Leu Ser
        35                  40                  45
Ser Ser Val Met Asn Trp Val Arg Gln Ala Pro Gly Lys Gly Leu Glu
    50                  55                  60
Trp Ile Gly Ala Ile Trp Ser Gly Gly Tyr Thr Tyr Tyr Ala Thr Trp
65                  70                  75                  80
Ala Lys Gly Arg Phe Thr Ile Ser Arg Thr Ser Thr Thr Val Asp Leu
                85                  90                  95
Lys Met Thr Ser Leu Thr Ala Ala Asp Thr Ala Thr Tyr Phe Cys Ala
            100                 105                 110
Arg Gly Gln Phe Gly Ala Ser Gly Gly Gly Asp Val Leu Trp Gly Pro
        115                 120                 125
Gly Thr Leu Val Thr Val Ser Ser
    130                 135

<210> 21
<211> 408
<212> DNA
<213> Oryctolagus cuniculus

<400> 21
atggagacag gcctgcgctg gcttctcctg gtcgctgtgc tcaaaggtgt ccagtgtcag 60
tcggtggagg agtccggggg tcgcctggtc acgcctggga cacccctgac actcacctgc 120
aaagtctctg gattctccct cagttcctct gtaatgaact gggtccgcca ggctccaggg 180
aaggggctgg agtggatcgg agccatttgg agtggtggtt acacatacta cgcgacctgg 240
gcaaaaggcc gattcaccat ctccagaacc tcgaccacgg tggatctgaa aatgaccagt 300

ctgacagccg cggacacggc cacctacttc tgtgccagag gacaatttgg tgctagtggt 360
ggtggtgatg ttttgtgggg cccgggcacc ctggtcaccg tctccagc 408

<210> 22
<211> 136
<212> PRT
<213> Oryctolagus cuniculus

<400> 22
Met Asp Ile Arg Ala Pro Thr Gln Leu Leu Gly Leu Leu Leu Leu Trp
1               5                   10                  15
Leu Pro Gly Ala Thr Phe Ala Gln Val Leu Thr Gln Thr Pro Ser Ser
            20                  25                  30
Val Ser Ala Ala Val Gly Gly Thr Val Thr Ile Ser Cys Gln Ser Ser
        35                  40                  45
Pro Ser Val Asp Asn Asn Asn Leu Leu Ser Trp Phe Gln Gln Lys Pro
    50                  55                  60
Gly Gln Pro Pro Lys Leu Leu Ile Tyr Asp Ala Ser Thr Leu Ala Ser
65                  70                  75                  80
Gly Val Pro Ser Arg Phe Glu Gly Ser Gly Ser Gly Thr Gln Phe Thr
                85                  90                  95
Leu Ala Ile Ser Gly Val Gln Cys Asp Asp Ala Ala Thr Tyr Tyr Cys
            100                 105                 110
Val Ala Gly Tyr Gly Lys Arg Ser Arg Asp Ile Arg Val Phe Gly Gly
        115                 120                 125
Gly Thr Gly Val Val Val Lys Gly
    130                 135

<210> 23
<211> 408
<212> DNA
<213> Oryctolagus cuniculus

<400> 23
atggacataa gggcccccac tcagctgctg gggctcctgc tgctctggct cccaggtgcc 60
acatttgccc aagtgctgac ccagactcca tcctccgtgt ctgcagctgt gggaggcaca 120
gtcaccatca gttgccagtc cagtccgagt gttgataata acaacctctt atcctggttt 180
cagcagaaac cagggcagcc tcccaagctc ctgatctatg atgcttccac tctggcatct 240
ggggtcccat cgcggttcga aggcagtgga tctgggacac aattcactct cgccatcagc 300
ggcgttcagt gtgacgatgc tgccacttac tactgtgtag ccggttatgg taaaaggagt 360
cgagatatac gagttttcgg cggagggacc ggggtggtgg tcaaagga 408

<210> 24
<211> 130
<212> PRT
<213> Oryctolagus cuniculus

<400> 24
Met Glu Thr Gly Leu Arg Trp Leu Leu Leu Val Ala Val Leu Lys Gly
1               5                   10                  15
Val Gln Cys Gln Ser Val Glu Glu Ser Gly Gly Arg Leu Val Thr Pro
            20                  25                  30
Gly Thr Pro Leu Thr Leu Thr Cys Thr Val Ser Gly Phe Asp Ile Ser
        35                  40                  45
Ser His Asn Met Gln Trp Val Arg Gln Ala Pro Gly Lys Gly Leu Glu
    50                  55                  60
Trp Ile Gly Ile Ile Tyr Pro Ser Asn Asn Ala Tyr Tyr Ser Asn Trp
65                  70                  75                  80
Ala Lys Gly Arg Phe Thr Ile Ser Lys Thr Ser Thr Thr Met Ser Leu
                85                  90                  95
Gln Met Asn Ser Leu Thr Thr Glu Asp Thr Ala Thr Tyr Phe Cys Ala
            100                 105                 110

Arg Asp Ile Asn Ser Ala Ile Trp Gly Pro Gly Thr Leu Val Thr Val
        115                 120                 125
Ser Ser
    130

<210> 25
<211> 390
<212> DNA
<213> Oryctolagus cuniculus

<400> 25
   atggagacag gcctgcgctg gcttctcctg gtcgctgtgc tcaaaggtgt ccagtgtcag 60
   tcggtggagg agtccggggg tcgcctggtc acgcctggga cacccctgac actcacctgc 120
   accgtctctg gattcgacat cagtagccac aatatgcaat gggtccgcca ggctccaggg 180
   aaggggctgg aatggatcgg aatcatttat cctagtaata atgcatacta ttccaactgg 240
   gcgaaaggcc gattcaccat ctccaaaact tcgaccacga tgtctctgca aatgaacagt 300
   ctgacaaccg aggacacggc cacctatttc tgcgccagag acataaatag tgctatttgg 360
   ggcccaggca ccctggtcac cgtctccagc                                  390

<210> 26
<211> 134
<212> PRT
<213> Oryctolagus cuniculus

<400> 26
   Met Asp Thr Arg Ala Pro Thr Gln Leu Leu Gly Leu Leu Leu Leu Trp
   1 5 10 15
   Leu Pro Gly Val Ile Cys Ala Glu Val Ala Met Thr Gln Thr Pro Ser
   20 25 30
   Ser Val Ser Ala Ala Val Gly Gly Thr Val Thr Ile Asp Cys Gln Ala
   35 40 45
   Ser Glu Ser Ile Tyr Ser Asn Leu Ala Trp Tyr Gln Gln Lys Pro Gly
   50 55 60
   Gln Pro Pro Lys Leu Leu Ile Tyr Asp Ala Ser Asp Leu Ala Ser Gly
   65 70 75 80
   Val Pro Ser Arg Phe Lys Gly Ser Gly Ser Gly Thr Glu Tyr Thr Leu
   85 90 95
   Thr Ile Ser Asp Leu Glu Cys Ala Asp Ala Ala Thr Tyr Tyr Cys Gln
   100 105 110
   Cys Ser Trp Gly Gly Ser Thr Tyr Gly Phe Ala Phe Gly Gly Gly Thr
   115 120 125
   Glu Val Val Val Lys Arg
   130

<210> 27
<211> 402
<212> DNA
<213> Oryctolagus cuniculus

<400> 27
atggacacga gggcccccac tcagctgctg gggctcctgc tgctctggct cccaggtgtc 60
atatgtgccg aagtagcgat gacccagact ccatcctccg tgtctgcagc tgtgggaggc 120
acagtcacca tcgattgcca ggccagtgag agcattta[?]a gcaatttagc ctggtatcag 180
cagaaaccag ggcagcctcc caagctcctg atctatga[?]g catccgatct ggcatctggg 240
gtcccatcgc ggttcaaagg cagtggatct gggacagagt acactctcac catcagcgac 300
ctggagtgtg ccgatgctgc cacttactac tgtcaatg[?]a gttgggtgg tagtacttat 360
ggttttgctt tcggcggagg gaccgaggtg gtggtcaaac gt                    402

<210> 28
<211> 135
<212> PRT

<213> Oryctolagus cuniculus

<400> 28
Met Glu Thr Gly Leu Arg Trp Leu Leu Leu Val Ala Val Leu Lys Gly
1               5                   10                  15
Val Gln Cys Gln Ser Leu Glu Glu Ser Gly Gly Arg Leu Val Thr Pro
            20                  25                  30
Gly Thr Pro Leu Thr Leu Thr Cys Thr Val Ser Gly Ile Asp Leu Ser
        35                  40                  45
Ile Tyr Val Met Thr Trp Val Arg Gln Ala Pro Gly Lys Gly Leu Glu
    50                  55                  60
Trp Ile Gly Ser Ile Asp Ala Asp Asp Ser Ala Tyr Tyr Ala Thr Trp
65                  70                  75                  80
Ala Thr Ser Arg Ser Thr Ile Ser Arg Thr Ser Thr Thr Val Ala Leu
                85                  90                  95
Ser Ile Thr Ser Pro Thr Thr Glu Asp Thr Ala Thr Tyr Phe Cys Ala
            100                 105                 110
Arg Gly Leu Tyr Ala Asn Gly Gly Pro Phe Thr Leu Trp Gly Pro Gly
        115                 120                 125
Thr Leu Val Thr Val Ser Ser
    130                 135


<210> 29
<211> 405
<212> DNA
<213> Oryctolagus cuniculus

<400> 29
atggagactg ggctgcgctg gcttctcctg gtcgctgtgc tcaaaggtgt ccagtgtcag 60
tcgctggagg agtccggggg tcgcctggta acgcctggga cacccctgac actcacctgc 120
acagtctctg gaatcgacct cagtatctat gtaatgactt gggtccgcca ggctccaggg 180
aaggggctgg aatggatcgg aagcatcgat gctgacgata gcgcatacta cgcgacctgg 240
gcgacaagcc gatccaccat ctccagaacc tcgaccacgg tggctctgag catcaccagt 300
ccgacaaccg aggacacggc gacctatttc tgtgccaggg gactatatgc taatggtggt 360
ccctttacct tatggggccc aggcaccctc gtcaccgtct cgagc                405

<210> 30
<211> 135
<212> PRT
<213> Oryctolagus cuniculus

<400> 30
Met Asp Met Arg Ala Pro Thr Gln Leu Leu Gly Leu Leu Leu Leu Trp
1               5                   10                  15
Leu Pro Gly Ala Thr Phe Ala Ala Val Leu Thr Gln Thr Pro Ser Pro
            20                  25                  30
Val Ser Ala Ala Val Gly Gly Thr Val Thr Ile Lys Cys Gln Ser Ser
        35                  40                  45
Pro Ser Val Tyr Asn Arg Asn Gln Leu Ser Trp Phe Gln Gln Lys Ala
    50                  55                  60
Gly Gln Pro Pro Lys Leu Leu Ile Phe Thr Ala Ser Thr Leu Ala Ser
65                  70                  75                  80
Gly Val Pro Ser Arg Phe Lys Gly Ser Gly Ser Gly Thr Asp Phe Thr
                85                  90                  95
Leu Thr Ile Ser Asp Leu Glu Cys Ala Asp Ala Ala Thr Tyr Tyr Cys
            100                 105                 110
Gln Gly Tyr Tyr Asn Thr Gly Ser Asp Thr Tyr Ala Phe Gly Gly Gly
        115                 120                 125
Thr Glu Val Val Val Lys Gly
    130                 135

<210> 31
<211> 405
<212> DNA
<213> Oryctolagus cuniculus

<400> 31
atggacatga gggcccccac tcagctgctg gggctcctgc tgctctggct cccaggtgcc 60
acatttgccg ccgtgctgac ccagactcca tctcccgtgt ctgcagctgt gggaggcaca 120
gtcaccatca agtgccagtc cagtccgagt gtttataatc gcaaccaatt atcctggttt 180
cagcagaaag cagggcagcc tcccaagctc ctgatcttta ctgcgtccac tctggcatct 240
ggggtcccat cgcggttcaa aggcagtgga tctgggacag atttcactct caccatcagc 300
gacctggagt gtgccgatgc tgccacttac tactgtcaag gctattataa tactggtagt 360
gatacgtatg ctttcggcgg agggaccgag gtggtggtca aagga 405

<210> 32
<211> 133
<212> PRT
<213> Oryctolagus cuniculus

<400> 32
Met Glu Thr Gly Leu Arg Trp Leu Leu Leu Val Ala Val Leu Lys Gly
1               5                   10                  15
Val Gln Cys Gln Ser Val Glu Glu Ser Gly Gly Arg Leu Val Thr Pro
            20                  25                  30
Gly Thr Pro Leu Thr Leu Thr Cys Thr Val Ser Gly Phe Ser Leu Asn
        35                  40                  45
Asn Tyr Asp Met Ser Trp Val Arg Gln Ala Pro Gly Lys Gly Leu Glu
    50                  55                  60
Tyr Ile Gly Phe Ile Asn Thr Val Gly Tyr Ala Tyr Tyr Ala Ser Trp
65                  70                  75                  80
Ala Lys Gly Arg Phe Thr Ile Ser Arg Thr Ser Thr Thr Val Asp Leu
                85                  90                  95
Lys Met Thr Ser Leu Thr Thr Glu Asp Thr Ala Thr Tyr Phe Cys Ala
            100                 105                 110
Ser Leu Asp Asn Tyr Tyr Thr Trp Gly Ile Trp Gly Pro Gly Thr Leu
        115                 120                 125
Val Thr Val Ser Leu
    130

<210> 33
<211> 399
<212> DNA
<213> Oryctolagus cuniculus

<400> 33
atggagacag gcctgcgctg gcttctcctg gtcgctgtgc tcaaaggtgt ccagtgtcag 60
tcggtggagg agtccggggg tcgcctggtc acgcctggga cacccctgac actcacctgc 120
acagtctctg gattctccct caataactac gacatgagct gggtccgcca ggctccaggg 180
aaggggctgg aatatatcgg attcattaat actgttggtt acgcatacta cgcgagctgg 240
gcaaaaggcc gattcaccat ctccagaacc tcgaccacgg tggatctgaa aatgaccagt 300
ctgacaaccg aggacacggc cacctatttc tgtgccagcc ttgataatta ctatacttgg 360
ggcatctggg gcccaggcac cctggtcacc gtttccata 399

<210> 34
<211> 134
<212> PRT
<213> Oryctolagus cuniculus

<400> 34
Met Asp Thr Arg Ala Pro Thr Gln Leu Leu Gly Leu Leu Leu Leu Trp
1               5                   10                  15
Leu Pro Gly Ala Arg Cys Ala Asp Ile Val Met Thr Gln Thr Pro Gly

            20                  25                  30
Ser Val Ser Ala Ala Val Gly Gly Thr Val Thr Ile Asn Cys Gln Ala
        35                  40                  45
Ala Glu Asp Ile Tyr Ser Ser Leu Ala Trp Tyr Gln Gln Lys Ser Gly
    50                  55                  60
Gln Pro Pro Lys Leu Leu Ile Tyr Ala Ala Ser Ile Leu Ala Ser Gly
65                  70                  75                  80
Ala Pro Ser Arg Phe Ser Gly Ser Gly Ser Gly Thr Glu Tyr Thr Leu
                85                  90                  95
Thr Ile Ser Gly Val Gln Cys Asp Asp Ala Ala Thr Tyr Tyr Cys Gln
            100                 105                 110
Thr Asn Tyr Gly Ile Ser Ser Tyr Gly Ala Ala Phe Gly Gly Gly Thr
        115                 120                 125
Glu Val Val Val Lys Gly
    130

<210> 35
<211> 402
<212> DNA
<213> Oryctolagus cuniculus

<400> 35
atggacacga gggcccccac tcagctgctg gggctcctgc tgctctggct cccaggtgcc 60
agatgtgccg acatcgtgat gacccagact ccaggctccg tgtctgcagc tgtgggaggc 120
acagtcacca tcaattgcca ggccgctgag gacatttata gctctttggc ctggtatcag 180
cagaaatcag ggcagcctcc caagctcctg atctatgctg catctattct ggcatctggg 240
gccccatcgc ggttcagtgg cagtggatct gggacagagt acactctcac catcagcggc 300
gtgcagtgtg acgatgctgc cacttactac tgtcaaacca attatggtat cagtagttat 360
ggtgcggctt tcggcggagg gaccgaggtg gtggtcaaag ga 402

<210> 36
<211> 134
<212> PRT
<213> Oryctolagus cuniculus

<400> 36
Met Glu Thr Gly Leu Arg Trp Leu Leu Leu Val Ala Val Leu Lys Gly
1               5                   10                  15
Val Gln Cys Gln Ser Val Glu Glu Ser Gly Gly Arg Leu Val Thr Pro
            20                  25                  30
Gly Thr Pro Leu Thr Leu Thr Cys Thr Val Ser Gly Phe Ser Leu Ser
        35                  40                  45
Asn Lys Pro Ile Thr Trp Val Arg Gln Ala Pro Gly Glu Gly Leu Glu
    50                  55                  60
Tyr Ile Gly Trp Ile Ser Thr Thr Gly Ser Ala Tyr Tyr Ala Ser Trp
65                  70                  75                  80
Ala Lys Gly Arg Phe Thr Ile Ser Lys Thr Ser Thr Thr Val Asp Leu
                85                  90                  95
Lys Ile Thr Ser Pro Thr Thr Glu Asp Thr Ala Thr Tyr Phe Cys Ala
            100                 105                 110
Arg Tyr Ser Ser Asp Tyr Gly His His Asp Leu Trp Gly Pro Gly Thr
        115                 120                 125
Leu Val Thr Val Ser Ser
    130

<210> 37
<211> 402
<212> DNA
<213> Oryctolagus cuniculus

<400> 37

atggagacag gcctgcgctg gcttctcctg gtcgctgtgc tcaaaggtgt ccagtgtcag 60
tcggtggagg agtccggggg tcgcctggtc acgcctggga cacccctgac actcacctgc 120
accgtctctg ggttctccct cagtaacaag ccaataacat gggtccgcca ggctccaggg 180
gaggggctgg aatacatcgg atggattagt actactggta gcgcatacta cgcgagctgg 240
gcgaaaggcc gattcaccat ctccaaaacc tcgaccacgg tggatctgaa aatcaccagt 300
ccgacaaccg aggacacggc cacctatttc tgtgccagat atagtagtga ttatggacat 360
catgacttgt ggggcccagg caccctggtc accgtctcca gc 402

<210> 38
<211> 132
<212> PRT
<213> Oryctolagus cuniculus

<400> 38
Met Asp Thr Arg Ala Pro Thr Gln Leu Leu Gly Leu Leu Leu Leu Trp
1               5                   10                  15
Leu Pro Gly Ala Thr Phe Ala Gln Val Leu Thr Gln Thr Pro Ser Pro
            20                  25                  30
Val Ser Ala Ala Leu Gly Gly Ser Val Thr Ile Asn Cys Gln Ala Ser
        35                  40                  45
Gln Ser Val Tyr Arg Asp Tyr Leu Ser Trp Phe Gln Gln Lys Pro Gly
    50                  55                  60
Gln Pro Pro Lys Leu Leu Ile Asn Gly Ala Ser Asn Leu Ala Ser Gly
65                  70                  75                  80
Val Pro Ser Arg Phe Ser Gly Ser Gly Ser Gly Thr Gln Phe Thr Leu
                85                  90                  95
Thr Ile Ser Asp Val Gln Cys Asp Asp Ala Ala Thr Tyr Tyr Cys Leu
            100                 105                 110
Gly Gly Phe Ser Gly Asn Ile Asn Thr Phe Gly Gly Gly Thr Glu Val
        115                 120                 125
Val Val Lys Gly
    130

<210> 39
<211> 396
<212> DNA
<213> Oryctolagus cuniculus

<400> 39
atggacacca gggcccccac tcagctgctg gggctcctgc tgctctggct cccaggtgcc 60
acatttgcgc aagtgctgac ccagactcca tcacccgtgt ctgcagctct gggaggctca 120
gtcaccatca attgccaggc cagtcagagt gtttatagag attacttatc ctggtttcag 180
cagaaaccag gtcagcctcc caagctcctg atcaatggtg catccaatct ggcatctggg 240
gtcccatcgc ggttcagcgg cagtggatct gggacacagt tcactctcac catcagcgac 300
gtgcagtgtg acgatgctgc cacttactac tgtctaggcg gttttagtgg taatatcaat 360
actttcggcg gagggaccga ggtggtggtc aaggga 396

<210> 40
<211> 131
<212> PRT
<213> Oryctolagus cuniculus

<400> 40
Met Glu Thr Gly Leu Arg Trp Leu Leu Leu Val Ala Val Leu Lys Gly
1               5                   10                  15
Val Gln Cys Gln Ser Leu Glu Glu Ser Gly Gly Arg Leu Val Thr Pro
            20                  25                  30
Gly Gly Ser Leu Thr Leu Thr Cys Thr Val Ser Gly Ile Asp Leu Asn
        35                  40                  45
Asn Tyr His Met Cys Trp Val Arg Gln Ala Pro Gly Lys Gly Leu Lys
    50                  55                  60
Tyr Ile Gly Ile Ile Ser Asn Thr Gly Tyr Thr Tyr Tyr Ser Ser Trp

65                  70                  75                  80
Ala Lys Gly Arg Phe Thr Ile Ser Lys Thr Ser Ser Thr Thr Val Asp
                85                  90                  95
Leu Lys Met Thr Ser Leu Thr Thr Glu Asp Thr Ala Thr Tyr Phe Cys
            100                 105                 110
Ala Gly Asp Arg Leu Ala Asn Leu Trp Gly Pro Gly Thr Leu Val Thr
        115                 120                 125
Val Ser Ser
    130

<210> 41
<211> 393
<212> DNA
<213> Oryctolagus cuniculus

<400> 41
atggagacag gcctgcgctg gcttctcctg gtcgctgtgc tcaaaggtgt ccagtgtcag 60
tcgctggagg agtccggggg tcgcctggta acgcctggag gatccctgac actcacctgt 120
acagtctctg gaatcgacct caataactac cacatgtgct gggtccgcca ggctccaggg 180
aaggggctga aatacatcgg aatcattagt aatactggtt acacatacta ctcgagctgg 240
gcgaaaggcc gattcaccat ctccaaaacc tcgtcgacca cggtggatct gaaaatgacc 300
agtctgacaa ccgaggacac ggccacctat ttctgtgctg gagaccggct tgctaacttg 360
tggggcccag gcaccctggt cactgtctcc agc                              393

<210> 42
<211> 136
<212> PRT
<213> Oryctolagus cuniculus

<400> 42
Met Asp Thr Arg Ala Pro Thr Gln Leu Leu Gly Leu Leu Leu Leu Trp
 1               5                  10                  15
Leu Pro Gly Ala Arg Cys Ala Asp Val Val Met Thr Gln Thr Pro Ala
            20                  25                  30
Ser Val Glu Ala Ala Val Gly Gly Thr Val Thr Ile Lys Cys Gln Ala
        35                  40                  45
Ser Gln Ser Ile Ser Thr Tyr Leu Lys Trp Tyr Gln Gln Lys Pro Gly
    50                  55                  60
Gln Arg Pro Lys Arg Leu Ile Tyr Ser Ala Ser Thr Leu Thr Ser Gly
65                  70                  75                  80
Val Pro Ser Arg Phe Lys Gly Ser Gly Ser Gly Thr Asp Phe Thr Leu
                85                  90                  95
Thr Ile Ser Asp Leu Glu Cys Gly Asp Ala Ala Thr Tyr Tyr Cys Gln
            100                 105                 110
Ser Asn Ala Gly Ser Ser Ser Ser Ser Cys Gly Tyr Ala Phe Gly Gly
        115                 120                 125
Gly Thr Glu Val Val Val Lys Gly
    130                 135

<210> 43
<211> 408
<212> DNA
<213> Oryctolagus cuniculus

<400> 43
atggacacga gggcccccac tcagctgctg gggctcctgc tgctctggct cccaggtgcc 60
agatgtgccg acgtcgtgat gacccagact ccagcctccg tggaggcagc tgtgggaggc 120
acagtcacca tcaagtgcca ggccagtcag agcattagta cctacttaaa gtggtatcag 180
cagaaaccag gacagcgtcc caagcgcctg atctattctg catccactct gacatctggg 240
gtcccatcgc ggttcaaagg cagtggatct gggacagatt tcactctcac catcagcgac 300
ctggagtgtg gcgatgctgc cacttactac tgtcaaagca atgctggtag tagtagtagt 360

agttgtggtt atgctttcgg cggagggacc gaggtggtgg tcaaagga                408

<210> 44
<211> 127
<212> PRT
<213> Oryctolagus cuniculus

<400> 44
Met Glu Thr Gly Leu Arg Trp Leu Leu Leu Val Ala Val Leu Lys Gly
1               5                   10                  15
Val Gln Cys Gln Ser Val Glu Glu Ser Gly Gly Arg Leu Val Thr Pro
            20                  25                  30
Gly Thr Pro Leu Thr Leu Thr Cys Thr Val Ser Gly Ile Asp Leu Ser
        35                  40                  45
Tyr Tyr Gly Met Gly Trp Val Arg Gln Ala Pro Gly Lys Gly Leu Glu
    50                  55                  60
Tyr Ile Gly Ile Ile Ser Gly Ile Gly Asn Thr Tyr Tyr Pro Thr Trp
65                  70                  75                  80
Ala Lys Gly Arg Phe Thr Ile Ser Lys Thr Pro Thr Thr Val Asp Leu
                85                  90                  95
Arg Ile Thr Ser Pro Thr Thr Glu Asp Thr Ala Thr Tyr Phe Cys Ala
            100                 105                 110
Thr Gly Asp Phe Trp Gly Pro Gly Thr Leu Val Thr Val Ser Ser
        115                 120                 125

<210> 45
<211> 381
<212> DNA
<213> Oryctolagus cuniculus

<400> 45
atggagacag gcctgcgctg gcttctcctg gtcgctgtgc tcaaaggtgt ccagtgtcag 60
tcggtggagg agtccggggg tcgcctggtc acgcctggga cacccctgac actcacctgc 120
acagtctctg gaattgacct cagttactat gggatgggct gggtccgcca ggctccaggg 180
aaggggctgg aatacatcgg gatcattagt ggtattggta atacatacta tccgacctgg 240
gcgaaaggcc gattcaccat ctccaaaacc ccgaccacgg tggatctaag gatcaccagt 300
ccgacaaccg aggacacggc cacctatttc tgtgccactg gggacttctg gggcccaggc 360
accctggtca ccgtctccag c                                           381

<210> 46
<211> 136
<212> PRT
<213> Oryctolagus cuniculus

<400> 46
Met Asp Thr Arg Ala Pro Thr Gln Leu Leu Gly Leu Leu Leu Leu Trp
1               5                   10                  15
Leu Pro Gly Ala Arg Cys Ala Asp Val Val Met Thr Gln Thr Pro Ala
            20                  25                  30
Ser Val Ser Glu Pro Val Gly Gly Thr Val Thr Ile Lys Cys Gln Ala
        35                  40                  45
Ser Gln Ser Ile Ser Arg Tyr Leu Lys Trp Tyr Gln Gln Lys Pro Gly
    50                  55                  60
Gln Arg Pro Lys Arg Leu Ile Tyr Ser Ala Ser Thr Leu Thr Ser Gly
65                  70                  75                  80
Val Pro Ser Arg Phe Lys Gly Ser Gly Ser Gly Thr Asp Phe Thr Leu
                85                  90                  95
Thr Ile Ser Asp Leu Glu Cys Ala Asp Ala Ala Thr Tyr Tyr Cys Gln
            100                 105                 110
Ser Asn Ala Gly Ser Ser Ser Ser Ser Cys Gly Tyr Ala Phe Gly Gly
        115                 120                 125
Gly Thr Glu Val Val Val Lys Gly

130                 135

<210> 47
<211> 408
<212> DNA
<213> Oryctolagus cuniculus

<400> 47
atggacacga gggcccccac tcagctgctg gggctcctgc tgctctggct cccaggtgcc 60
agatgtgccg acgtcgtgat gacccagact ccagcctccg tgtctgaacc tgtgggaggc 120
acagtcacca tcaagtgcca ggccagtcag agcattagta ggtacttaaa ttggtatcag 180
cagaaaccag ggcagcgtcc caagcgcctg atctattctg catccactct ggcatctggg 240
gtcccatcgc ggttcaaagg cagtggatct gggacagatt tcactctcac catcagcgac 300
ctggagtgtg ccgatgctgc cacttactac tgtcaaagca atgctggtag tagcagtagt 360
agttgtggtt atgctttcgg cggagggacc gaggtggtgg tcaaagga             408

<210> 48
<211> 127
<212> PRT
<213> Oryctolagus cuniculus

<400> 48
Met Glu Thr Gly Leu Arg Trp Leu Leu Leu Val Ala Val Leu Lys Gly
1               5                   10                  15
Val Gln Cys Gln Ser Val Glu Glu Ser Gly Gly Arg Leu Val Thr Pro
            20                  25                  30
Gly Thr Pro Leu Thr Leu Thr Cys Thr Val Ser Gly Val Asp Leu Ser
        35                  40                  45
Tyr Tyr Gly Met Gly Trp Val Arg Gln Ala Pro Gly Lys Gly Leu Glu
    50                  55                  60
Tyr Ile Gly Ile Ile Ser Gly Ser Gly Asn Thr Tyr Tyr Ala Ser Trp
65                  70                  75                  80
Ala Lys Gly Arg Phe Thr Ile Ser Lys Thr Ser Thr Thr Val Asp Leu
                85                  90                  95
Lys Ile Thr Ser Pro Thr Thr Glu Asp Thr Ala Thr Tyr Phe Cys Ala
            100                 105                 110
Ser Gly Asp Phe Trp Gly Pro Gly Thr Leu Val Thr Val Ser Ser
        115                 120                 125

<210> 49
<211> 381
<212> DNA
<213> Oryctolagus cuniculus

<400> 49
atggagacag gcctgcgctg gcttctcctg gtcgctgtgc tcaaaggtgt ccagtgtcag 60
tcggtggagg agtccggggg tcgcctggtc acgcctggga cacccctgac actcacctgc 120
acagtctctg gagtcgacct cagttactat ggaatgggct gggtccgcca ggctccaggg 180
aaggggctgg aatacatcgg gatcattagt ggtagtggta acacatacta cgcgagctgg 240
gcgaaaggcc gattcaccat ctccaaaacc tcgaccacgg tggatctgaa aatcaccagt 300
ccgacaaccg aggacacggc cacctatttc tgtgccagtg gggacttctg gggcccaggc 360
accctggtca ccgtctccag c                                          381

<210> 50
<211> 134
<212> PRT
<213> Oryctolagus cuniculus

<400> 50
Met Asp Thr Arg Ala Pro Thr Gln Leu Leu Gly Leu Leu Leu Leu Trp
1               5                   10                  15

Leu Pro Gly Ala Arg Cys Ala Leu Val Met Thr Gln Thr Ala Ser Pro
            20                  25                  30
Val Ser Ala Ala Val Gly Gly Thr Val Thr Ile Ser Cys Gln Ser Ser
        35                  40                  45
Gln Ser Val Tyr Ser Asn Tyr Leu Ser Trp Phe Gln Gln Lys Pro Gly
    50                  55                  60
Gln Pro Pro Lys Leu Leu Ile Tyr Val Ala Ser Ser Leu Ala Ser Gly
65                  70                  75                  80
Val Pro Ser Arg Phe Lys Gly Ser Gly Ser Gly Thr Gln Phe Thr Leu
                85                  90                  95
Thr Ile Ser Asp Leu Glu Cys Asp Asp Ala Ala Thr Tyr Tyr Cys Gly
            100                 105                 110
Gly Phe Gln Lys Tyr Ile Asp Asp Gly Gly Ala Phe Gly Gly Gly Thr
        115                 120                 125
Glu Val Val Val Lys Gly
    130

<210> 51
<211> 402
<212> DNA
<213> Oryctolagus cuniculus

<400> 51
atggacacga gggcccccac tcagctgctg gggctcctgc tgctctggct cccaggtgcc 60
agatgtgccc ttgtgatgac ccagactgca tcccccgtgt ctgcagctgt gggaggcaca 120
gtcaccatca gttgccagtc cagtcagagt gtttatagta actacttatc ctggtttcag 180
cagaaaccag ggcagcctcc caagctcctg atctatgttg catccagtct ggcatctggg 240
gtcccatcgc gattcaaagg cagtggatct gggacacagt tcactctcac catcagtgac 300
ctggagtgtg acgatgctgc cacttactac tgtggaggct ttcagaaata tattgatgat 360
ggggggcctt tcggcggagg gaccgaggtg gtggtcaaag ga                    402

<210> 52
<211> 131
<212> PRT
<213> Oryctolagus cuniculus

<400> 52
Met Glu Thr Gly Leu Arg Trp Leu Leu Leu Val Ala Val Leu Lys Gly
 1               5                  10                  15
Val Gln Cys Gln Ser Val Glu Glu Ser Gly Gly Arg Leu Val Thr Pro
            20                  25                  30
Gly Gly Ser Leu Thr Leu Thr Cys Thr Val Ser Gly Ile Asp Leu Ser
        35                  40                  45
Thr Tyr Ala Met Ser Trp Val Arg Gln Ala Pro Gly Lys Gly Leu Glu
    50                  55                  60
Trp Ile Gly Ile Met Ser Ser Ser Gly Ser Ala Tyr Tyr Ala Ser Trp
65                  70                  75                  80
Ala Lys Gly Arg Phe Thr Ile Ser Lys Thr Ala Ser Thr Thr Val Asp
                85                  90                  95
Leu Lys Met Thr Ser Leu Thr Ile Glu Asp Thr Ala Thr Tyr Phe Cys
            100                 105                 110
Ala Arg Ser Ser Ser Phe Gly Leu Trp Gly Pro Gly Thr Leu Val Thr
        115                 120                 125
Val Ser Ser
    130

<210> 53
<211> 393
<212> DNA
<213> Oryctolagus cuniculus

<400> 53
atggagacag gcctgcgctg gcttctcctg gtcgctgtgc tcaaaggtgt ccagtgtcag 60
tcggtggagg agtccggggg tcgcctggtc acgcctggag gatccctgac acttacctgc 120
acagtctctg gaatcgacct cagtacctat gcaatgagtt gggtccgcca ggctccaggg 180
aaggggctgg aatggatcgg aatcatgagt agtagtggta gcgcatacta cgcgagctgg 240
gcgaaaggcc gattcaccat ctccaaaacc gcgtcgacca cggtggatct gaaaatgacc 300
agtctgacaa tcgaggacac ggccacctat ttctgtgcca gaagttctag ttttggattg 360
tggggcccag gcaccctggt caccgtctcc agc 393

<210> 54
<211> 106
<212> PRT
<213> Mus musculus

<400> 54
Thr Asp Ala Ala Pro Thr Val Ser Ile Phe Pro Pro Ser Ser Glu Gln
1               5                   10                  15
Leu Thr Ser Gly Gly Ala Ser Val Val Cys Phe Leu Asn Asn Phe Tyr
            20                  25                  30
Pro Lys Asp Ile Asn Val Lys Trp Lys Ile Asp Gly Ser Glu Arg Gln
        35                  40                  45
Asn Gly Val Leu Asn Ser Trp Thr Asp Gln Asp Ser Lys Asp Ser Thr
    50                  55                  60
Tyr Ser Met Ser Ser Thr Leu Thr Leu Thr Lys Asp Glu Tyr Glu Arg
65                  70                  75                  80
His Asn Ser Tyr Thr Cys Glu Ala Thr His Lys Thr Ser Thr Ser Pro
                85                  90                  95
Ile Val Lys Ser Phe Asn Arg Asn Glu Cys
            100                 105

<210> 55
<211> 324
<212> PRT
<213> Mus musculus

<400> 55
Ala Lys Thr Thr Pro Pro Ser Val Tyr Pro Leu Ala Pro Gly Ser Ala
1               5                   10                  15
Ala Gln Thr Asn Ser Met Val Thr Leu Gly Cys Leu Val Lys Gly Tyr
            20                  25                  30
Phe Pro Glu Pro Val Thr Val Thr Trp Asn Ser Gly Ser Leu Ser Ser
        35                  40                  45
Gly Val His Thr Phe Pro Ala Val Leu Gln Ser Asp Leu Tyr Thr Leu
    50                  55                  60
Ser Ser Ser Val Thr Val Pro Ser Ser Thr Trp Pro Ser Glu Thr Val
65                  70                  75                  80
Thr Cys Asn Val Ala His Pro Ala Ser Ser Thr Lys Val Asp Lys Lys
                85                  90                  95
Ile Val Pro Arg Asp Cys Gly Cys Lys Pro Cys Ile Cys Thr Val Pro
            100                 105                 110
Glu Val Ser Ser Val Phe Ile Phe Pro Pro Lys Pro Lys Asp Val Leu
        115                 120                 125
Thr Ile Thr Leu Thr Pro Lys Val Thr Cys Val Val Val Asp Ile Ser
    130                 135                 140
Lys Asp Asp Pro Glu Val Gln Phe Ser Trp Phe Val Asp Asp Val Glu
145                 150                 155                 160
Val His Thr Ala Gln Thr Gln Pro Arg Glu Glu Gln Phe Asn Ser Thr
                165                 170                 175
Phe Arg Ser Val Ser Glu Leu Pro Ile Met His Gln Asp Trp Leu Asn
            180                 185                 190
Gly Lys Glu Phe Lys Cys Arg Val Asn Ser Ala Ala Phe Pro Ala Pro
        195                 200                 205

Ile Glu Lys Thr Ile Ser Lys Thr Lys Gly Arg Pro Lys Ala Pro Gln
    210                 215                 220
Val Tyr Thr Ile Pro Pro Pro Lys Glu Gln Met Ala Lys Asp Lys Val
225                 230                 235                 240
Ser Leu Thr Cys Met Ile Thr Asp Phe Phe Pro Glu Asp Ile Thr Val
                245                 250                 255
Glu Trp Gln Trp Asn Gly Gln Pro Ala Glu Asn Tyr Lys Asn Thr Gln
            260                 265                 270
Pro Ile Met Asp Thr Asp Gly Ser Tyr Phe Val Tyr Ser Lys Leu Asn
        275                 280                 285
Val Gln Lys Ser Asn Trp Glu Ala Gly Asn Thr Phe Thr Cys Ser Val
    290                 295                 300
Leu His Glu Gly Leu His Asn His His Thr Glu Lys Ser Leu Ser His
305                 310                 315                 320
Ser Pro Gly Lys

<210> 56
<211> 130
<212> PRT
<213> Mus musculus

<400> 56
Met Asp Phe Gln Val Gln Ile Phe Ser Phe Met Leu Ile Ser Val Thr
 1               5                  10                  15
Val Ile Leu Ser Ser Gly Glu Ile Val Leu Thr Gln Ser Pro Ala Leu
            20                  25                  30
Met Ala Ala Ser Pro Gly Glu Lys Val Thr Ile Ala Cys Ser Val Ser
        35                  40                  45
Ser Ser Ile Ser Ser Thr Asn Leu His Trp Ser Gln Gln Lys Ser Gly
    50                  55                  60
Thr Ser Pro Lys Leu Trp Ile Tyr Gly Thr Ser Asn Leu Ala Ser Gly
65                  70                  75                  80
Val Pro Val Arg Phe Ser Gly Ser Gly Ser Gly Thr Ser Tyr Ser Leu
                85                  90                  95
Thr Ile Ser Ser Met Glu Ala Glu Asp Ala Ala Thr Tyr Tyr Cys Gln
            100                 105                 110
Gln Trp Ser Thr Thr Tyr Thr Phe Gly Ser Gly Thr Lys Leu Glu Leu
        115                 120                 125
Lys Arg
    130

<210> 57
<211> 387
<212> DNA
<213> Mus musculus

<400> 57
atggattttc aagtgcagat tttcagcttc atgctaatca gtgtcacagt catattgtcc 60
agtggagaaa ttgtgctcac ccagtctcca gcactcatgg ctgcatctcc aggggagaag 120
gtcaccatcg cctgcagtgt cagctcaagt ataagttcca ccaacttaca ctggtcccag 180
cagaagtcag gaacctcccc caaactctgg atttatggca catccaacct tgcttctgga 240
gtccctgttc gcttcagtgg cagtggatct gggacctctt attctctcac aatcagcagc 300
atggaggctg aagatgctgc cacctattac tgtcaacagt ggagtactac gtatacgttc 360
ggatcgggga ccaagctgga gctgaaa                                     387

<210> 58
<211> 141
<212> PRT
<213> Mus musculus

<400> 58
Met Gly Trp Asn Trp Ile Ile Phe Phe Leu Met Ala Val Val Thr Gly
1               5                   10                  15
Val Asn Ser Glu Val Gln Leu Gln Gln Ser Gly Ala Asp Leu Val Lys
            20                  25                  30
Pro Gly Ala Ser Val Lys Leu Ser Cys Thr Ala Ser Gly Phe Asn Ile
        35                  40                  45
Lys Asp Tyr Tyr Ile His Trp Val Lys Gln Arg Pro Ala Gln Gly Leu
    50                  55                  60
Glu Trp Ile Gly Arg Ile Asp Pro Asp Asn Gly Glu Ser Thr Tyr Val
65                  70                  75                  80
Pro Lys Phe Gln Gly Lys Ala Thr Ile Thr Ala Asp Thr Ser Ser Asn
                85                  90                  95
Thr Ala Tyr Leu Gln Leu Arg Ser Leu Thr Ser Glu Asp Thr Ala Ile
            100                 105                 110
Tyr Tyr Cys Gly Arg Glu Gly Leu Asp Tyr Gly Asp Tyr Tyr Ala Val
        115                 120                 125
Asp Tyr Trp Gly Gln Gly Thr Ser Val Thr Val Ser Ser
    130                 135                 140

<210> 59
<211> 420
<212> DNA
<213> Mus musculus

<400> 59
atgggatgga actggatcat cttcttcctg atggcagtgg ttacaggggt caattcagag 60
gtgcagttgc agcagtctgg ggcagacctt gtgaagccag gggcctcagt caagttgtcc 120
tgcacagctt ctggcttcaa cattaaagac tactatatac actgggtgaa gcagaggcct 180
gcacagggcc tggagtggat tggaaggatt gatcctgata atggtgaaag tacatatgtc 240
ccgaagttcc agggcaaggc cactataaca gcagacacat catccaacac agcctaccta 300
caactcagaa gcctgacatc tgaggacact gccatctatt attgtgggag agaggggctc 360
gactatggtg actactatgc tgtggactac tggggtcaag gaacctcagt cactgtctcg 420

<210> 60
<211> 132
<212> PRT
<213> Mus musculus

<400> 60
Met Glu Ser Asp Thr Leu Leu Leu Trp Val Leu Leu Leu Trp Val Pro
1               5                   10                  15
Gly Ser Thr Gly Lys Ile Val Leu Thr Gln Ser Pro Ala Ser Leu Ala
            20                  25                  30
Val Ser Leu Arg Gln Arg Ala Thr Ile Ser Cys Arg Ala Ser Glu Ser
        35                  40                  45
Val Asp Ser Tyr Gly Asn Ser Phe Met His Trp Tyr Gln Gln Lys Pro
    50                  55                  60
Gly Gln Pro Pro Lys Leu Leu Ile Tyr Arg Ala Ser Asn Leu Glu Ser
65                  70                  75                  80
Gly Val Pro Ala Arg Phe Ser Gly Ser Gly Ser Arg Thr Asp Phe Thr
                85                  90                  95
Leu Thr Ile Asp Pro Val Glu Ala Asp Asp Ala Ala Thr Tyr Tyr Cys
            100                 105                 110
Gln Gln Asn Tyr Glu Asp Pro Leu Thr Phe Gly Ala Gly Thr Lys Leu
        115                 120                 125
Glu Leu Lys Arg
    130

<210> 61

<211> 393
<212> DNA
<213> Mus musculus

<400> 61
atggagtcag acacactcct gctatgggtg ctactgctct gggttccagg ttccacaggt 60
aaaattgtac tgacccaatc tccagcttct ttggctgtgt ctctaaggca gagggccacc 120
atatcctgca gagccagtga aagtgttgat agttatggca atagttttat gcactggtac 180
cagcagaaac caggacagcc acccaaactc ctcatctatc gtgcatccaa cctagaatct 240
ggggtccctg ccaggttcag tggcagtggg tctaggacag acttcaccct caccattgat 300
cctgtggagg ctgatgatgc tgcaacctat tactgtcagc aaaattatga ggatccgctc 360
acgttcggtg ctgggaccaa gttggagctg aaa 393

<210> 62
<211> 145
<212> PRT
<213> Mus musculus

<400> 62
Met Gly Trp Pro Trp Val Phe Ile Phe Leu Leu Ser Val Thr Ala Gly
1               5                   10                  15
Val His Ser Gln Val Gln Leu Gln Gln Ser Gly Ala Glu Leu Val Arg
            20                  25                  30
Pro Gly Thr Ser Val Lys Val Ser Cys Lys Ala Ser Gly Tyr Ala Phe
        35                  40                  45
Thr Asn Tyr Leu Ile Glu Trp Ile Lys Gln Arg Pro Gly Gln Gly Leu
    50                  55                  60
Glu Trp Ile Gly Val Ile Asn Pro Gly Ser Gly Ile Ile Asn Tyr Asn
65                  70                  75                  80
Glu Lys Phe Lys Ile Lys Ala Thr Leu Thr Ala Asp Lys Ser Ser Ser
                85                  90                  95
Thr Ala Tyr Met Gln Leu Ser Ser Leu Thr Ser Asp Asp Ser Ala Val
            100                 105                 110
Tyr Phe Cys Ala Arg Asp Trp Asp Thr Phe Tyr Ser Tyr Glu Arg Glu
        115                 120                 125
Val Tyr Ala Met Asp Tyr Trp Gly Gln Gly Thr Ser Val Thr Val Ser
    130                 135                 140
Ser
145

<210> 63
<211> 432
<212> DNA
<213> Mus musculus

<400> 63
atgggatggc cctgggtctt tatatttctc ctgtcagtaa ctgcaggtgt tcactcccag 60
gtccagctgc agcagtctgg agctgagctg gtaaggcctg ggacctcagt gaaggtgtcc 120
tgcaaggcct ctggatacgc cttcactaat tacttgatag agtggataaa gcagaggcct 180
ggacagggcc ttgagtggat tggagtgatt aatcctggaa gcggtattat taattacaat 240
gagaagttca aaatcaaggc aacactgact gcagacaaat cctccagcac tgcctacatg 300
cagctcagca gcctgacatc tgatgattct gcggtctatt tctgtgcaag agactgggat 360
actttctata gttacgagag ggaggtctat gctatggact actggggtca agggacctca 420
gtcaccgtct cg 432

<210> 64
<211> 128
<212> PRT
<213> Mus musculus

<400> 64
Met Glu Ser Gln Thr Leu Val Leu Val Phe Leu Leu Phe Trp Ile Pro

1                   5                   10                  15
Ala Ser Arg Gly Asp Ile Leu Leu Thr Gln Ser Pro Ala Ile Leu Ser
            20                  25                  30
Val Ser Pro Gly Glu Arg Val Ser Phe Ser Cys Arg Ala Ser Gln Ser
        35                  40                  45
Ile Gly Thr Asn Ile His Trp Tyr Gln Gln Arg Thr Asp Gly Ser Pro
    50                  55                  60
Arg Leu Leu Ile Lys Phe Ala Ser Glu Ser Ile Ser Gly Ile Pro Ser
65                  70                  75                  80
Arg Phe Ser Gly Ser Gly Ser Gly Thr Asp Phe Thr Leu Ser Ile Asn
                85                  90                  95
Ser Val Glu Ser Glu Asp Ile Ala Asp Tyr Tyr Cys Gln Gln Ser Asn
            100                 105                 110
Ser Trp Pro Leu Thr Phe Gly Ala Gly Thr Lys Leu Glu Leu Lys Arg
        115                 120                 125

<210> 65
<211> 381
<212> DNA
<213> Mus musculus

<400> 65
atggaatcac agactctggt ccttgtattt ttgcttttct ggattccagc ctccagaggt 60
gacatcttgc tgactcagtc tccagccatc ctgtctgtga gtccaggaga aagagtcagt 120
ttctcctgca gggccagtca gagcattggc acaaacatac actggtatca gcaaagaaca 180
gatggttctc caaggcttct cataaagttt gcttctgagt ctatctctgg gatcccttcc 240
aggtttagtg gcagtggctc agggacagat tttactctta gcatcaacag tgtggagtct 300
gaagatattg cagattatta ctgtcaacaa agtaatagct ggccactcac gttcggtgct 360
gggaccaagc tggaactgaa a                                           381

<210> 66
<211> 137
<212> PRT
<213> Mus musculus

<400> 66
Met Asp Trp Ser Trp Ile Ile Phe Phe Leu Met Ala Val Val Ile Gly
1                   5                   10                  15
Ile Asn Ser Glu Val Gln Leu Gln Gln Ser Gly Ala Glu Leu Val Arg
            20                  25                  30
Ser Gly Ala Ser Val Lys Leu Ser Cys Thr Ala Ser Gly Phe Asn Ile
        35                  40                  45
Glu Asp Tyr Tyr Val His Trp Val Lys Gln Arg Pro Glu Gln Gly Leu
    50                  55                  60
Glu Trp Ile Gly Trp Ile Asp Pro Glu Asn Gly Asp Thr Glu Tyr Ala
65                  70                  75                  80
Pro Lys Phe Gln Gly Lys Ala Thr Met Thr Ala Asp Thr Ser Ser Asn
                85                  90                  95
Thr Ala Tyr Leu Gln Leu Ser Ser Leu Thr Ser Glu Asp Thr Ala Val
            100                 105                 110
Tyr Tyr Cys Asn Gly Gly Tyr Gly Asn Phe Tyr Phe Asp Tyr Trp Gly
        115                 120                 125
Gln Gly Thr Thr Val Thr Val Ser Ser
    130                 135

<210> 67
<211> 408
<212> DNA
<213> Mus musculus

<400> 67

atggattgga gctggatcat cttcttcctg atggcagtgg ttataggaat caattcagag 60
gttcagctgc agcagtctgg ggcagagctt gtgaggtcag gggcctcagt caagttgtcc 120
tgcacagctt ctggcttcaa cattgaagac tactatgtgc actgggtgaa gcagaggcct 180
gaacagggcc tggagtggat tggatggatt gatcctgaga atggtgatac tgaatatgcc 240
ccgaagttcc agggcaaggc cactatgact gcagacacat cctccaacac agcctacctg 300
cagctcagca gcctgacatc tgaggacact gccgtctatt actgtaatgg ggggtatggt 360
aacttttact ttgactactg gggccaaggc accactgtca ccgtctcg 408

<210> 68
<211> 128
<212> PRT
<213> Mus musculus

<400> 68
Met Glu Phe Gln Thr Gln Val Phe Val Tyr Met Leu Leu Trp Leu Ser
1               5                   10                  15
Gly Val Glu Gly Asp Ile Val Met Thr Gln Ser His Lys Phe Met Ser
            20                  25                  30
Thr Ser Val Gly Asp Arg Val Ser Ile Thr Cys Lys Ala Ser Gln Asp
        35                  40                  45
Val Gly Thr Ala Val Ala Trp Tyr Gln Gln Lys Pro Gly Gln Ser Pro
    50                  55                  60
Lys Leu Leu Ile Tyr Trp Ala Ser Thr Arg His Thr Gly Val Pro Asp
65                  70                  75                  80
Arg Phe Thr Gly Ser Gly Ser Gly Thr Asp Phe Thr Leu Thr Ile Ser
                85                  90                  95
Asn Val Gln Ser Glu Asp Leu Ala Asp Tyr Phe Cys Gln Gln Tyr Ser
            100                 105                 110
Ser Tyr Pro Tyr Thr Phe Gly Gly Gly Thr Lys Leu Glu Leu Lys Arg
        115                 120                 125

<210> 69
<211> 381
<212> DNA
<213> Mus musculus

<400> 69
atggaattcc agactcaggt ctttgtatac atgttgctgt ggttgtctgg tgttgaagga 60
gacattgtga tgacccagtc tcacaaattc atgtccacat cagtaggaga cagggtcagc 120
atcacctgca aggccagtca ggatgtgggt actgctgtag cctggtatca acagaaacca 180
gggcaatctc ctaaactact gatttactgg gcatccaccc ggcacactgg agtccctgat 240
cgcttcacag gcagtggatc tgggacagat ttcactctca ccattagcaa tgtgcagtct 300
gaagacttgg cagattattt ctgtcagcaa tatagcagct atccgtacac gttcggaggg 360
gggaccaagc tggagctgaa a 381

<210> 70
<211> 137
<212> PRT
<213> Mus musculus

<400> 70
Met Lys Cys Asn Trp Val Ile Phe Phe Leu Met Ala Val Val Ile Gly
1               5                   10                  15
Ile Asn Ser Glu Val Gln Leu Gln Gln Ser Gly Ala Glu Leu Val Arg
            20                  25                  30
Ser Gly Ala Ser Val Lys Leu Ser Cys Thr Ala Ser Gly Phe Asn Ile
        35                  40                  45
Lys Asp Tyr Phe Met His Trp Val Lys Gln Arg Pro Glu Gln Gly Leu
    50                  55                  60
Glu Trp Ile Gly Trp Ile Asp Pro Glu Asn Gly Asp Thr Glu Tyr Ala
65                  70                  75                  80
Pro Lys Phe Gln Gly Lys Ala Thr Met Thr Ala Asp Thr Ser Ser Asn

                    85                  90                  95
Thr Ala Tyr Leu Gln Leu Ser Ser Leu Thr Ser Glu Asp Thr Ala Val
            100                 105                 110
Tyr Tyr Cys Asn Ala Gly Gln Ala Thr Tyr Tyr Phe Asp Tyr Trp Gly
        115                 120                 125
Gln Gly Thr Thr Val Thr Val Ser Ser
    130                 135

<210> 71
<211> 408
<212> DNA
<213> Mus musculus

<400> 71
atgaaatgca actgggtcat cttcttcctg atggcagtgg ttataggaat caattcagag 60
gttcagctgc agcagtctgg ggcagagctt gtgaggtcag gggcctcagt caagttgtcc 120
tgcacagctt ctggcttcaa cattaaagac tactttatgc actgggtgaa gcagaggcct 180
gaacagggcc tggagtggat tggatggatt gatcctgaga atggtgatac tgaatatgcc 240
ccgaagttcc agggcaaggc cactatgact gcagacacat cctccaacac agcctacctg 300
cagctcagca gcctgacatc tgaggacact gccgtctatt actgtaatgc agggcaggca 360
acttactact ttgactactg gggccaaggc accacggtca ccgtctcg             408

<210> 72
<211> 133
<212> PRT
<213> Mus musculus

<400> 72
Met Glu Thr Asp Thr Leu Arg Ala Lys Leu Arg Ser His His Glu Ala
 1               5                  10                  15
Ala Cys Ser Ala Phe Arg Cys Glu Met Asp Ile Gln Met Asn Gln Ser
            20                  25                  30
Pro Ser Ser Leu Ser Ala Ser Leu Gly Asp Thr Ile Thr Ile Thr Cys
        35                  40                  45
His Ala Thr Gln Asn Ile Asn Val Trp Leu Ser Trp Tyr Gln Gln Lys
    50                  55                  60
Pro Gly Asn Ile Pro Thr Leu Leu Ile Tyr Lys Thr Ser Asn Leu His
65                  70                  75                  80
Thr Gly Val Pro Ser Arg Phe Ser Gly Ser Gly Ser Gly Thr Gly Phe
                85                  90                  95
Thr Leu Thr Ile Ser Ser Leu Gln Ser Glu Asp Ile Ala Thr Tyr Tyr
            100                 105                 110
Cys Gln Gln Gly Gln Ser Phe Pro Tyr Thr Phe Gly Gly Gly Thr Lys
        115                 120                 125
Leu Glu Leu Lys Arg
    130

<210> 73
<211> 397
<212> DNA
<213> Mus musculus

<400> 73
atggagacag acacactccg agcgaagctt cgaagccacc atgaagctgc ctgttctgct 60
tttaggtgtg agatgtgaca tccagatgaa ccagtctcca tccagtctgt ctgcatccct 120
tggagacaca attaccatca cttgccatgc cactcagaac attaatgttt ggttaagctg 180
gtaccagcag aaaccaggaa atattcctac acttttgatc tataagactt ccaacttgca 240
cacaggcgtc ccatcaaggt ttagtggcag tggatctgga acaggtttca cattaaccat 300
cagcagcctg cagtctgaag acattgccac ttactactgt caacagggtc aaagttttcc 360
gtacacgttc ggaggaggca ccaagctgga gctgaaa                         397

<210> 74
<211> 137
<212> PRT
<213> Mus musculus

<400> 74
Met Lys Cys Asn Trp Val Ile Phe Phe Leu Met Ala Val Val Ile Gly
1               5                   10                  15
Ile Asn Ser Glu Val Gln Leu Gln Gln Ser Gly Ala Glu Leu Val Arg
            20                  25                  30
Ser Gly Ala Ser Val Lys Leu Ser Cys Thr Ala Ser Gly Phe Asn Ile
        35                  40                  45
Lys Asp Tyr Phe Met His Trp Val Lys Gln Arg Pro Glu Gln Gly Leu
    50                  55                  60
Glu Trp Ile Gly Trp Ile Asp Pro Glu Asn Gly Asp Thr Glu Tyr Ala
65                  70                  75                  80
Pro Lys Phe Gln Gly Lys Ala Thr Met Thr Ala Asp Thr Ser Ser Asn
                85                  90                  95
Thr Ala Tyr Leu Gln Leu Ser Ser Leu Thr Ser Glu Asp Thr Ala Val
            100                 105                 110
Tyr Tyr Cys Asn Ala Gly Gln Ala Thr Tyr Tyr Phe Asp Tyr Trp Gly
        115                 120                 125
Gln Gly Thr Thr Val Thr Val Ser Ser
    130                 135

<210> 75
<211> 408
<212> DNA
<213> Mus musculus

<400> 75
atgaaatgca actgggtcat cttcttcctg atggcagtgg ttataggaat caattcagag 60
gttcagctgc agcagtctgg ggcagagctt gtgaggtcag gggcctcagt caagttgtcc 120
tgcacagctt ctggcttcaa cattaaagac tactttatgc actgggtgaa gcagaggcct 180
gaacagggcc tggagtggat tggatggatt gatcctgaga atggtgatac tgaatatgcc 240
ccgaagttcc agggcaaggc cactatgact gcagacacat cctccaacac agcctacctg 300
cagctcagca gcctgacatc tgaggacact gccgtctatt actgtaatgc agggcaggca 360
acttactact ttgactactg gggccaaggc accacggtca ccgtctcg             408

<210> 76
<211> 134
<212> PRT
<213> Mus musculus

<400> 76
Met Glu Ser Gln Thr Gln Val Leu Met Leu Leu Leu Leu Trp Val Ser
1               5                   10                  15
Gly Thr Cys Gly Asp Ile Met Met Ser Gln Ser Pro Ser Ser Leu Ala
            20                  25                  30
Val Ser Val Gly Glu Lys Val Thr Met Ser Cys Lys Ser Ser Gln Ser
        35                  40                  45
Leu Leu Tyr Ser Asn Asn Gln Lys Asn Tyr Leu Ala Trp Tyr Gln Gln
    50                  55                  60
Lys Pro Gly Gln Ser Pro Lys Leu Leu Ile Ser Trp Ala Ser Thr Arg
65                  70                  75                  80
Glu Ser Gly Val Pro Asp Arg Phe Thr Gly Ser Gly Ser Gly Thr Asp
                85                  90                  95
Phe Thr Leu Thr Ile Ser Ser Val Lys Ala Glu Asp Leu Ala Val Tyr
            100                 105                 110
Tyr Cys Gln Gln Tyr Tyr Asp Tyr Pro Trp Thr Phe Gly Gly Gly Ser
        115                 120                 125
Lys Leu Glu Leu Lys Arg

    130

<210> 77
<211> 399
<212> DNA
<213> Mus musculus

<400> 77
atggaatcac agacccaggt tcttatgtta ctgctgctat gggtatctgg tacctgtggg 60
gacattatga tgtcacagtc tccatcctcc ctagctgtgt cagttggaga gaaggttact 120
atgagctgca agtccagtca gagcctttta tatagtaaca atcaaaagaa ctacttggcc 180
tggtaccagc agaaaccagg gcagtctcct aaactgctga tttcctgggc atccactagg 240
gaatctgggg tccctgatcg cttcacaggc agtggatctg ggacagattt cactctcacc 300
atcagcagtg tgaaggctga agacctggct gtttattact gtcagcaata ttatgactat 360
ccgtggacgt tcggtggagg ctccaagctg gagctgaaa 399

<210> 78
<211> 137
<212> PRT
<213> Mus musculus

<400> 78
Met Gly Trp Asn Trp Ile Ile Phe Phe Leu Met Ala Val Val Ile Gly
1               5                   10                  15
Ile Asn Ser Glu Val Gln Leu Gln Gln Ser Gly Ala Glu Leu Val Arg
            20                  25                  30
Ser Gly Ala Ser Val Lys Leu Ser Cys Thr Ala Ser Gly Phe Asn Ile
        35                  40                  45
Lys Asp Tyr Phe Met His Trp Val Lys Gln Arg Pro Glu Gln Gly Leu
    50                  55                  60
Glu Trp Ile Gly Trp Ile Asp Pro Glu Asn Gly Asp Thr Glu Tyr Ala
65                  70                  75                  80
Pro Lys Phe Gln Gly Lys Ala Thr Met Thr Ala Asp Thr Ser Ser Asn
                85                  90                  95
Thr Ala Tyr Leu Gln Leu Ser Ser Leu Thr Ser Glu Asp Thr Ala Val
            100                 105                 110
Tyr Tyr Cys Asn Ala Gly Gln Ala Thr Tyr Tyr Phe Asp Tyr Trp Gly
        115                 120                 125
Gln Gly Thr Thr Val Thr Val Ser Ser
    130                 135

<210> 79
<211> 408
<212> DNA
<213> Mus musculus

<400> 79
atgggatgga actggatcat cttcttcctg atggcagtgg ttataggaat caattcagag 60
gttcagctgc agcagtctgg ggcagagctt gtgaggtcag gggcctcagt caagttgtcc 120
tgcacagctt ctggcttcaa cattaaagac tactttatgc actgggtgaa gcagaggcct 180
gaacagggcc tggagtggat tggatggatt gatcctgaga atggtgatac tgaatatgcc 240
ccgaagttcc agggcaaggc cactatgact gcagacacat cctccaacac agcctacctg 300
cagctcagca gcctgacatc tgaggacact gccgtctatt actgtaatgc agggcaggca 360
acttactact ttgactactg gggccaaggc accacggtca ccgtctcg 408

<210> 80
<211> 130
<212> PRT
<213> Mus musculus

<400> 80

Met Asp Met Arg Thr Pro Ala Gln Phe Leu Gly Leu Leu Leu Leu Cys
1               5                   10                  15
Phe Gln Gly Thr Arg Cys Asp Ile Gln Met Thr Gln Thr Ser Ser Ser
            20                  25                  30
Leu Ser Ala Ser Leu Gly Asp Arg Val Thr Ile Ser Cys Arg Thr Ser
        35                  40                  45
Gln Asp Ile Asn Asn Phe Leu Asn Trp Tyr Gln Gln Lys Pro Asp Gly
    50                  55                  60
Thr Val Lys Phe Leu Ile His His Thr Ser Arg Leu Lys Ser Gly Val
65                  70                  75                  80
Pro Ser Arg Phe Ser Gly Ser Gly Ser Gly Thr Asp Tyr Ser Leu Thr
                85                  90                  95
Ile Ser His Leu Glu Pro Glu Asp Ile Ala Thr Tyr Tyr Cys Gln His
            100                 105                 110
Tyr Tyr Asn Leu Pro Trp Thr Phe Gly Gly Gly Thr Lys Leu Glu Leu
        115                 120                 125
Lys Arg
    130

<210> 81
<211> 387
<212> DNA
<213> Mus musculus

<400> 81
atggacatga ggacccctgc tcagttcctt ggtctcctgt tgctctgttt tcaaggtacc 60
agatgtgata tccagatgac acagacttca tcttccctgt ctgcctctct gggagacaga 120
gtcaccatca gttgcaggac aagtcaggat attaacaatt ttttaaactg gtatcagcag 180
aaaccagatg gaactgttaa attcctgatc caccacacat caagattaaa gtcaggagtc 240
ccatcaaggt tcagtggcag tgggtctggg acagattatt ctctcaccat cagccacctg 300
gaacctgaag atattgccac ttactattgt cagcactatt ataatcttcc gtggacgttc 360
ggtggaggca ccaagttgga gctgaaa                                     387

<210> 82
<211> 138
<212> PRT
<213> Mus musculus

<400> 82
Met Gly Trp Ser Trp Thr Phe Leu Phe Leu Leu Ser Gly Thr Ala Gly
1               5                   10                  15
Val His Ser Glu Ile Gln Leu Gln Gln Thr Gly Pro Glu Leu Val Lys
            20                  25                  30
Pro Gly Ala Ser Val Lys Ile Ser Cys Lys Ala Ser Gly Tyr Ser Phe
        35                  40                  45
Thr Asp Asn Ile Met Val Trp Val Lys Gln Ser His Gly Lys Ser Leu
    50                  55                  60
Glu Trp Ile Gly Asn Ile Asn Pro Tyr Tyr Gly Ser Pro Ser Tyr Asn
65                  70                  75                  80
Leu Lys Phe Lys Asp Lys Ala Thr Leu Thr Val Asp Lys Ser Ser Ser
                85                  90                  95
Thr Ala Tyr Met Gln Leu Asn Ser Leu Thr Ser Glu Asp Ser Ala Val
            100                 105                 110
Tyr Tyr Cys Ala Arg Glu Gly Gly Asn Tyr Gly Ser Leu Asp Asn Trp
        115                 120                 125
Gly Gln Gly Thr Ser Val Thr Val Ser Ser
    130                 135

<210> 83
<211> 411
<212> DNA

<213> Mus musculus

<400> 83
atgggatgga gctggacctt tctcttcctc ctgtcaggaa ctgcaggtgt ccactctgag 60
atccagctgc agcagactgg acctgagctg gtgaagcctg gggcttcagt gaagatatcc 120
tgcaaggctt ctggttattc attcactgac aacatcatgg tctgggtgaa gcagagccat 180
ggaaagagcc ttgagtggat tggaaacatt aatccttact atggtagtcc tagttacaat 240
ctgaagttca aggacaaggc cacattgact gtagacaaat cttccagcac agcctacatg 300
cagctcaata gtctgacatc tgaggactct gcagtctatt actgtgcaag agaggggggt 360
aattacggtt ctctggacaa ctggggtcaa ggaacctcgg tcaccgtctc g 411

<210> 84
<211> 134
<212> PRT
<213> Mus musculus

<400> 84
Met Lys Ser Gln Thr Gln Val Leu Met Leu Leu Leu Leu Trp Val Ser
1               5                   10                  15
Gly Thr Cys Gly Asp Ile Met Met Ser Gln Ser Pro Ser Ser Leu Ala
            20                  25                  30
Val Ser Val Gly Glu Lys Val Thr Met Ser Cys Lys Ser Ser Gln Ser
        35                  40                  45
Leu Leu Tyr Ser Asn Asn Gln Lys Asn Tyr Leu Ala Trp Tyr Gln Gln
    50                  55                  60
Lys Pro Gly Gln Ser Pro Lys Leu Leu Ile Ser Trp Ala Ser Thr Arg
65                  70                  75                  80
Glu Ser Gly Val Pro Asp Arg Phe Thr Gly Ser Gly Ser Gly Thr Asp
                85                  90                  95
Phe Thr Leu Thr Ile Ser Ser Val Lys Ala Glu Asp Leu Ala Val Tyr
            100                 105                 110
Tyr Cys Gln Gln Tyr Tyr Asp Tyr Pro Trp Thr Phe Gly Gly Gly Ser
        115                 120                 125
Lys Leu Glu Ile Lys Arg
    130

<210> 85
<211> 399
<212> DNA
<213> Mus musculus

<400> 85
atgaagtcac agacccaggt tcttatgtta ctgctgctat gggtatctgg tacctgtggg 60
gacattatga tgtcacagtc tccatcctcc ctagctgtgt cagttggaga gaaggttact 120
atgagctgca agtccagtca gagcctttta tatagtaaca atcaaaagaa ctacttggcc 180
tggtaccagc agaaaccagg gcagtctcct aaactgctga tttcctgggc atccactagg 240
gaatctgggg tccctgatcg cttcacaggc agtggatctg ggacagattt cactctcacc 300
atcagcagtg tgaaggctga agacctggct gtttattact gtcagcaata ttatgactat 360
ccgtggacgt tcggtggagg ctccaagctg gaaataaaa 399

<210> 86
<211> 138
<212> PRT
<213> Mus musculus

<400> 86
Met Gly Trp Ser Trp Thr Phe Leu Phe Leu Leu Ser Gly Thr Ala Gly
1               5                   10                  15
Val His Ser Glu Ile Gln Leu Gln Gln Thr Gly Pro Glu Leu Val Lys
            20                  25                  30
Pro Gly Ala Ser Val Lys Ile Ser Cys Lys Ala Ser Gly Tyr Ser Phe
        35                  40                  45

Thr Asp Asn Ile Met Val Trp Val Lys Gln Ser His Gly Lys Ser Leu
    50                  55                  60
Glu Trp Ile Gly Asn Ile Asn Pro Tyr Tyr Gly Ser Pro Ser Tyr Asn
65                  70                  75                  80
Leu Lys Phe Lys Asp Lys Ala Thr Leu Thr Val Asp Lys Ser Ser Ser
                85                  90                  95
Thr Ala Tyr Met Gln Leu Asn Ser Leu Thr Ser Glu Asp Ser Ala Val
            100                 105                 110
Tyr Tyr Cys Ala Arg Glu Gly Gly Asn Tyr Gly Ser Leu Asp Asn Trp
        115                 120                 125
Gly Gln Gly Thr Ser Val Thr Val Ser Ser
    130                 135

<210> 87
<211> 411
<212> DNA
<213> Mus musculus

<400> 87
atgggatgga gctggacctt tctcttcctc ctgtcaggaa ctgcaggtgt ccactctgag 60
atccagctgc agcagactgg acctgagctg gtgaagcctg gggcttcagt gaagatatcc 120
tgcaaggctt ctggttattc attcactgac aacatcatgg tctgggtgaa gcagagccat 180
ggaaagagcc ttgagtggat tggaaacatt aatccttact atggtagtcc tagttacaat 240
ctgaagttca aggacaaggc cacattgact gtagacaaat cttccagcac agcctacatg 300
cagctcaata gtctgacatc tgaggactct gcagtctatt actgtgcaag agaggggggt 360
aattacggtt ctctggacaa ctggggtcaa ggaacctcgg tcaccgtctc g 411

<210> 88
<211> 127
<212> PRT
<213> Mus musculus

<400> 88
Met Ser Pro Ala Gln Phe Leu Gly Leu Leu Leu Leu Cys Phe Gln Gly
 1               5                  10                  15
Thr Arg Cys Asp Ile Gln Met Thr Gln Thr Ser Ser Ser Leu Ser Ala
            20                  25                  30
Ser Leu Gly Asp Arg Val Thr Ile Ser Cys Arg Thr Ser Gln Asp Ile
        35                  40                  45
Asn Asn Phe Leu Asn Trp Tyr Gln Gln Lys Pro Asp Gly Thr Val Lys
    50                  55                  60
Phe Leu Ile His His Thr Ser Arg Leu Lys Ser Gly Val Pro Ser Arg
65                  70                  75                  80
Phe Ser Gly Ser Gly Ser Gly Thr Asp Tyr Ser Leu Thr Ile Ser His
                85                  90                  95
Leu Glu Pro Glu Asp Ile Ala Thr Tyr Tyr Cys Gln His Tyr Tyr Asn
            100                 105                 110
Leu Pro Trp Thr Phe Gly Gly Gly Thr Lys Leu Glu Ile Lys Arg
        115                 120                 125

<210> 89
<211> 378
<212> DNA
<213> Mus musculus

<400> 89
atgagtcctg cccagttcct tggtctcctg ttgctctgtt ttcaaggtac cagatgtgat 60
atccagatga cacagacttc atcttccctg tctgcctctc tgggagacag agtcaccatc 120
agttgcagga caagtcagga tattaacaat tttttaaact ggtatcagca gaaaccagat 180
ggaactgtta aattcctgat ccaccacaca tcaagattaa agtcaggagt cccatcaagg 240
ttcagtggca gtgggtctgg gacagattat tctctcacca tcagccacct ggaacctgaa 300

gatatcgcca cttactattg tcagcactat tataatcttc cgtggacgtt cggtggaggc 360
acaaagttgg aaataaaa 378

<210> 90
<211> 134
<212> PRT
<213> Mus musculus

<400> 90
Met Gly Trp Ser Trp Thr Phe Leu Phe Leu Leu Ser Gly Thr Ala Gly
1               5                   10                  15
Val His Ser Glu Val Gln Leu Val Glu Ser Gly Gly Gly Leu Val Lys
            20                  25                  30
Pro Gly Gly Ser Leu Lys Leu Ser Cys Ala Val Ser Gly Phe Ser Leu
        35                  40                  45
Ser Tyr Tyr Tyr Met Ser Trp Val Arg Gln Thr Pro Glu Lys Arg Leu
    50                  55                  60
Glu Trp Ile Gly Thr Ile Ser Ile Asp Gly Ser Thr Tyr Tyr Ala Ser
65                  70                  75                  80
Trp Ala Glu Gly Arg Phe Thr Ile Ser Lys Asp Ser Thr Thr Val Tyr
                85                  90                  95
Leu Gln Met Ser Ser Leu Lys Ser Glu Asp Thr Ala Met Tyr Tyr Cys
            100                 105                 110
Ala Arg Gly His Ile Asn Thr Gly Met Asp Pro Trp Gly Gln Gly Thr
        115                 120                 125
Pro Val Thr Val Ser Ser
    130

<210> 91
<211> 399
<212> DNA
<213> Mus musculus

<400> 91
atgggatgga gctggacctt tctcttcctc ctgtcaggaa ctgcaggtgt ccactctgag 60
gttcagctcg ttgaatccgg aggcggactc gtgaagcccg ggggcagtct taaactgtcc 120
tgcgccgtca gcgggttttc cctcagttac tattacatgt catgggtgcg gcagaccccc 180
gagaaaaggt tggaatggat tggcaccatc tcaattgacg gaagcacata ttacgcatct 240
tgggccgagg gtcgcttcac aatttctaag gacagtacta ccgtgtatct gcagatgtcc 300
agccttaaga gcgaagatac agccatgtac tactgtgcta gagggcacat caacactgga 360
atggatcctt ggggccaggg caccccggtc acagtctcg 399

<210> 92
<211> 132
<212> PRT
<213> Mus musculus

<400> 92
Met Lys Leu Pro Val Arg Leu Leu Val Leu Met Phe Trp Ile Pro Ala
1               5                   10                  15
Ser Ser Ser Asp Val Leu Met Thr Gln Asn Pro Leu Ser Leu Pro Val
            20                  25                  30
Ser Leu Gly Asp Gln Ala Ser Ile Ser Cys Arg Ser Ser Gln Thr Ile
        35                  40                  45
Val His Ser Asn Gly Asn Thr Tyr Leu Glu Trp Tyr Leu Gln Lys Pro
    50                  55                  60
Gly Gln Ser Pro Lys Leu Leu Ile Tyr Glu Val Ser Asn Arg Phe Ser
65                  70                  75                  80
Gly Val Pro Asp Arg Phe Ser Gly Ser Gly Ser Gly Thr Asp Phe Thr
                85                  90                  95
Leu Lys Ile Ser Arg Val Glu Ala Glu Asp Leu Gly Ile Tyr Tyr Cys
            100                 105                 110

Phe Gln Gly Ser His Phe Pro His Thr Phe Gly Gly Gly Thr Lys Leu
        115                 120                 125
Glu Leu Lys Arg
    130

<210> 93
<211> 393
<212> DNA
<213> Mus musculus

<400> 93
atgaagttgc ctgttaggct gttggtgctg atgttctgga ttcctgcttc cagcagtgat 60
gttttgatga cccaaaatcc actctccctg cctgtcagtc ttggagatca agcctccatc 120
tcctgcagat caagtcagac cattgtacat agtaatggaa acacctattt agaatggtac 180
ctgcagaaac caggccagtc tccaaagctc ctgatctacg aagtttccaa ccgattttct 240
ggggtcccag acaggttcag tggcagtgga tcagggacag atttcacact caaaatcagc 300
agagtggagg ctgaggatct gggaatttat tactgctttc aaggttcaca ttttccgcac 360
acgttcggag gggggaccaa gctggaactg aaa                              393

<210> 94
<211> 143
<212> PRT
<213> Mus musculus

<400> 94
Met Glu Trp Ser Gly Val Ile Phe Phe Leu Met Ala Val Val Ile Gly
 1               5                  10                  15
Ile Asn Ser Glu Val Gln Leu Gln Gln Ser Gly Ala Glu Phe Val Arg
            20                  25                  30
Ser Gly Ala Ser Val Lys Leu Ser Cys Thr Ala Ser Gly Phe Asn Ile
        35                  40                  45
Lys Asp Tyr Tyr Met Tyr Trp Val Lys Gln Arg Pro Glu Gln Gly Leu
    50                  55                  60
Glu Trp Ile Gly Trp Ile Asp Pro Glu Asn Gly Asp Thr Glu Cys Ala
65                  70                  75                  80
Pro Lys Phe Gln Gly Lys Ala Thr Met Thr Ala Asp Thr Ser Ser Asn
                85                  90                  95
Thr Ala Tyr Leu Gln Leu Ser Ser Leu Thr Ser Glu Asp Thr Ala Val
            100                 105                 110
Tyr Tyr Cys Asn Ala Asp Arg Tyr Asp Glu Gly Ala Ala Ser Asp Tyr
        115                 120                 125
Gly Met Asp Tyr Trp Gly Gln Gly Ser Ser Val Thr Val Ser Ser
    130                 135                 140

<210> 95
<211> 426
<212> DNA
<213> Mus musculus

<400> 95
atggaatgga gcggggtcat cttcttcctg atggcagtgg ttataggaat caattcagag 60
gttcagctgc agcagtctgg ggcagagttt gtgaggtcag gggcctcagt caagttgtcc 120
tgcacagctt ctggcttcaa cattaaagac tactatatgt attgggtgaa acagaggcct 180
gaacagggcc tggagtggat tggatggatt gatcctgaga atggtgatac tgaatgtgcc 240
ccgaagttcc agggcaaggc cactatgact gcagacacat cctccaacac agcctacctg 300
caactcagca gcctgacatc tgaggacact gccgtctatt actgtaatgc cgataggtac 360
gacgaggggg ccgcgtcgga ctatggtatg gactactggg gtcaaggaag ttcagtcaca 420
gtctcg                                                            426

<210> 96
<211> 132

<212> PRT
<213> Mus musculus

<400> 96
Met Lys Leu Pro Val Arg Leu Leu Val Leu Met Phe Trp Ile Pro Ala
1               5                   10                  15
Ser Ser Ser Asp Val Leu Met Thr Gln Asn Pro Leu Ser Leu Pro Val
            20                  25                  30
Ser Leu Gly Asp Gln Ala Ser Ile Ser Cys Arg Ser Ser Gln Ser Ile
        35                  40                  45
Val His Ser Asn Gly Asn Thr His Leu Glu Trp Tyr Leu Gln Lys Pro
    50                  55                  60
Gly Gln Ser Pro Lys Leu Leu Ile Tyr Lys Val Ser Asn Arg Phe Ser
65                  70                  75                  80
Gly Val Pro Asp Arg Phe Ser Gly Ser Gly Ser Gly Thr Asp Phe Thr
                85                  90                  95
Leu Lys Ile Ser Arg Val Glu Ala Glu Asp Leu Gly Val Tyr Tyr Cys
            100                 105                 110
Phe Gln Gly Ser His Ala Pro His Thr Phe Gly Gly Gly Thr Lys Leu
        115                 120                 125
Glu Leu Lys Arg
    130

<210> 97
<211> 393
<212> DNA
<213> Mus musculus

<400> 97
atgaagttgc ctgttaggct gttggtgctg atgttctgga ttcctgcttc cagcagtgat 60
gttttgatga cccaaaatcc actctccctg cctgtcagtc ttggagatca agcctccatc 120
tcttgcagat ctagtcagag cattgtacat agtaatggaa acacccattt agaatggtac 180
ctgcagaaac caggccagtc tccaaagctc ctgatctaca aagtttccaa ccgattttct 240
ggggtcccag acaggttcag tggcagtgga tcagggacag atttcacact caagatcagc 300
agagtggagg ctgaggatct gggagtttat tactgctttc aaggttcaca tgctccgcac 360
acgttcggag gggggaccaa gctggaactg aaa                              393

<210> 98
<211> 143
<212> PRT
<213> Mus musculus

<400> 98
Met Lys Cys Ser Trp Val Ile Phe Phe Leu Met Ala Val Val Ile Gly
1               5                   10                  15
Ile Asn Ser Glu Val Gln Leu Gln Gln Phe Gly Ala Glu Phe Val Arg
            20                  25                  30
Ser Gly Ala Ser Val Lys Leu Ser Cys Thr Ala Ser Gly Phe Asn Ile
        35                  40                  45
Lys Asp Tyr Tyr Met Tyr Trp Val Lys Gln Arg Pro Glu Gln Gly Leu
    50                  55                  60
Glu Trp Ile Gly Trp Ile Asp Pro Gly Asn Gly Asp Thr Glu Cys Ala
65                  70                  75                  80
Pro Lys Phe Gln Gly Lys Ala Thr Met Thr Ala Asp Thr Ser Ser Asn
                85                  90                  95
Thr Ala Tyr Leu Gln Leu Ser Ser Leu Thr Ser Glu Asp Thr Ala Val
            100                 105                 110
Tyr Tyr Cys Asn Ala Asp Arg Tyr Asp Glu Gly Ala Ala Ser Asp Tyr
        115                 120                 125
Ala Val Asp Tyr Trp Gly Gln Gly Ser Ser Val Thr Val Ser Ser
    130                 135                 140

<210> 99
<211> 426
<212> DNA
<213> Mus musculus

<400> 99
atgaaatgca gctgggtcat cttcttcctg atggcagtgg ttataggaat caattcagag 60
gttcagctgc agcagtttgg ggcagagttt gtgaggtcag gggcctcagt caagttgtcc 120
tgcacagctt ctggcttcaa cattaaagac tactatatgt attgggtgaa acagaggcct 180
gaacagggcc tggagtggat tggatggatt gatcctggga atggtgatac tgaatgtgcc 240
ccgaagttcc agggcaaggc cactatgact gcagacacat cctccaacac agcctacctg 300
cagctcagca gcctgacatc tgaggacact gccgtctatt actgtaatgc cgatagatac 360
gacgaggggg ccgcgtcgga ctatgctgtg gactactggg gtcaaggaag ttcggtcaca 420
gtctcg 426

<210> 100
<211> 132
<212> PRT
<213> Mus musculus

<400> 100
Met Lys Leu Pro Val Arg Leu Leu Val Leu Met Phe Trp Ile Pro Ala
1               5                   10                  15
Ser Ser Ser Asp Val Leu Met Thr Gln Asn Pro Leu Ser Leu Pro Val
            20                  25                  30
Ser Leu Gly Asp Gln Ala Ser Ile Ser Cys Arg Ser Ser Gln Thr Ile
        35                  40                  45
Val His Ser Asn Gly Asn Thr Tyr Leu Glu Trp Tyr Leu Gln Lys Pro
    50                  55                  60
Gly Gln Ser Pro Lys Leu Leu Ile Tyr Glu Val Ser Asn Arg Phe Ser
65                  70                  75                  80
Gly Val Pro Asp Arg Phe Ser Gly Ser Gly Ser Gly Thr Asp Phe Thr
                85                  90                  95
Leu Lys Ile Ser Arg Val Gly Ala Glu Asp Leu Gly Ile Tyr Tyr Cys
            100                 105                 110
Phe Gln Gly Ser Leu Phe Pro His Thr Phe Gly Gly Gly Thr Lys Leu
        115                 120                 125
Glu Leu Lys Arg
    130

<210> 101
<211> 393
<212> DNA
<213> Mus musculus

<400> 101
atgaagttgc ctgttaggct gttggtgctg atgttctgga ttcctgcttc cagcagtgat 60
gttttgatga cccaaaatcc actctccctg cctgtcagtc ttggagatca agcctccatc 120
tcttgcagat ctagtcagac cattgtacat agtaatggaa acacctattt agaatggtac 180
ctgcagaaac caggccagtc tccaaagctc ctgatctacg aagtttccaa ccgattttct 240
ggggtcccag acaggttcag tggcagtggc tcagggacag atttcacact caaaatcagc 300
agagtggggg ctgaagatct gggaatttat tactgctttc aaggttcact ttttccgcac 360
acgttcggag gggggaccaa gttggagctc aaa 393

<210> 102
<211> 143
<212> PRT
<213> Mus musculus

<400> 102
Met Glu Trp Ser Cys Ile Ile Phe Phe Leu Met Ala Val Val Ile Gly
1               5                   10                  15

Ile Asn Ser Glu Val Gln Leu Gln Gln Ser Gly Ala Glu Phe Val Arg
            20                  25                  30
Ser Gly Ala Ser Val Lys Leu Ser Cys Thr Ala Ser Gly Phe Asn Ile
        35                  40                  45
Lys Asp Tyr Tyr Met Tyr Trp Val Lys Gln Arg Pro Glu Gln Gly Leu
    50                  55                  60
Glu Trp Ile Gly Trp Ile Asp Pro Glu Asn Gly Asp Thr Glu Cys Ala
65                  70                  75                  80
Pro Lys Phe Gln Gly Lys Ala Thr Met Thr Ala Asp Thr Ser Ser Asn
                85                  90                  95
Thr Ala Tyr Leu Gln Leu Ser Ser Leu Thr Ser Glu Asp Thr Ala Val
            100                 105                 110
Tyr Tyr Cys Asn Ala Asp Arg Tyr Asp Glu Gly Ala Ala Ser Asp Tyr
        115                 120                 125
Ala Val Asp Tyr Trp Gly Gln Gly Ser Ser Val Thr Val Ser Ser
    130                 135                 140

<210> 103
<211> 426
<212> DNA
<213> Mus musculus

<400> 103
atggaatgga gctgtatcat cttcttcctg atggcagtgg ttataggaat caattcagag 60
gttcagctgc agcagtctgg ggcagagttt gtgaggtcag gggcctcagt caagttgtcc 120
tgcacagctt ctggcttcaa cattaaagac tactatatgt attgggtgaa acagaggcct 180
gaacagggcc tggagtggat tggatggatt gatcctgaga atggtgatac tgaatgtgcc 240
ccgaagttcc agggcaaggc cactatgact gcagacacat cctccaacac agcctacctg 300
cagctcagca gcctgacatc tgaggacact gccgtctatt actgtaatgc cgatagatac 360
gacgaggggg ccgcgtcgga ctatgctgtg gactactggg gtcaaggaag ctcagtcacc 420
gtctcg                                                            426

<210> 104
<211> 106
<212> PRT
<213> Mus musculus

<400> 104
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Val Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu Cys
            100                 105

<210> 105
<211> 327
<212> PRT
<213> Mus musculus

<400> 105
Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95
Arg Val Glu Ser Lys Tyr Gly Pro Pro Cys Pro Pro Cys Pro Ala Pro
            100                 105                 110
Glu Phe Leu Gly Gly Pro Ser Val Phe Leu Phe Pro Pro Lys Pro Lys
        115                 120                 125
Asp Thr Leu Met Ile Ser Arg Thr Pro Glu Val Thr Cys Val Val Val
    130                 135                 140
Asp Val Ser Gln Glu Asp Pro Glu Val Gln Phe Asn Trp Tyr Val Asp
145                 150                 155                 160
Gly Val Glu Val His Asn Ala Lys Thr Lys Pro Arg Glu Glu Gln Phe
                165                 170                 175
Asn Ser Thr Tyr Arg Val Val Ser Val Leu Thr Val Leu His Gln Asp
            180                 185                 190
Trp Leu Asn Gly Lys Glu Tyr Lys Cys Lys Val Ser Asn Lys Gly Leu
        195                 200                 205
Pro Ser Ser Ile Glu Lys Thr Ile Ser Lys Ala Lys Gly Gln Pro Arg
    210                 215                 220
Glu Pro Gln Val Tyr Thr Leu Pro Pro Ser Gln Glu Glu Met Thr Lys
225                 230                 235                 240
Asn Gln Val Ser Leu Thr Cys Leu Val Lys Gly Phe Tyr Pro Ser Asp
                245                 250                 255
Ile Ala Val Glu Trp Glu Ser Asn Gly Gln Pro Glu Asn Asn Tyr Lys
            260                 265                 270
Thr Thr Pro Pro Val Leu Asp Ser Asp Gly Ser Phe Phe Leu Tyr Ser
        275                 280                 285
Arg Leu Thr Val Asp Lys Ser Arg Trp Gln Glu Gly Asn Val Phe Ser
    290                 295                 300
Cys Ser Val Met His Glu Ala Leu His Asn His Tyr Thr Gln Lys Ser
305                 310                 315                 320
Leu Ser Leu Ser Leu Gly Lys
                325

<210> 106
<211> 13
<212> PRT
<213> Oryctolagus cuniculus

<400> 106
Gln Ser Ser Gln Arg Val Phe Asn Asn Asn Glu Leu Ser
1               5                   10

<210> 107
<211> 7
<212> PRT
<213> Oryctolagus cuniculus

<400> 107
Glu Ala Ser Thr Leu Ala Ser
1               5

<210> 108

<211> 11
<212> PRT
<213> Oryctolagus cuniculus

<400> 108
Leu Gly Gly Tyr Asp Asp Asp Gly Asp Asn Ala
1               5                   10


<210> 109
<211> 5
<212> PRT
<213> Oryctolagus cuniculus

<400> 109
Asn Tyr Tyr Met Thr
1               5


<210> 110
<211> 16
<212> PRT
<213> Oryctolagus cuniculus

<400> 110
Leu Ile Tyr Pro Asp Gly Ser Thr Phe Tyr Ala Asn Trp Ala Glu Gly
1               5                   10                  15


<210> 111
<211> 18
<212> PRT
<213> Oryctolagus cuniculus

<400> 111
Glu Gly Gly Ala Gly Asp Asn Thr Gly Thr Glu Tyr Tyr Tyr Gly Val
1               5                   10                  15
Asp Leu


<210> 112
<211> 13
<212> PRT
<213> Oryctolagus cuniculus

<400> 112
Gln Ala Ser Glu Asn Val Tyr Asp Lys Ser Ala Leu Ser
1               5                   10


<210> 113
<211> 7
<212> PRT
<213> Oryctolagus cuniculus

<400> 113
Leu Ala Ser Thr Leu Ala Ser
1               5


<210> 114
<211> 12

<212> PRT
<213> Oryctolagus cuniculus

<400> 114
Ala Gly Tyr Lys Ser Ser Ile Ile Asp Gly Thr Ala
1               5                   10

<210> 115
<211> 5
<212> PRT
<213> Oryctolagus cuniculus

<400> 115
Asn Asn Ala Leu Ser
1               5

<210> 116
<211> 16
<212> PRT
<213> Oryctolagus cuniculus

<400> 116
Ala Ile Gly Ala Gly Gly Asn Thr Tyr Tyr Ala Ser Trp Ala Lys Gly
1               5                   10                  15

<210> 117
<211> 7
<212> PRT
<213> Oryctolagus cuniculus

<400> 117
Gly Asp Leu Pro Gly Gly Ile
1               5

<210> 118
<211> 11
<212> PRT
<213> Oryctolagus cuniculus

<400> 118
Gln Ala Ser Gln Ser Ile Ser Pro Ala Leu Ala
1               5                   10

<210> 119
<211> 7
<212> PRT
<213> Oryctolagus cuniculus

<400> 119
Asp Val Ser Lys Leu Ala Ser
1               5

<210> 120
<211> 14
<212> PRT
<213> Oryctolagus cuniculus

<400> 120
Gln Ser Tyr Tyr Gly Ile Asn Ser Asn Ser Tyr Gly Asn Ile
1               5                   10


<210> 121
<211> 5
<212> PRT
<213> Oryctolagus cuniculus

<400> 121
Ser Tyr Ala Met Gly
1               5


<210> 122
<211> 16
<212> PRT
<213> Oryctolagus cuniculus

<400> 122
Tyr Ile Tyr Gly Asn Tyr Asn Lys Tyr Tyr Ala Ser Trp Ala Lys Gly
1               5                   10                  15


<210> 123
<211> 6
<212> PRT
<213> Oryctolagus cuniculus

<400> 123
Gly Gly Ala Met Asp Val
1               5


<210> 124
<211> 11
<212> PRT
<213> Oryctolagus cuniculus

<400> 124
Gln Ala Ser Glu Ser Ile Ser Thr Trp Leu Ala
1               5                   10


<210> 125
<211> 7
<212> PRT
<213> Oryctolagus cuniculus

<400> 125
Arg Ala Ser Thr Leu Ala Ser
1               5


<210> 126
<211> 12
<212> PRT
<213> Oryctolagus cuniculus

<400> 126
Gln Gln Gly Trp Asn Ile Asn Asn Ile Asp Asn Ile
1               5                   10

<210> 127
<211> 5
<212> PRT
<213> Oryctolagus cuniculus

<400> 127
Ser Tyr Ser Met Tyr
1               5


<210> 128
<211> 16
<212> PRT
<213> Oryctolagus cuniculus

<400> 128
Phe Ile Leu Ser Ala Thr Ala Val Ser Tyr Ala Thr Trp Ala Lys Gly
1               5                   10                  15


<210> 129
<211> 13
<212> PRT
<213> Oryctolagus cuniculus

<400> 129
Asp Arg Asp Gly Gly Thr Thr Leu Asp Gly Phe Asp Pro
1               5                   10


<210> 130
<211> 13
<212> PRT
<213> Oryctolagus cuniculus

<400> 130
Gln Ser Ser Gln Ser Val Val Asn Asn Asn Asn Leu Ala
1               5                   10


<210> 131
<211> 7
<212> PRT
<213> Oryctolagus cuniculus

<400> 131
Phe Ala Ser Thr Leu Ala Ser
1               5


<210> 132
<211> 12
<212> PRT
<213> Oryctolagus cuniculus

<400> 132
Gln Gly Thr Tyr Leu Ser Asp Asp Trp Ser Asp Ala
1               5                   10


<210> 133
<211> 5

<212> PRT
<213> Oryctolagus cuniculus

<400> 133
Ser Ser Val Met Asn
1               5


<210> 134
<211> 16
<212> PRT
<213> Oryctolagus cuniculus

<400> 134
Ala Ile Trp Ser Gly Gly Tyr Thr Tyr Tyr Ala Thr Trp Ala Lys Gly
1               5                   10                  15


<210> 135
<211> 12
<212> PRT
<213> Oryctolagus cuniculus

<400> 135
Gly Gln Phe Gly Ala Ser Gly Gly Gly Asp Val Leu
1               5                   10


<210> 136
<211> 13
<212> PRT
<213> Oryctolagus cuniculus

<400> 136
Gln Ser Ser Pro Ser Val Asp Asn Asn Asn Leu Leu Ser
1               5                   10


<210> 137
<211> 7
<212> PRT
<213> Oryctolagus cuniculus

<400> 137
Asp Ala Ser Thr Leu Ala Ser
1               5


<210> 138
<211> 13
<212> PRT
<213> Oryctolagus cuniculus

<400> 138
Val Ala Gly Tyr Gly Lys Arg Ser Arg Asp Ile Arg Val
1               5                   10


<210> 139
<211> 5
<212> PRT
<213> Oryctolagus cuniculus

<400> 139
Ser His Asn Met Gln
1               5

<210> 140
<211> 16
<212> PRT
<213> Oryctolagus cuniculus

<400> 140
Ile Ile Tyr Pro Ser Asn Asn Ala Tyr Tyr Ser Asn Trp Ala Lys Gly
1               5                   10                  15

<210> 141
<211> 6
<212> PRT
<213> Oryctolagus cuniculus

<400> 141
Asp Ile Asn Ser Ala Ile
1               5

<210> 142
<211> 11
<212> PRT
<213> Oryctolagus cuniculus

<400> 142
Gln Ala Ser Glu Ser Ile Tyr Ser Asn Leu Ala
1               5                   10

<210> 143
<211> 7
<212> PRT
<213> Oryctolagus cuniculus

<400> 143
Asp Ala Ser Asp Leu Ala Ser
1               5

<210> 144
<211> 12
<212> PRT
<213> Oryctolagus cuniculus

<400> 144
Gln Cys Ser Trp Gly Gly Ser Thr Tyr Gly Phe Ala
1               5                   10

<210> 145
<211> 5
<212> PRT
<213> Oryctolagus cuniculus

<400> 145
Ile Tyr Val Met Thr
1               5

<210> 146
<211> 16
<212> PRT
<213> Oryctolagus cuniculus

<400> 146
Ser Ile Asp Ala Asp Asp Ser Ala Tyr Tyr Ala Thr Trp Ala Thr Ser
1               5                   10                  15


<210> 147
<211> 11
<212> PRT
<213> Oryctolagus cuniculus

<400> 147
Gly Leu Tyr Ala Asn Gly Gly Pro Phe Thr Leu
1               5                   10


<210> 148
<211> 13
<212> PRT
<213> Oryctolagus cuniculus

<400> 148
Gln Ser Ser Pro Ser Val Tyr Asn Arg Asn Gln Leu Ser
1               5                   10


<210> 149
<211> 7
<212> PRT
<213> Oryctolagus cuniculus

<400> 149
Thr Ala Ser Thr Leu Ala Ser
1               5


<210> 150
<211> 12
<212> PRT
<213> Oryctolagus cuniculus

<400> 150
Gln Gly Tyr Tyr Asn Thr Gly Ser Asp Thr Tyr Ala
1               5                   10


<210> 151
<211> 5
<212> PRT
<213> Oryctolagus cuniculus

<400> 151
Asn Tyr Asp Met Ser
1               5


<210> 152
<211> 16

<212> PRT
<213> Oryctolagus cuniculus

<400> 152
Phe Ile Asn Thr Val Gly Tyr Ala Tyr Tyr Ala Ser Trp Ala Lys Gly
1               5                   10                  15


<210> 153
<211> 9
<212> PRT
<213> Oryctolagus cuniculus

<400> 153
Leu Asp Asn Tyr Tyr Thr Trp Gly Ile
1               5


<210> 154
<211> 11
<212> PRT
<213> Oryctolagus cuniculus

<400> 154
Gln Ala Ala Glu Asp Ile Tyr Ser Ser Leu Ala
1               5                   10


<210> 155
<211> 7
<212> PRT
<213> Oryctolagus cuniculus

<400> 155
Ala Ala Ser Ile Leu Ala Ser
1               5


<210> 156
<211> 12
<212> PRT
<213> Oryctolagus cuniculus

<400> 156
Gln Thr Asn Tyr Gly Ile Ser Ser Tyr Gly Ala Ala
1               5                   10


<210> 157
<211> 5
<212> PRT
<213> Oryctolagus cuniculus

<400> 157
Asn Lys Pro Ile Thr
1               5


<210> 158
<211> 16
<212> PRT
<213> Oryctolagus cuniculus

<400> 158
Trp Ile Ser Thr Thr Gly Ser Ala Tyr Tyr Ala Ser Trp Ala Lys Gly
1               5                   10                  15

<210> 159
<211> 10
<212> PRT
<213> Oryctolagus cuniculus

<400> 159
Tyr Ser Ser Asp Tyr Gly His His Asp Leu
1               5                   10

<210> 160
<211> 12
<212> PRT
<213> Oryctolagus cuniculus

<400> 160
Gln Ala Ser Gln Ser Val Tyr Arg Asp Tyr Leu Ser
1               5                   10

<210> 161
<211> 7
<212> PRT
<213> Oryctolagus cuniculus

<400> 161
Gly Ala Ser Asn Leu Ala Ser
1               5

<210> 162
<211> 10
<212> PRT
<213> Oryctolagus cuniculus

<400> 162
Leu Gly Gly Phe Ser Gly Asn Ile Asn Thr
1               5                   10

<210> 163
<211> 5
<212> PRT
<213> Oryctolagus cuniculus

<400> 163
Asn Tyr His Met Cys
1               5

<210> 164
<211> 16
<212> PRT
<213> Oryctolagus cuniculus

<400> 164
Ile Ile Ser Asn Thr Gly Tyr Thr Tyr Tyr Ser Ser Trp Ala Lys Gly
1               5                   10                  15

<210> 165
<211> 6
<212> PRT
<213> Oryctolagus cuniculus

<400> 165
Asp Arg Leu Ala Asn Leu
1               5

<210> 166
<211> 11
<212> PRT
<213> Oryctolagus cuniculus

<400> 166
Gln Ala Ser Gln Ser Ile Ser Thr Tyr Leu Lys
1               5                   10

<210> 167
<211> 7
<212> PRT
<213> Oryctolagus cuniculus

<400> 167
Ser Ala Ser Thr Leu Thr Ser
1               5

<210> 168
<211> 14
<212> PRT
<213> Oryctolagus cuniculus

<400> 168
Gln Ser Asn Ala Gly Ser Ser Ser Ser Ser Cys Gly Tyr Ala
1               5                   10

<210> 169
<211> 5
<212> PRT
<213> Oryctolagus cuniculus

<400> 169
Tyr Tyr Gly Met Gly
1               5

<210> 170
<211> 16
<212> PRT
<213> Oryctolagus cuniculus

<400> 170
Ile Ile Ser Gly Ile Gly Asn Thr Tyr Tyr Pro Thr Trp Ala Lys Gly
1               5                   10                  15

<210> 171
<211> 3

<212> PRT
<213> Oryctolagus cuniculus

<400> 171
Gly Asp Phe
1

<210> 172
<211> 11
<212> PRT
<213> Oryctolagus cuniculus

<400> 172
Gln Ala Ser Gln Ser Ile Ser Arg Tyr Leu Lys
1               5                   10

<210> 173
<211> 7
<212> PRT
<213> Oryctolagus cuniculus

<400> 173
Ser Ala Ser Thr Leu Thr Ser
1               5

<210> 174
<211> 14
<212> PRT
<213> Oryctolagus cuniculus

<400> 174
Gln Ser Asn Ala Gly Ser Ser Ser Ser Ser Cys Gly Tyr Ala
1               5                   10

<210> 175
<211> 5
<212> PRT
<213> Oryctolagus cuniculus

<400> 175
Tyr Tyr Gly Met Gly
1               5

<210> 176
<211> 16
<212> PRT
<213> Oryctolagus cuniculus

<400> 176
Ile Ile Ser Gly Ser Gly Asn Thr Tyr Tyr Ala Ser Trp Ala Lys Gly
1               5                   10                  15

<210> 177
<211> 3
<212> PRT
<213> Oryctolagus cuniculus

<400> 177
Gly Asp Phe
1

<210> 178
<211> 12
<212> PRT
<213> Oryctolagus cuniculus

<400> 178
Gln Ser Ser Gln Ser Val Tyr Ser Asn Tyr Leu Ser
 1               5                   10

<210> 179
<211> 7
<212> PRT
<213> Oryctolagus cuniculus

<400> 179
Val Ala Ser Ser Leu Ala Ser
 1               5

<210> 180
<211> 12
<212> PRT
<213> Oryctolagus cuniculus

<400> 180
Gly Gly Phe Gln Lys Tyr Ile Asp Asp Gly Gly Ala
 1               5                   10

<210> 181
<211> 5
<212> PRT
<213> Oryctolagus cuniculus

<400> 181
Thr Tyr Ala Met Ser
 1               5

<210> 182
<211> 16
<212> PRT
<213> Oryctolagus cuniculus

<400> 182
Ile Met Ser Ser Ser Gly Ser Ala Tyr Tyr Ala Ser Trp Ala Lys Gly
 1               5                   10                  15

<210> 183
<211> 6
<212> PRT
<213> Oryctolagus cuniculus

<400> 183
Ser Ser Ser Phe Gly Leu
 1               5

<210> 184
<211> 12
<212> PRT
<213> Mus musculus

<400> 184
Ser Val Ser Ser Ser Ile Ser Ser Thr Asn Leu His
1               5                   10


<210> 185
<211> 7
<212> PRT
<213> Mus musculus

<400> 185
Gly Thr Ser Asn Leu Ala Ser
1               5


<210> 186
<211> 8
<212> PRT
<213> Mus musculus

<400> 186
Gln Gln Trp Ser Thr Thr Tyr Thr
1               5


<210> 187
<211> 5
<212> PRT
<213> Mus musculus

<400> 187
Asp Tyr Tyr Ile His
1               5


<210> 188
<211> 17
<212> PRT
<213> Mus musculus

<400> 188
Arg Ile Asp Pro Asp Asn Gly Glu Ser Thr Tyr Val Pro Lys Phe Gln
1               5                   10                  15
Gly


<210> 189
<211> 13
<212> PRT
<213> Mus musculus

<400> 189
Glu Gly Leu Asp Tyr Gly Asp Tyr Tyr Ala Val Asp Tyr
1               5                   10

<210> 190
<211> 15
<212> PRT
<213> Mus musculus

<400> 190
Arg Ala Ser Glu Ser Val Asp Ser Tyr Gly Asn Ser Phe Met His
1               5                   10                  15


<210> 191
<211> 7
<212> PRT
<213> Mus musculus

<400> 191
Arg Ala Ser Asn Leu Glu Ser
1               5


<210> 192
<211> 9
<212> PRT
<213> Mus musculus

<400> 192
Gln Gln Asn Tyr Glu Asp Pro Leu Thr
1               5


<210> 193
<211> 5
<212> PRT
<213> Mus musculus

<400> 193
Asn Tyr Leu Ile Glu
1               5


<210> 194
<211> 17
<212> PRT
<213> Mus musculus

<400> 194
Val Ile Asn Pro Gly Ser Gly Ile Ile Asn Tyr Asn Glu Lys Phe Lys
1               5                   10                  15
Ile


<210> 195
<211> 17
<212> PRT
<213> Mus musculus

<400> 195
Asp Trp Asp Thr Phe Tyr Ser Tyr Glu Arg Glu Val Tyr Ala Met Asp
1               5                   10                  15
Tyr

<210> 196
<211> 11
<212> PRT
<213> Mus musculus

<400> 196
Arg Ala Ser Gln Ser Ile Gly Thr Asn Ile His
1               5                   10


<210> 197
<211> 7
<212> PRT
<213> Mus musculus

<400> 197
Phe Ala Ser Glu Ser Ile Ser
1               5


<210> 198
<211> 9
<212> PRT
<213> Mus musculus

<400> 198
Gln Gln Ser Asn Ser Trp Pro Leu Thr
1               5


<210> 199
<211> 5
<212> PRT
<213> Mus musculus

<400> 199
Asp Tyr Tyr Val His
1               5


<210> 200
<211> 17
<212> PRT
<213> Mus musculus

<400> 200
Trp Ile Asp Pro Glu Asn Gly Asp Thr Glu Tyr Ala Pro Lys Phe Gln
1               5                   10                  15
Gly


<210> 201
<211> 9
<212> PRT
<213> Mus musculus

<400> 201
Gly Tyr Gly Asn Phe Tyr Phe Asp Tyr
1               5


<210> 202

<211> 11
<212> PRT
<213> Mus musculus

<400> 202
Lys Ala Ser Gln Asp Val Gly Thr Ala Val Ala
1               5                   10


<210> 203
<211> 7
<212> PRT
<213> Mus musculus

<400> 203
Trp Ala Ser Thr Arg His Thr
1               5


<210> 204
<211> 9
<212> PRT
<213> Mus musculus

<400> 204
Gln Gln Tyr Ser Ser Tyr Pro Tyr Thr
1               5


<210> 205
<211> 5
<212> PRT
<213> Mus musculus

<400> 205
Asp Tyr Phe Met His
1               5


<210> 206
<211> 17
<212> PRT
<213> Mus musculus

<400> 206
Trp Ile Asp Pro Glu Asn Gly Asp Thr Glu Tyr Ala Pro Lys Phe Gln
1               5                   10                  15
Gly


<210> 207
<211> 9
<212> PRT
<213> Mus musculus

<400> 207
Gly Gln Ala Thr Tyr Tyr Phe Asp Tyr
1               5


<210> 208
<211> 11

<212> PRT
<213> Mus musculus

<400> 208
His Ala Thr Gln Asn Ile Asn Val Trp Leu Ser
1               5                   10


<210> 209
<211> 7
<212> PRT
<213> Mus musculus

<400> 209
Lys Thr Ser Asn Leu His Thr
1               5


<210> 210
<211> 9
<212> PRT
<213> Mus musculus

<400> 210
Gln Gln Gly Gln Ser Phe Pro Tyr Thr
1               5


<210> 211
<211> 5
<212> PRT
<213> Mus musculus

<400> 211
Asp Tyr Phe Met His
1               5


<210> 212
<211> 17
<212> PRT
<213> Mus musculus

<400> 212
Trp Ile Asp Pro Glu Asn Gly Asp Thr Glu Tyr Ala Pro Lys Phe Gln
1               5                   10                  15
Gly


<210> 213
<211> 9
<212> PRT
<213> Mus musculus

<400> 213
Gly Gln Ala Thr Tyr Tyr Phe Asp Tyr
1               5


<210> 214
<211> 17
<212> PRT

<213> Mus musculus

<400> 214
Lys Ser Ser Gln Ser Leu Leu Tyr Ser Asn Asn Gln Lys Asn Tyr Leu
1               5                   10                  15
Ala

<210> 215
<211> 7
<212> PRT
<213> Mus musculus

<400> 215
Trp Ala Ser Thr Arg Glu Ser
1               5

<210> 216
<211> 9
<212> PRT
<213> Mus musculus

<400> 216
Gln Gln Tyr Tyr Asp Tyr Pro Trp Thr
1               5

<210> 217
<211> 5
<212> PRT
<213> Mus musculus

<400> 217
Asp Tyr Phe Met His
1               5

<210> 218
<211> 17
<212> PRT
<213> Mus musculus

<400> 218
Trp Ile Asp Pro Glu Asn Gly Asp Thr Glu Tyr Ala Pro Lys Phe Gln
1               5                   10                  15
Gly

<210> 219
<211> 9
<212> PRT
<213> Mus musculus

<400> 219
Gly Gln Ala Thr Tyr Tyr Phe Asp Tyr
1               5

<210> 220
<211> 11

<212> PRT
<213> Mus musculus

<400> 220
Arg Thr Ser Gln Asp Ile Asn Asn Phe Leu Asn
1               5                   10


<210> 221
<211> 7
<212> PRT
<213> Mus musculus

<400> 221
His Thr Ser Arg Leu Lys Ser
1               5


<210> 222
<211> 9
<212> PRT
<213> Mus musculus

<400> 222
Gln His Tyr Tyr Asn Leu Pro Trp Thr
1               5


<210> 223
<211> 5
<212> PRT
<213> Mus musculus

<400> 223
Asp Asn Ile Met Val
1               5


<210> 224
<211> 17
<212> PRT
<213> Mus musculus

<400> 224
Asn Ile Asn Pro Tyr Tyr Gly Ser Pro Ser Tyr Asn Leu Lys Phe Lys
1               5                   10                  15
Asp


<210> 225
<211> 10
<212> PRT
<213> Mus musculus

<400> 225
Glu Gly Gly Asn Tyr Gly Ser Leu Asp Asn
1               5                   10


<210> 226
<211> 17
<212> PRT

<213> Mus musculus

<400> 226
Lys Ser Ser Gln Ser Leu Leu Tyr Ser Asn Asn Gln Lys Asn Tyr Leu
1               5                   10                  15
Ala

<210> 227
<211> 7
<212> PRT
<213> Mus musculus

<400> 227
Trp Ala Ser Thr Arg Glu Ser
1               5

<210> 228
<211> 9
<212> PRT
<213> Mus musculus

<400> 228
Gln Gln Tyr Tyr Asp Tyr Pro Trp Thr
1               5

<210> 229
<211> 5
<212> PRT
<213> Mus musculus

<400> 229
Asp Asn Ile Met Val
1               5

<210> 230
<211> 17
<212> PRT
<213> Mus musculus

<400> 230
Asn Ile Asn Pro Tyr Tyr Gly Ser Pro Ser Tyr Asn Leu Lys Phe Lys
1               5                   10                  15
Asp

<210> 231
<211> 10
<212> PRT
<213> Mus musculus

<400> 231
Glu Gly Gly Asn Tyr Gly Ser Leu Asp Asn
1               5                   10

<210> 232
<211> 11

<212> PRT
<213> Mus musculus

<400> 232
Arg Thr Ser Gln Asp Ile Asn Asn Phe Leu Asn
1               5                   10


<210> 233
<211> 7
<212> PRT
<213> Mus musculus

<400> 233
His Thr Ser Arg Leu Lys Ser
1               5


<210> 234
<211> 9
<212> PRT
<213> Mus musculus

<400> 234
Gln His Tyr Tyr Asn Leu Pro Trp Thr
1               5


<210> 235
<211> 5
<212> PRT
<213> Mus musculus

<400> 235
Tyr Tyr Tyr Met Ser
1               5


<210> 236
<211> 16
<212> PRT
<213> Mus musculus

<400> 236
Thr Ile Ser Ile Asp Gly Ser Thr Tyr Tyr Ala Ser Trp Ala Glu Gly
1               5                   10                  15


<210> 237
<211> 9
<212> PRT
<213> Mus musculus

<400> 237
Gly His Ile Asn Thr Gly Met Asp Pro
1               5


<210> 238
<211> 16
<212> PRT
<213> Mus musculus

<400> 238
Arg Ser Ser Gln Thr Ile Val His Ser Asn Gly Asn Thr Tyr Leu Glu
1               5                   10                  15

<210> 239
<211> 7
<212> PRT
<213> Mus musculus

<400> 239
Glu Val Ser Asn Arg Phe Ser
1               5

<210> 240
<211> 9
<212> PRT
<213> Mus musculus

<400> 240
Phe Gln Gly Ser His Phe Pro His Thr
1               5

<210> 241
<211> 5
<212> PRT
<213> Mus musculus

<400> 241
Asp Tyr Tyr Met Tyr
1               5

<210> 242
<211> 17
<212> PRT
<213> Mus musculus

<400> 242
Trp Ile Asp Pro Glu Asn Gly Asp Thr Glu Cys Ala Pro Lys Phe Gln
1               5                   10                  15
Gly

<210> 243
<211> 15
<212> PRT
<213> Mus musculus

<400> 243
Asp Arg Tyr Asp Glu Gly Ala Ala Ser Asp Tyr Gly Met Asp Tyr
1               5                   10                  15

<210> 244
<211> 16
<212> PRT
<213> Mus musculus

<400> 244

Arg Ser Ser Gln Ser Ile Val His Ser Asn Gly Asn Thr His Leu Glu
1               5                   10                  15

<210> 245
<211> 7
<212> PRT
<213> Mus musculus

<400> 245
Lys Val Ser Asn Arg Phe Ser
1               5

<210> 246
<211> 9
<212> PRT
<213> Mus musculus

<400> 246
Phe Gln Gly Ser His Ala Pro His Thr
1               5

<210> 247
<211> 5
<212> PRT
<213> Mus musculus

<400> 247
Asp Tyr Tyr Met Tyr
1               5

<210> 248
<211> 17
<212> PRT
<213> Mus musculus

<400> 248
Trp Ile Asp Pro Gly Asn Gly Asp Thr Glu Cys Ala Pro Lys Phe Gln
1               5                   10                  15
Gly

<210> 249
<211> 15
<212> PRT
<213> Mus musculus

<400> 249
Asp Arg Tyr Asp Glu Gly Ala Ala Ser Asp Tyr Ala Val Asp Tyr
1               5                   10                  15

<210> 250
<211> 16
<212> PRT
<213> Mus musculus

<400> 250
Arg Ser Ser Gln Thr Ile Val His Ser Asn Gly Asn Thr Tyr Leu Glu

 1               5                   10                  15

<210> 251
<211> 7
<212> PRT
<213> Mus musculus

<400> 251
Glu Val Ser Asn Arg Phe Ser
 1               5

<210> 252
<211> 9
<212> PRT
<213> Mus musculus

<400> 252
Phe Gln Gly Ser Leu Phe Pro His Thr
 1               5

<210> 253
<211> 5
<212> PRT
<213> Mus musculus

<400> 253
Asp Tyr Tyr Met Tyr
 1               5

<210> 254
<211> 17
<212> PRT
<213> Mus musculus

<400> 254
Trp Ile Asp Pro Glu Asn Gly Asp Thr Glu Cys Ala Pro Lys Phe Gln
 1               5                   10                  15
Gly

<210> 255
<211> 15
<212> PRT
<213> Mus musculus

<400> 255
Asp Arg Tyr Asp Glu Gly Ala Ala Ser Asp Tyr Ala Val Asp Tyr
 1               5                   10                  15

## Description

### TECHNICAL FIELD

The present invention relates generally to epitopes of sclerostin protein, including human sclerostin protein, and binding agents (such as antibodies) capable of binding to sclerostin or fragments thereof.

### BACKGROUND OF THE INVENTION

Two or three distinct phases of changes to bone mass occur over the life of an individual (*see* Riggs, West J. Med., 154:63-77 (1991)). The first phase occurs in both men and women and proceeds to attainment of a peak bone mass. This first phase is achieved through linear growth of the endochondral growth plates and radial growth due to a rate of periosteal apposition. The second phase begins around age 30 for trabecular bone (flat bones such as the vertebrae and pelvis) and about age 40 for critical bone (*e.g*., long bones found in the limbs) and continues to old age. This phase is characterized by slow bone loss and occurs in both men and women. In women, a third phase of bone loss also occurs, most likely due to postmenopausal estrogen deficiencies. During this phase alone, women may lose an additional bone mass from the critical bone and from the trabecular compartment (*see* Riggs, *supra*).

Loss of bone mineral content can be caused by a wide variety of conditions and may result in significant medical problems. For example, osteoporosis is a debilitating disease in humans and is characterized by marked decreases in skeletal bone mass and mineral density, structural deterioration of bone, including degradation of bone microarchitecture and corresponding increases in bone fragility (*i.e.,* decreases in bone strength), and susceptibility to fracture in afflicted individuals. Osteoporosis in humans is generally preceded by clinical osteopenia (bone mineral density that is greater than one standard deviation but less than 2.5 standard deviations below the mean value for young adult bone), a condition found in approximately 25 million people in the United States. Another 7-8 million patients in the United States have been diagnosed with clinical osteoporosis (defined as bone mineral content greater than 2.5 standard deviations below that of mature young adult bone). The frequency of osteoporosis in the human population increases with age. Among Caucasian, osteoporosis is predominant in women who, in the United States, comprise 80% of the osteoporosis patient pool. The increased fragility and susceptibility to fracture of skeletal bone in the aged is aggravated by the greater risk of accidental falls in this population. Fractured hips, wrists, and vertebrae are among the most common injuries associated with osteoporosis. Hip fractures in particular are extremely uncomfortable and expensive for the patient, and for women, correlate with high rates of mortality and morbidity.

Although osteoporosis has been regarded as an increase in the risk of fracture due to decreased bone mass, few of the presently available treatments for skeletal disorders can increase the bone density of adults, and most of the presently available treatments work primarily by inhibiting further bone resorption rather than stimulating new bone formation. Estrogen is now being prescribed to retard bone loss. However, some controversy exists over whether patients gain any long-term benefit and whether estrogen has any effect on patients over 75 years old. Moreover, use of estrogen is believed to increase the risk of breast and endometrial cancer. Calcitonin, osteocalcin with vitamin K, or high doses of dietary calcium, with or without vitamin D, have also been suggested for postmenopausal women. High doses of calcium, however, often have undesired gastrointestinal side effects, and serum and urinary calcium levels must be continuously monitored (*e.g*., Khosla and Riggs, Mayo Clin. Proc., 70:978982 (1995)).

Other current therapeutic approaches to osteoporosis include bisphosphonates (*e.g*., Fosamax^{™}, Actonel^{™}, Bonviva^{™}, Zometa^{™}, olpadronate, neridronate, skelid, bonefos), parathyroid hormone, calcilytics, calcimimetics (*e.g*., cinacalcet), statins, anabolic steroids, lanthanum and strontium salts, and sodium fluoride. Such therapeutics, however, are often associated with undesirable side effects *(see* Khosla and Riggs, *supra*).

Sclerostin, the product of the SOST gene, is absent in sclerosteosis, a skeletal disease characterized by bone overgrowth and strong dense bones (Brunkow et al., Am. J. Hum. Genet., 68:577-589 (2001); Balemans et al., Hum. Mal. Genet., 10:537-543 (2001)). The amino acid sequence of human sclerostin is reported by Brunkow *et al. ibid* and is disclosed herein as SEQ ID NO:1.

### BRIEF SUMMARY OF THE INVENTION

The invention relates to an isolated antibody selected from the group consisting of antibodies A, B, C, D, E, F, G, H, I, J, K, L, M, N, O, P, Q, R, S, T, U, V, W, X, and Y; the isolated antibody, or an antigen-binding fragment thereof, may be a polyclonal antibody, a monoclonal antibody, a humanized antibody, a human antibody, or a human/non-human chimeric antibody, such as a mouse/human or rabbit/human chimeric antibody.

The invention further relates to a methods for detecting, diagnosing, and determining the progression or regression of a bone disorder associated with at least one of low bone mass, low bone mineral density, and poor bone quality in a mammalian subject which comprises obtaining a biological sample from a subject suspected of suffering from the disorder, contacting the biological sample with an agent capable of detecting sclerostin, and identifying or quantitating a binding complex between the agent and sclerostin, wherein the agent comprises an anti-sclerostin antibody, or sclerostin-binding fragment thereof.

Provided herein are antibodies that specifically bind to human sclerostin. The antibodies can be characterized by their ability to bind to human sclerostin or a fragment thereof.

Also provided is an isolated antibody, or an antigen-binding fragment thereof, that specifically binds to human sclerostin and has at least one CDR sequence selected from SEQ ID NOs: 106-255 and variants thereof.

These and other aspects of the present invention will become apparent upon reference to the following detailed description and attached drawings. All references disclosed herein are hereby incorporated by reference in their entireties as if each was incorporated individually.

### BRIEF DESCRIPTION OF THE DRAWINGS

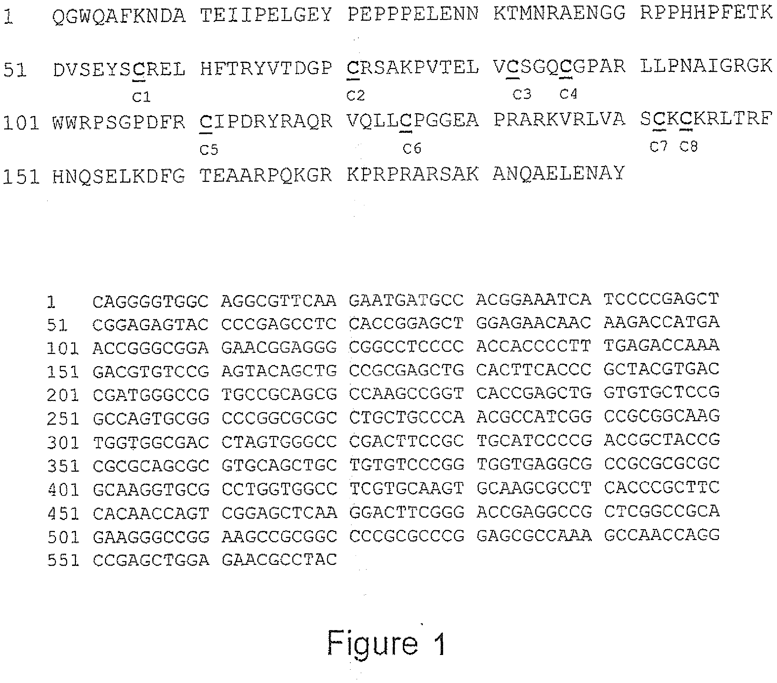

Figure 1 depicts the amino acid sequence of the mature form (signal peptide cleaved off) of human sclerostin (SEQ ID NO:1). Also depicted is the nucleotide sequence of the human sclerostin coding region that encodes the mature form of human sclerostin. The eight cysteines are numbered C1 through C8. The cystine-knot is formed by three disulfide bonds (C1-C5; C3-C7; C4-C8). C2 and C6 also form a disulfide bond, however this disulfide is not part of the cystine-knot.

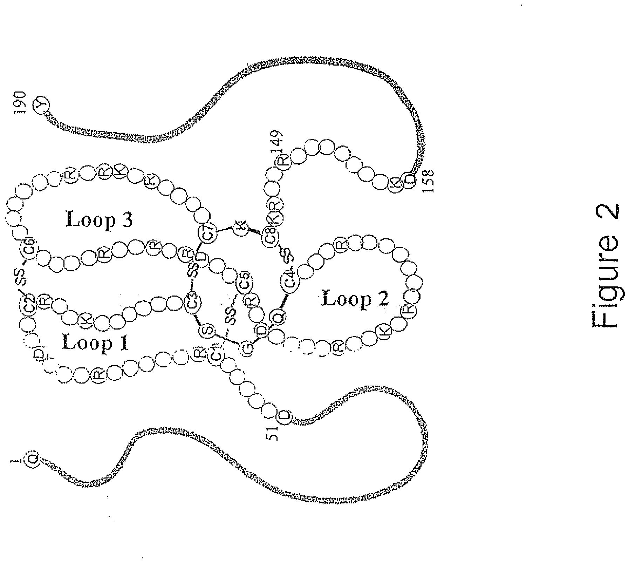

Figure 2 depicts a schematic of the basic structure of human sclerostin. There is an N-terminal arm (from the first Q to C1) and a C-terminal arm (from C8 to the terminal Y). In between these arms there is the cystine-knot structure (formed by three disulfides: C1-C5; C3-C7; C4-C8) and three loops which are designated Loop 1, Loop 2 and Loop 3. The distal regions of Loop 1 and Loop 3 are linked by the C2-C6 disulfide. Potential trypsin cleavage sites are indicated (arginine=R and lysine=K). Some of the potential AspN cleavage sites are indicated [only aspartic acid (D) residues are shown].

### DETAILED DESCRIPTION

The present invention relates to regions of the human sclerostin protein that contain epitopes recognized by antibodies that also bind to full-length sclerostin, and methods of making and using these epitopes. The invention also provides binding agents (such as antibodies) that specifically bind to sclerostin or portions of sclerostin, and methods for using such binding agents. The binding agents are useful to block or impair binding of human sclerostin to one or more ligand.

Recombinant human sclerostin/SOST is commercially available from R&D Systems (Minneapolis, MN, USA; 2006 cat# 1406-ST-025). Additionally, recombinant mouse sclerostin/SOST is commercially available from R&D Systems (Minneapolis, MN, USA; 2006 cat# 1589-ST-025). Research grade sclerostin binding monoclonal antibodies are commercially available from R&D Systems (Minneapolis, MN, USA; mouse monoclonal: 2006 cat# MAB1406; rat monoclonal: 2006 cat# MAB1589). U.S. Patent Nos. 6,395,511 and 6,803,453, and U.S. Patent Publications 2004/0009535 and 2005/0106683 refer to anti-sclerostin antibodies generally.

As used herein, the term human sclerostin is intended to include the protein of SEQ ID NO:1 and allelic variants thereof. Sclerostin can be purified from 293T host cells that have been transfected by a gene encoding sclerostin by elution of filtered supernatant of host cell culture fluid using a Heparin HP column, using a salt gradient. The preparation and further purification using cation exchange chromatography are described in Examples 1 and 2.

Binding agents of the invention are preferably antibodies, as defined herein. The term "antibody" refers to an intact antibody, or a binding fragment thereof. An antibody may comprise a complete antibody molecule (including polyclonal, monoclonal, chimeric, humanized, or human versions having full length heavy and/or light chains), or comprise an antigen binding fragment thereof. Antibody fragments include F(ab')_{2,} Fab, Fab', Fv, Fc, and Fd fragments, and can be incorporated into single domain antibodies, single-chain antibodies, maxibodies, minibodies, intrabodies, diabodies, triabodies, tetrabodies, v-NAR and bis-scFv (*see e.g.*, Hollinger and Hudson, Nature Biotechnology, 23(9):1126-1136 (2005)). Antibody polypeptides are also disclosed in U.S. Patent No. 6,703,199, including fibronectin polypeptide monobodies. Other antibody polypeptides are disclosed in U.S. Patent Publication 2005/0238646, which are single-chain polypeptides.

Antigen binding fragments derived from an antibody can be obtained, for example, by proteolytic hydrolysis of the antibody, for example, pepsin or papain digestion of whole antibodies according to conventional methods. By way of example, antibody fragments can be produced by enzymatic cleavage of antibodies with pepsin to provide a 5S fragment termed F(ab')₂. This fragment can be further cleaved using a thiol reducing agent to produce 3.5S Fab' monovalent fragments. Optionally, the cleavage reaction can be performed using a blocking group for the sulfhydryl groups that result from cleavage of disulfide linkages. As an alternative, an enzymatic cleavage using papain produces two monovalent Fab fragments and an Fc fragment directly. These methods are described, for example, by Goldenberg, U.S. Patent No. 4,331,647, Nisonoff et al., Arch. Biochem. Biophys., 89:230 (1960); Porter, Biochem. J., 73:119 (1959); Edelman et al., in Methods in Enzymology, 1:422 (Academic Press 1967); and by Andrews, S.M, and Titus, J.A. in Current Protocols in Immunology (Coligan J.E., et al., eds), John Wiley & Sons, New York (2003), pages 2.8.1-2.8.10 and 2.10A.1-2.10A.5. Other methods for cleaving antibodies, such as separating heavy chains to form monovalent light-heavy chain fragments (Fd), further cleaving of fragments, or other enzymatic, chemical, or genetic techniques may also be used, so long as the fragments bind to the antigen that is recognized by the intact antibody.

An antibody fragment may also be any synthetic or genetically engineered protein. For example, antibody fragments include isolated fragments consisting of the light chain variable region, "Fv" fragments consisting of the variable regions of the heavy and light chains, and recombinant single chain polypeptide molecules in which light and heavy variable regions are connected by a peptide linker (scFv proteins).

Another form of an antibody fragment is a peptide comprising one or more complementarity determining regions (CDRs) of an antibody. CDRs (also termed "minimal recognition units," or "hypervariable region") can be obtained by constructing polynucleotides that encode the CDR of interest. Such polynucleotides are prepared, for example, by using the polymerase chain reaction to synthesize the variable region using mRNA of antibody-producing cells as a template (*see,* for example, Larrick et al., Methods: A Companion to Methods in Enzymology 2:106, 1991; Courtenay-Luck, "Genetic Manipulation of Monoclonal Antibodies," in Monoclonal Antibodies: Production, Engineering and Clinical Application, Ritter et al. (eds.), page 166 (Cambridge University Press 1995); and Ward et al., "Genetic Manipulation and Expression of Antibodies," in Monoclonal Antibodies: Principles and Applications, Birch et al., (eds.), page 137 (Wiley-Liss, Inc, 1995)).

Thus, in one embodiment, the binding agent comprises at least one CDR as described wherein. The binding agent may comprise at least two, three, four, five or six CDR's as described herein. The binding agent further may comprise at least one variable region domain of an antibody described herein. The variable region domain may be of any size or amino acid composition and will generally comprise at least one CDR sequence responsible for binding to human sclerostin, for example CDR-H1, CDR-H2, CDR-H3 and/or the light chain CDRs specifically described herein and which is adjacent to or in frame with one or more framework sequences. In general terms, the variable (V) region domain may be any suitable arrangement of immunoglobulin heavy (V_{H}) and/or light (V_{L}) chain variable domains, Thus, for example, the V region domain may be monomeric and be a V_{H} or V_{L} domain, which is capable of independently binding human sclerostin with an affinity at least equal to 1 x 10⁻⁷M or less as described below. Alternatively, the V region domain may be dimeric and contain V_{H}-V_{H}, V_{H}-V_{L}, or V_{L}-V_{L}, dimers. The V region dimer comprises at least one V_{H} and at least one V_{L} chain that may be non-covalently associated (hereinafter referred to as Fᵥ). If desired, the chains may be covalently coupled either directly, for example, via a disulfide bond between the two variable domains, or through a linker, for example, a peptide linker, to form a single chain Fv (scFv).

The variable region domain may be any naturally occurring variable domain or an engineered version thereof. By engineered version is meant a variable region domain that has been created using recombinant DNA engineering techniques. Such engineered versions include those created, for example, from a specific antibody variable region by insertions, deletions, or changes in or to the amino acid sequences of the specific antibody. Particular examples include engineered variable region domains containing at least one CDR and optionally one or more framework amino acids from a first antibody and the remainder of the variable region domain from a second antibody.

The variable region domain may be covalently attached at a C-terminal amino acid to at least one other antibody domain or a fragment thereof. Thus, for example, a V_{H} domain that is present in the variable region domain may be linked to an immunoglobulin CH1 domain, or a fragment thereof. Similarly a V_{L} domain may be linked to a C_{K} domain or a fragment thereof, In this way, for example, the antibody may be an Fab fragment wherein the antigen binding domain contains associated V_{H} and V_{L} domains covalently linked at their C-termini to a CH1 and C_{K} domain, respectively. The CH1 domain may be extended with further amino acids, for example, to provide a hinge region or a portion of a hinge region domain as found in a Fab' fragment, or to provide further domains, such as antibody CH2 and CH3 domains.

As described herein, binding agents comprise at least one of these CDRs, For example, one or more CDR may be incorporated into known antibody framework regions (IgG1, IgG2, etc.), or conjugated to a suitable vehicle to enhance the half-life thereof. Suitable vehicles include, but are not limited to Fc, polyethylene glycol (PEG), albumin, transferrin, and the like. These and other suitable vehicles are known in the art. Such conjugated CDR peptides may be in monomeric, dimeric, tetrameric, or other form. In one embodiment, one or more water-soluble polymer is bonded at one or more specific position, for example at the amino terminus, of a binding agent.

In certain preferred embodiments, a binding agent comprises one or more water soluble polymer attachments, including, but not limited to, polyethylene glycol, polyoxyethylene glycol, or polypropylene glycol. *See, e.g.,* U.S. Patent Nos. 4,640,835, 4,496,689, 4,301,144, 4,670,417, 4,791,192 and 4,179,337. In certain embodiments, a derivative binding agent comprises one or more of mono methoxy-polyethylene glycol, dextran, cellulose, or other carbohydrate based polymers, poly-(N-vinyl pyrrolidone)-polyethylene glycol, propylene glycol homopolymers, a polypropylene oxide/ethylene oxide co-polymer, polyoxyethylated polyols (*e.g.,* glycerol) and polyvinyl alcohol, as well as mixtures of such polymers, In certain embodiments, one or more water-soluble polymers are randomly attached to one or more side chains. In certain embodiments, PEG can act to improve the therapeutic capacity for a binding agent, such as an antibody. Certain such methods are discussed, for example, in U.S. Patent No. 6,133,426, which is hereby incorporated by reference for any purpose.

It will be appreciated that a binding agent of the present invention may have at least one amino acid substitution, providing that the binding agent retains binding specificity. Therefore, modifications to the binding agent structures are encompassed within the scope of the invention. These may include amino acid substitutions, which may be conservative or non-conservative, that do not destroy the sclerostin binding capability of a binding agent. Conservative amino acid substitutions may encompass non-naturally occurring amino acid residues, which are typically incorporated by chemical peptide synthesis rather than by synthesis in biological systems. These include peptidomimetics and other reversed or inverted forms of amino acid moieties. A conservative amino acid substitution may also involve a substitution of a native amino acid residue with a normative residue such that there is little or no effect on the polarity or charge of the amino acid residue at that position.

Non-conservative substitutions may involve the exchange of a member of one class of amino acids or amino acid mimetics for a member from another class with different physical properties (*e.g*., size, polarity, hydrophobicity, and/or charge). Such substituted residues may be introduced into regions of the human antibody that are homologous with non-human antibodies, or into the non-homologous regions of the molecule.

Moreover, one skilled in the art may generate test variants containing a single amino acid substitution at each desired amino acid residue. The variants can then be screened using activity assays known to those skilled in the art. Such variants could be used to gather information about other suitable variants. For example, if one discovered that a change to a particular amino acid residue resulted in destroyed, undesirably reduced, or unsuitable activity, variants with such a change may be avoided. In other words, based on information gathered from such routine experiments, one skilled in the art can readily determine the amino acids where further substitutions should be avoided either alone or in combination with other mutations.

A skilled artisan will be able to determine suitable variants of the polypeptide as set forth herein using well-known techniques. In certain embodiments, one skilled in the art may identify suitable areas of the molecule that may be changed without destroying activity by targeting regions not believed to be important for activity. In certain embodiments, one can identify residues and portions of the molecules that are conserved among similar polypeptides. In certain embodiments, even areas that may be important for biological activity or for structure may be subject to conservative amino acid substitutions without destroying the biological activity or without adversely affecting the polypeptide structure.

Additionally, one skilled in the art can review structure-function studies identifying residues in similar polypeptides that are important for activity or structure, In view of such a comparison, one can predict the importance of amino acid residues in a protein that correspond to amino acid residues which are important for activity or structure in similar proteins. One skilled in the art may opt for chemically similar amino acid substitutions for such predicted important amino acid residues.

One skilled in the art can also analyze the three-dimensional structure and amino acid sequence in relation to that structure in similar polypeptides. In view of such information, one skilled in the art may predict the alignment of amino acid residues of an antibody with respect to its three dimensional structure. In certain embodiments, one skilled in the art may choose not to make radical changes to amino acid residues predicted to be on the surface of the protein, since such residues may be involved in important interactions with other molecules.

A number of scientific publications have been devoted to the prediction of secondary structure. *See* Moult J., Curr. Op. in Biotech., 7(4):422-427 (1996), Chou et al., Biochemistry, 13(2):222-245 (1974); Chou et al., Biochemistry, 113(2):211-222 (1974); Chou et al., Adv. Enzymol. Relat. Areas Mol. Biol., 47:45-148 (1978); Chou et al., Ann. Rev. Biochem., 47:251-276 and Chou et al., Biophys. J., 26:367-384 (1979). Moreover, computer programs are currently available to assist with predicting secondary structure. One method of predicting secondary structure is based upon homology modeling. For example, two polypeptides or proteins which have a sequence identity of greater than 30%, or similarity greater than 40% often have similar structural topologies. The recent growth of the protein structural database (PDB) has provided enhanced predictability of secondary structure, including the potential number of folds within a polypeptide's or protein's structure. *See* Holm et al., Nucl. Acid, Res., 27(1):244-247 (1999). It has been suggested (Brenner et al., Curr. Op. Struct. Biol., 7(3):369-376 (1997)) that there are a limited number of folds in a given polypeptide or protein and that once a critical number of structures have been resolved, structural prediction will become dramatically more accurate.

Additional methods of predicting secondary structure include "threading" (Jones, D., Curr. Opin. Struct. Biol., 7(3):377-87 (1997); Sippl et al., Structure, 4(1):15-19 (1996)), "profile analysis" (Bowie et al., Science, 253:164-170 (1991); Gribskov et al., Meth. Enzym., 183:146-159 (1990); Gribskov et al., Proc. Nat. Acad. Sci., 84(13):4355-4358 (1987)), and "evolutionary linkage" (*See* Holm, *supra* (1999), and Brenner, *supra* (1997)).

In certain embodiments, variants of binding agents include glycosylation variants wherein the number and/or type of glycosylation site has been altered compared to the amino acid sequences of a parent polypeptide. In certain embodiments, variants comprise a greater or a lesser number of N-linked glycosylation sites than the native protein. An N-linked glycosylation site is characterized by the sequence: Asn-X-Ser or Asn-X-Thr, wherein the amino acid residue designated as X may be any amino acid residue except proline. The substitution of amino acid residues to create this sequence provides a potential new site for the addition of an N-linked carbohydrate chain. Alternatively, substitutions which eliminate this sequence will remove an existing N-linked carbohydrate chain. Also provided is a rearrangement of N-linked carbohydrate chains wherein one or more N-linked glycosylation sites (typically those that are naturally occurring) are eliminated and one or more new N-linked sites are created. Additional preferred antibody variants include cysteine variants wherein one or more cysteine residues are deleted from or substituted for another amino acid (*e.g.,* serine) as compared to the parent amino acid sequence. Cysteine variants may be useful when antibodies must be refolded into a biologically active conformation such as after the isolation of insoluble inclusion bodies. Cysteine variants generally have fewer cysteine residues than the native protein, and typically have an even number to minimize interactions resulting from unpaired cysteines.

Desired amino acid substitutions (whether conservative or non-conservative) can be determined by those skilled in the art at the time such substitutions are desired. In certain embodiments, amino acid substitutions can be used to identify important residues of antibodies to sclerostin, or to increase or decrease the affinity of the antibodies to sclerostin described herein.

According to certain embodiments, preferred amino acid substitutions are those which: (1) reduce susceptibility to proteolysis, (2) reduce susceptibility to oxidation, (3) alter binding affinity for forming protein complexes, (4) alter binding affinities, and/or (5) confer or modify other physiochemical or functional properties on such polypeptides. According to certain embodiments, single or multiple amino acid substitutions (in certain embodiments, conservative amino acid substitutions) may be made in the naturally-occurring sequence (in certain embodiments, in the portion of the polypeptide outside the domain(s) forming intermolecular contacts). In certain embodiments, a conservative amino acid substitution typically may not substantially change the structural characteristics of the parent sequence (*e.g*., a replacement amino acid should not tend to break a helix that occurs in the parent sequence, or disrupt other types of secondary structure that characterizes the parent sequence). Examples of art-recognized polypeptide secondary and tertiary structures are described in Proteins, Structures and Molecular Principles (Creighton, Ed., W. H. Freeman and Company, New York (1984)); Introduction to Protein Structure (C. Branden and J. Tooze, eds., Garland Publishing, New York, N.Y. (1991)); and Thornton et al., Nature, 354:105 (1991), which are each incorporated herein by reference.

In certain embodiments, binding agents of the invention may be chemically bonded with polymers, lipids, or other moieties.

The binding agents may comprise at least one of the CDRs described herein incorporated into a biocompatible framework structure. In one example, the biocompatible framework structure comprises a polypeptide or portion thereof that is sufficient to form a conformationally stable structural support, or framework, or scaffold, which is able to display one or more sequences of amino acids that bind to an antigen (*e.g*., CDRs, a variable region, etc.) in a localized surface regions. Such structures can be a naturally occurring polypeptide or polypeptide "fold" (a structural motif), or can have one or more modifications, such as additions, deletions or substitutions of amino acids, relative to a naturally occurring polypeptide or fold. These scaffolds can be derived from a polypeptide of any species (or of more than one species), such as a human, other mammal, other vertebrate, invertebrate, plant, bacteria, or virus.

Typically the biocompatible framework structures are based on protein scaffolds or skeletons other than immunoglobulin domains. For example, those based on fibronectin, ankyrin, lipocalin, neocarzinostain, cytochrome b, CP1 zinc finger, PST1, coiled coil, LACT-D1, Z domain and tendramisat domains may be used (*see, e.g.,* Nygren and Uhlen, Current Opinion in Structural Biology, 7:463-469 (1997)).

In preferred embodiments, it will be appreciated that the binding agents of the invention include the humanized antibodies described herein. Humanized antibodies such as those described herein can be produced using techniques known to those skilled in the art (Zhang, W., et al., Molecular Immunology, 42(12):1445-1451 (2005); Hwang W. et al., Methods, 36(1):35-42 (2005); Dall'Acqua WF, et al., Methods, 36(1):43-60 (2005); and Clark, M., Immunology Today, 21(8):397-402 (2000)).

The present invention therefore relates to an isolated antibody, exemplified by antibody A but also applicable to all antibodies disclosed herein, or an antigen binding fragment thereof, which specifically binds to sclerostin and wherein the variable domain of the heavy chain comprises at least one CDR having the sequences given in SEQ ID NO:109, 115, 121, 127, 133, 139, 145, 151, 157, 163, 169, 175, 181, 187, 193, 199, 205, 211, 217, 223, 229, 235, 241, 247, and 253 for CDR-H1; SEQ ID NO:110, 116, 122, 128, 134, 140, 146, 152, 158, 164, 170, 176, 182, 188, 194, 200, 206, 212, 218, 224, 230, 236, 242, 248, and 254 for CDR-H2; and SEQ ID NO:111, 117, 123, 129, 135, 141, 147, 153, 159, 165, 171, 177, 183, 189, 195, 201, 217, 213, 219, 225, 231, 237, 243, 249, and 255 for CDR-H3. The antibody or antigen binding fragment thereof may comprise a heavy chain variable domain in which the CDRs consist of at least one of the peptides of SEQ ID NO:109, 115, 121, 127, 133, 139, 145, 151, 157, 163, 169, 175, 181, 187, 193, 199, 205, 211, 217, 223, 229, 235, 241,247, and 253 for CDR-H1; SEQ ID NO:110, 116, 122, 128, 134, 140, 146, 152, 158, 164, 170, 176, 182, 188, 194, 200, 206, 212, 218, 224, 230, 236, 242, 248, and 254 for CDR-H2; and SEQ ID NO:111, 117, 123, 129, 135, 141, 147, 153, 159, 165, 171, 177, 183, 189, 195, 201, 217, 213, 219, 225, 231, 237, 243, 249, and 255 for CDR-H3.

When a light chain is present in antibodies of the invention, the light chain may be any suitable complementary chain and may in particular be selected from a light chain wherein the variable domain comprises at least one or two or all of the CDRs consisting of (or comprising) at least one of the peptides of SEQ ID NO:106, 112, 118, 124, 130, 136, 142, 148, 154, 160, 166, 172, 178, 184, 190, 196, 202, 208, 214, 220, 226, 232, 238, 244, and 250 for CDR-L1; SEQ ID NO:107, 113, 119, 125, 131, 137, 143, 149, 155, 161, 167, 173, 179, 185, 191, 197, 203, 209, 215, 221, 227, 233, 239, 245, and 251 for CDR-L2 and SEQ ID NO:108, 114, 120, 126, 132, 138, 144, 150, 156, 162, 168, 174, 180, 186, 192, 198, 204, 210, 216, 222, 228, 234, 240, 246, and 252 for CDR-L3.

Where an antibody comprises one or more of CDR-H1, CDR-H2, CDR-H3, CDR-L1, CDR-L2 and CDR-L3 as described above, it may be obtained by expression from a host cell containing DNA coding for these sequences. A DNA coding for each CDR sequence may be determined on the basis of the amino acid sequence of the CDR and synthesized together with any desired antibody variable region framework and constant region DNA sequences using oligonucleotide synthesis techniques, site-directed mutagenesis and polymerase chain reaction (PCR) techniques as appropriate. DNA coding for variable region frameworks and constant regions is widely available to those skilled in the art from genetic sequences databases such as GenBank®. Each of the above-mentioned CDRs will be typically located in a variable region framework at positions 31-35 (CDR-H1), 50-65 (CDR-H2) and 95-102 (CDR-H3) of the heavy chain and positions 24-34 (CDR-L1), 50-56 (CDR-L2) and 89-97 (CDR-L3) of the light chain according to the Kabat numbering system (Kabat et al., 1987 in Sequences of Proteins of Immunological Interest, U.S. Department of Health and Human Services, NIH, USA).

Once synthesized, the DNA encoding an antibody of the invention or fragment thereof may be propagated and expressed according to any of a variety of well-known procedures for nucleic acid excision, ligation, transformation, and transfection using any number of known expression vectors. Thus, in certain embodiments, expression of an antibody fragment may be preferred in a prokaryotic host, such as *Escherichia coli* (*see, e.g.,* Pluckthun et al., Methods Enzymol., 178:497-515 (1989)). In certain other embodiments, expression of the antibody or a fragment thereof may be preferred in a eukaryotic host cell, including yeast (*e.g., Saccharomyces cerevisiae, Schizosaccharomyces pombe,* and *Pichia pastoris*), animal cells (including mammalian cells) or plant cells. Examples of suitable animal cells include, but are not limited to, myeloma (such as a mouse NSO line), COS, CHO, or hybridoma cells. Examples of plant cells include tobacco, corn, soybean, and rice cells.

One or more replicable expression vectors containing DNA encoding an antibody variable and/or constant region may be prepared and used to transform an appropriate cell line, for example, a non-producing myeloma cell line, such as a mouse NSO line or a bacteria, such as *E. coli,* in which production of the antibody will occur. In order to obtain efficient transcription and translation, the DNA sequence in each vector should include appropriate regulatory sequences, particularly a promoter and leader sequence operatively linked to the variable domain sequence. Particular methods for producing antibodies in this way are generally well-known and routinely used. For example, basic molecular biology procedures are described by Maniatis et al., Molecular Cloning, A Laboratory Manual, 2d ed., Cold Spring Harbor Laboratory, New York (1989). *See also* Maniatis *et al,* 3d ed., Cold Spring Harbor Laboratory, New York (2001). DNA sequencing can be performed as described in Sanger et al., PNAS, 74:5463 (1977), and the Amersham International plc sequencing handbook, and site directed mutagenesis can be carried out according to methods known in the art (Kramer et al., Nucleic Acids Res., 12:9441 (1984); Kunkel, Proc. Natl. Acad. Sci. USA, 82:488-92 (1985); Kunkel et al., Methods in Enzymol., 154:367-82 (1987); the Anglian Biotechnology Ltd handbook). Additionally, numerous publications describe techniques suitable for the preparation of antibodies by manipulation of DNA, creation of expression vectors, and transformation and culture of appropriate cells (Mountain, A. and Adair, J. R., in Biotechnology and Genetic Engineering Reviews, Tombs, M. P. (ed.), 10, Chapter 1, Intercept, Andover, UK (1992)); Current Protocols in Molecular Biology, F.M. Ausubel (ed.), Wiley Interscience, New York (1999)).

Antibodies with improved affinities containing one or more of the above-mentioned CDRs can be obtained by a number of affinity maturation protocols including maintaining the CDRs (Yang et al., J. Mol. Biol., 254:392-403 (1995)), chain shuffling (Marks et al., Biotechnology, 10:779-783 (1992)), use of mutation strains of *E*. *coli.* (Low et al., J. Mol. Biol., 250:350-368 (1996)), DNA shuffling (Patten et al., Curr. Opin. Biotechnol., 8:724-733 (1997)), phage display (Thompson et al., J. Mol. Biol., 256:7-88 (1996)) and sexual PCR (Crameri et al., Nature, 391:288-291 (1998)), All of these methods of affinity maturation are discussed by Vaughan et al. (Nature Biotechnology, 16:535-539 (1998)).

Other antibodies according to the invention may be obtained by conventional immunization and cell fusion procedures as described herein and known in the art. Monoclonal antibodies of the invention may be generated using a variety of known techniques. In general, monoclonal antibodies that bind to specific antigens may be obtained by methods known to those skilled in the art (*see,* for example, Kohler et al., Nature 256:495 (1975); Coligan et al. (eds.), Current Protocols in Immunology, 1:2.5.12.6.7 (John Wiley & Sons 1991); U.S. Patent Nos. RE 32,011, 4,902,614, 4,543,439, and 4,411,993; Monoclonal Antibodies, Hybridomas: A New Dimension in Biological Analyses, Plenum Press, Kennett, McKearn, and Bechtol (eds.) (1980); and Antibodies: A Laboratory Manual, Harlow and Lane (eds.), Cold Spring Harbor Laboratory Press (1988); Picksley et al., "Production of monoclonal antibodies against proteins expressed in E. coli," in DNA Cloning 2: Expression Systems, 2d Ed., Glover et al. (eds.), page 93 (Oxford University Press i995)). Antibody fragments may be derived therefrom using any suitable standard technique such as proteolytic digestion, or optionally, by proteolytic digestion (for example, using papain or pepsin) followed by mild reduction of disulfide bonds and alkylation. Alternatively, such fragments may also be generated by recombinant genetic engineering techniques as described herein.

Monoclonal antibodies can be obtained by injecting an animal, for example, a rat, hamster, a rabbit, or preferably a mouse, including for example a transgenic or a knock-out, as known in the art, with an immunogen comprising human sclerostin of SEQ ID NO:1, or a fragment thereof, according to methods known in the art and described herein. Specific antibody production may be monitored after the initial injection and/or after a booster injection by obtaining a serum sample and detecting the presence of an antibody that binds to human sclerostin (or fragment thereof) using any one of several immunodetection methods known in the art and described herein, From animals producing the desired antibodies, lymphoid cells, most commonly cells from the spleen or lymph node, are removed to obtain B-lymphocytes. The B-lymphocytes are then fused with a drug-sensitized myeloma cell fusion partner, preferably one that is syngeneic with the immunized animal and that optionally has other desirable properties (*e.g*., inability to express endogenous Ig gene products, *e.g*., P3X63 - Ag 8.653 (ATCC No. CRL 1580); NSO, SP20) to produce hybridomas, which are immortal eukaryotic cell lines. The lymphoid (*e.g*., spleen) cells and the myeloma cells may be combined for a few minutes with a membrane fusion-promoting agent, such as polyethylene glycol or a nonionic detergent, and then plated at low density on a selective medium that supports the growth of hybridoma cells but not unfused myeloma cells. A preferred selection media is HAT (hypoxanthine, aminopterin, thymidine). After a sufficient time, usually about one to two weeks, colonies of cells are observed. Single colonies are isolated, and antibodies produced by the cells may be tested for binding activity to human sclerostin, using any one of a variety of immunoassays known in the art and described herein, The hybridomas are cloned (*e.g*., by limited dilution cloning or by soft agar plaque isolation) and positive clones that produce an antibody specific to sclerostin are selected and cultured, The monoclonal antibodies from the hybridoma cultures may be isolated from the supernatants of hybridoma cultures. An alternative method for production of a murine monoclonal antibody is to inject the hybridoma cells into the peritoneal cavity of a syngeneic mouse, for example, a mouse that has been treated (*e.g*., pristane-primed) to promote formation of ascites fluid containing the Monoclonal antibody. Monoclonal antibodies can be isolated and purified by a variety of well-established techniques. Such isolation techniques include affinity chromatography with Protein-A Sepharose, size-exclusion chromatography, and ion-exchange chromatography (see, for example, Coligan at pages 2.7.1-2.7.12 and pages 2.9.1-2.9.3; Baines et al., "Purification of Immunoglobulin G (IgG)," in Methods in Molecular Biology, Vol. 10, pages 79-104 (Humana Press, Inc. (1992)). Monoclonal antibodies may be purified by affinity chromatography using an appropriate ligand selected based on particular properties of the antibody (*e.g*., heavy or light chain isotype, binding specificity, etc.). Examples of a suitable ligand, immobilized on a solid support, include Protein A, Protein G, an anticonstant region (light chain or heavy chain) antibody, an anti-idiotype antibody, and a TGF-beta binding protein, or fragment or variant thereof.

An antibody of the present invention may also be a human monoclonal antibody. Human monoclonal antibodies may be generated by any number of techniques with which those having ordinary skill in the art will be familiar. Such methods include, but are not limited to, Epstein Barr Virus (EBV) transformation of human peripheral blood cells (*e.g.,* containing B lymphocytes), *in vitro* immunization of human B cells, fusion of spleen cells from immunized transgenic mice carrying inserted human immunoglobulin genes, isolation from human immunoglobulin V region phage libraries, or other procedures as known in the art and based on the disclosure herein. For example, human monoclonal antibodies may be obtained from transgenic mice that have been engineered to produce specific human antibodies in response to antigenic challenge. Methods for obtaining human antibodies from transgenic mice are described, for example, by Green et al., Nature Genet., 7:13 (1994); Lonberg et al., Nature, 368:856 (1994); Taylor et al., Int. Immun., 6:579 (1994); U.S. Patent No. 5,877,397; Bruggemann et al., Curr. Opin. Biotechnol., 8:455-58 (1997); Jakobovits et al., Ann. N. Y. Acad. Sci., 764:525-35 (1995). In this technique, elements of the human heavy and light chain locus are introduced into strains of mice derived from embryonic stem cell lines that contain targeted disruptions of the endogenous heavy chain and light chain loci (see *also* Bruggemann et al., Curr. Opin. Biotechnol., 8:455-58 (1997)). For example, human immunoglobulin transgenes may be mini-gene constructs, or transloci on yeast artificial chromosomes, which undergo B cell-specific DNA rearrangement and hypermutation in the mouse lymphoid tissue. Human monoclonal antibodies may be obtained by immunizing the transgenic mice, which may then produce human antibodies specific for sclerostin. Lymphoid cells of the immunized transgenic mice can be used to produce human antibody-secreting hybridomas according to the methods described herein, Polyclonal sera containing human antibodies may also be obtained from the blood of the immunized animals.

Another method for generating human antibodies of the invention includes immortalizing human peripheral blood cells by EBV transformation. *See, e.g.,* U.S. Patent No. 4,464,456. Such an immortalized B cell line (or lymphoblastoid cell line) producing a monoclonal antibody that specifically binds to sclerostin can be identified by immunodetection methods as provided herein, for example, an ELISA, and then isolated by standard cloning techniques. The stability of the lymphoblastoid cell line producing an anti-sclerostin antibody may be improved by fusing the transformed cell line with a murine myeloma to produce a mouse-human hybrid cell line according to methods known in the art (*see, e.g.,* Glasky et al., Hybridoma 8:377-89 (1989)). Still another method to generate human monoclonal antibodies is *in vitro* immunization, which includes priming human splenic B cells with human sclerostin, followed by fusion of primed B cells with a heterohybrid fusion partner. *See, e.g.,* Boerner et al., J. Immunol., 147:86-95 (1991).

In certain embodiments, a B cell that is producing an anti-human sclerostin antibody is selected and the light chain and heavy chain variable regions are cloned from the B cell according to molecular biology techniques known in the art (WO 92/02551; U.S. Patent No. 5,627,052; Babcook et al., Proc. Natl. Acad. Sci. USA, 93:7843-48 (1996)) and described herein. B cells from an immunized animal may be isolated from the spleen, lymph node, or peripheral blood sample by selecting a cell that is producing an antibody that specifically binds to sclerostin. B cells may also be isolated from humans, for example, from a peripheral blood sample. Methods for detecting single B cells that are producing an antibody with the desired specificity are well known in the art, for example, by plaque formation, fluorescence-activated cell sorting, *in vitro* stimulation followed by detection of specific antibody, and the like, Methods for selecting specific antibody-producing B cells include, for example, preparing a single cell suspension of B cells in soft agar that contains human sclerostin. Binding of the specific antibody produced by the B cell to the antigen results in the formation of a complex, which may be visible as an immunoprecipitate. After the B cells producing the desired antibody are selected, the specific antibody genes may be cloned by isolating and amplifying DNA or mRNA according to methods known in the art and described herein.

An additional method for obtaining antibodies of the invention is by phage display. *See, e.g.,* Winter et al., Annu. Rev. Immunol., 12:433-55 (1994); Burton et al., Adv. Immunol., 57:191.-280 (1994). Human or murine immunoglobulin variable region gene combinatorial libraries may be created in phage vectors that can be screened to select Ig fragments (Fab, Fv, sFv, or multimers thereof) that bind specifically to TGF-beta binding protein or variant or fragment thereof. *See, e.g.,* U.S. Patent No. 5,223,409; Huse et al., Science, 246-.1275-81 (1989); Sastry et al., Proc. Natl. Acad. Sci. USA, 86:5728-32 (1989); Alting-Mees et al., Strategies in Molecular Biology, 3:1-9 (1990); Kang et al., Proc. Natl. Acad. Sci. USA, 88:4363-66 (1991); Hoogenboom et al., J. Molec. Biol., 227:381-388 (1992); Schlebusch et al., Hybridoma, 16:47-52 (1997) and references cited therein, For example, a library containing a plurality of polynucleotide sequences encoding Ig variable region fragments may be inserted into the genome of a filamentous bacteriophage, such as M13 or a variant thereof, in frame with the sequence encoding a phage coat protein, A fusion protein may be a fusion of the coat protein with the light chain variable region domain and/or with the heavy chain variable region domain. According to certain embodiments, immunoglobulin Fab fragments may also be displayed on a phage particle (*see, e.g.,* U.S. Patent No. 5,698,426).

Heavy and light chain immunoglobulin cDNA expression libraries may also be prepared in lambda phage, for example, using λlmmunoZap^{™}(H) and λImmunoZap^{™}(L) vectors (Stratagene, La Jolla, California). Briefly, mRNA is isolated from a B cell population, and used to create heavy and light chain immunoglobulin cDNA expression libraries in the λImmunoZap(H) and λImmunoZap(L) vectors. These vectors may be screened individually or co-expressed to form Fab fragments or antibodies *(see* Huse *et al., supra*; *see also* Sastry *et al., supra*). Positive plaques may subsequently be converted to a non-lytic plasmid that allows high level expression of monoclonal antibody fragments from *E. coli.*

In one embodiment, in a hybridoma the variable regions of a gene expressing a monoclonal antibody of interest are amplified using nucleotide primers. These primers may be synthesized by one of ordinary skill in the art, or may be purchased from commercially available sources. *See, e.g.,* Stratagene (La Jolla, California), which sells primers for mouse and human variable regions including, among others, primers for V_{Ha}, V_{Hb}, V_{Hc}, V_{Hc}, C_{H1}, V_{L} and C_{L} regions. These primers may be used to amplify heavy or light chain variable regions, which may then be inserted into vectors such as ImmunoZAP^{™}H or ImmunoZAP^{™}L (Stratagene), respectively. These vectors may then be introduced into *E*. *coli,* yeast, or mammalian-based systems for expression. Large amounts of a single-chain protein containing a fusion of the V_{H} and V_{L} domains may be produced using these methods (*see* Bird et al., Science, 242:423-426, (1988)).

Once cells producing antibodies according to the invention have been obtained using any of the above-described immunization and other techniques, the specific antibody genes may be cloned by isolating and amplifying DNA or mRNA therefrom according to standard procedures as described herein. The antibodies produced therefrom may be sequenced and the CDRs identified and the DNA coding for the CDRs may be manipulated as described previously to generate other antibodies according to the invention.

Preferably the binding agents specifically bind to sclerostin. As with all binding agents and binding assays, one of skill in this art recognizes that the various moieties to which a binding agent should not detectably bind in order to be therapeutically effective and suitable would be exhaustive and impractical to list. Therefore, for a binding agent disclosed herein, the term "specifically binds" refers to the ability of a binding agent to bind to sclerostin, preferably human sclerostin, with greater affinity than it binds to an unrelated control protein, Preferably the control protein is hen egg white lysozyme. Preferably the binding agents bind to sclerostin with an affinity that is at least, 50, 100, 250, 500, 1000, or 10,000 times greater than the affinity for a control protein. A binding agent may have a binding affinity for human sclerostin of less than or equal to 1 x 10⁻⁷ M, less than or equal to 1 x 10⁻⁸ M, less than or equal to 1 x 10⁻⁹ M, less than or equal to 1 x 10⁻¹⁰ M, less than or equal to 1 x 10⁻¹¹ M, or less than or equal to 1 x 10⁻¹²M.

Affinity may be determined by an affinity ELISA assay. In certain embodiments, affinity may be determined by a BIAcore assay. In certain embodiments, affinity may be determined by a kinetic method. In certain embodiments, affinity may be determined by an equilibrium/solution method. Such methods are described in further detail herein or known in the art.

Sclerostin binding agents of the present invention preferably modulate sclerostin function in the cell-based assay described herein and/or the *in vivo* assay described herein and/or bind to one or more of the epitopes described herein and/or cross-block the binding of one of the antibodies described in this application and/or are cross-blocked from binding sclerostin by one of the antibodies described in this application. Accordingly such binding agents can be identified using the assays described herein.

In certain embodiments, binding agents are generated by first identifying antibodies that bind to one more of the epitopes provided herein and/or neutralize in the cell-based and/or *in vivo* assays described herein and/or cross-block the antibodies described in this application and/or are cross-blocked from binding sclerostin by one of the antibodies described in this application. The CDR regions from these antibodies are then used to insert into appropriate biocompatible frameworks to generate sclerostin binding agents. The non-CDR portion of the binding agent may be composed of amino acids, or may be a non-protein molecule. The assays described herein allow the characterization of binding agents. Preferably the binding agents of the present invention are antibodies as defined herein.

It will be understood by one skilled in the art that some proteins, such as antibodies, may undergo a variety of posttranslational modifications. The type and extent of these modifications often depends on the host cell line used to express the protein as well as the culture conditions. Such modifications may include variations in glycosylation, methionine oxidation, diketopiperizine formation, aspartate isomerization and asparagine deamidation. A frequent modification is the loss of a carboxy-terminal basic residue (such as lysine or arginine) due to the action of carboxypeptidases (as described in Harris, R.J., Journal of Chromatography, 705:129-134 (1995).

To generate antibodies A-M, rabbits were immunized by subcutaneous injection of recombinant human sclerostin at three weekly intervals, initially with Freund's Complete Adjuvant and subsequently with Freund's Incomplete Adjuvant. Peripheral blood lymphocytes were harvested and purified on Lympholyte-R (Cedarlane Labs), Immune rabbit lymphocytes were cultures in the presence of irradiated EL-4 cells and rabbit T cell conditioned media, before supernatants were screened for binding to human sclerostin. Individual B cells secreting antibody with appropriate binding characteristics were isolated from positive microtiter wells according to the Selected Lymphocyte Antibody Method (Babcook et al., Proc. Natl. Acad Sci. USA, 93:784307848 (1996)), and heavy and light chain variable region genes were cloned from single rabbit B cells. Variable regions were expressed as recombinant chimeric IgGs to confirm binding.

In the amino acid sequences shown below, the signal peptides are underlined and the boxed-shaded amino acids represent complement-determining regions (CDR's).
Antibody A VL 
MDTRAPTQLLGLLLLWLPGATFAAVLTQTPSPVSAGVGGTVTIKCQSSQRVYNNNEL
SWYQQKPGQPPKRLIYEASTLASGVPDRFSGSGSGTQFTLTISGVQCDDAATYYCLG
GYDDDGDNAFGGGTEVVVKR (SEQ ID NO:2)
 
ATGGACACGAGGGCCCCCACTCAGCTGCTGGGGCTCCTGCTGCTCTGGCTCCCAG
GTGCCACATTTGCAGCCGTGCTGACCCAGACACCATCGCCCGTGTCTGCAGGTGT
GGGAGGCACAGTCACCATCAAGTGCCAGTCCAGTCAGAGGGTTTTTAATAACAA
CGAATTATCCTGGTATCAGCAGAAACCAGGGCAGCCGCCCAAACGCCTGATCTA
TGAAGCATCCACACTGGCATCTGGGGTCCCAGATAGGTTCAGCGGCAGTGGATCT
GGGACACAGTTCACTCTCACCATCAGCGGCGTGCAGTGTGACGATGCTGCCACTT
ACTACTGTCTAGGCGGTTATGATGATGATGGTGATAATGCTTTCGGCGGAGGGAC
CGAGGTGGTGGTCAAACGT (SEQ ID NO:3)
VH 
METGLRWLLLVAVLKGVQCQEQLKESGGRLVTPGTPLTLTCTASGFSLNNYYMTW
VRQAPGKGLEWIGLIYPDGSTFYANWAEGRFTSSKTSTTVTLKMTSPTTEDTATYFC
AREGGAGDNTGTEYYYGVDLWGQGTLVTVSS (SEQ ID NO:4)
 
ATGGAGACTGGGCTGCGCTGGCTTCTCCTGGTCGCTGTGCTCAAAGGTGTCCAGT
GTCAGGAGCAGCTGAAGGAGTCCGGGGGTCGCCTGGTCACGCCTGGGACACCCC
TGACACTCACCTGCACAGCCTCTGGATTCTCCCTCAATAATTACTATATGACCTG
GGTCCGCCAGGCTCCAGGGAAGGGACTGGAATGGATCGGACTCATTTATCCTGA
TGGTAGCACATTCTACGCGAACTGGGCGGAAGGCCGATTCACCAGCTCCAAGAC
CTCGACCACGGTGACTCTGAAGATGACCAGTCCGACAACCGAGGACACGGCCAC
CTATTTCTGTGCCAGAGAAGGTGGTGCTGGTGATAATACTGGTACCGAATATTAC
TACGGCGTGGACCTCTGGGGCCAGGGCACCCTCGTCACCGTCTCGAGC (SEQ ID
NO:5)
Antibody B VL 
MDMRAPTQLLGLLLLWLPGATFAIVMTQTPSSVSAAVGDTVTINCQASENVYDKSA
LSWYQQKPGQPPKLLIYLASTLASGVPSRFKGSGSGTQFTLTISDVVCADAATYYCA
GYKSSIIDGTAFGGGTDVVVKG (SEQ ID NO:6)
 
ATGGACATGAGGGCCCCCACTCAGCTGCTGGGGCTCCTGCTGCTCTGGCTCCCAG
GTGCCACATTTGCCATCGTGATGACCCAGACTCCATCTTCCGTGTCTGCAGCTGT
GGGAGATACAGTCACCATCAATTGCCAGGCCAGTGAGAACGTTTATGATAAAG
CGCCTTATCCTGGTATCAGCAGAAACCAGGGCAGCCTCCCAAGCTCCTGATCTAT
CTGGCATCCACTCTGGCATCTGGGGTCCCATCGCGGTTCAAAGGCAGTGGATCTG
GGACACAGTTCACTCTCACCATCAGCGATGTGGTGTGTGCGGATGCTGCCACTTA
CTACTGTGCAGGATATAAAAGTAGTATTATTGATGGTACTGCTTTCGGCGGAGGG
ACCGACGTGGTGGTCAAAGGA (SEQ ID NO:7)
VH 
METGLRWLLLVAVLKGVQCQSVEESGGRLVTPGTPLTLTCTVSGFSLSNNALSWVR
QAPGKGLEWIGAIGAGGNTYYASWAKGRFTISKTSSTTVDLRMTSPTTEDTATYFCA
NGDLPGGIWGPGTLVTVSL (SEQ ID NO:8)
 
ATGGAGACAGGCCTGCGCTGGCTTCTCCTGGTCGCTGTGCTCAAAGGTGTCCAGT
GTCAGTCGGTGGAGGAGTCCGGGGGTCGCCTGGTCACGCCTGGGACACCCCTGA
CACTCACCTGCACAGTCTCTGGATTCTCCCTCAGTAACAATGCACTGAGCTGGGT
CCGCCAGGCTCCAGGGAAGGGGCTGGAATGGATCGGGGCCATTGGTGCTGGTGG
TAACACATACTATGCGAGCTGGGCGAAAGGCCGATTCACCATCTCCAAAACCTC
GTCGACCACGGTGGATCTGAGAATGACCAGTCCGACAACCGAGGACACGGCCAC
CTATTTCTGTGCCAACGGGGACCTGCCAGGTGGCATCTGGGGCCCAGGCACCCTG
GTCACCGTCTCCCTA (SEQ ID NO:9)
Antibody C VL 
MDIRAPTQLLGLLLLWLPGARCADIVMTQTASPVSAAVGGTVTINCQASQSISPALA
WYQQKPGQRPKLLIYDVSKLASGVPSRFSGSGSGTQFTLTISDLECADGATYYCQSY
YGINSNSYGNIFGGGTEVVVKG (SEQ ID NO:10)
 
ATGGACATAAGGGCCCCCACTCAGCTGCTGGGGCTCCTACTGCTCTGGCTCCCAG
GTGCCAGATGTGCTGACATTGTGATGACCCAGACTGCATCCCCCGTGTCTGCAGC
TGTGGGAGGCACAGTCACCATCAATTGCCAGGCCAGTCAGAGCATTAGCCCTGC
ATTAGCCTGGTATCAGCAGAAACCAGGGCAGCGTCCCAAGCTCCTGATCTACGAT
GTATCGAAACTGGCATCTGGGGTCCCATCGCGGTTCAGTGGCAGTGGATCTGGGA
CACAGTTCACTCTCACCATCAGCGACCTGGAGTGTGCCGATGGTGCCACTTACTA
CTGTCAAAGCTATTATGGTATTAATAGTAATAGTTATGGTAATATTTTCGGCGGA
GGGACCGAGGTGGTGGTCAAAGGA (SEQ ID NO:11)
VH 
METGLRWLLLVAVLKGVQCQSVEESGGRLVTPGTPLTLTCTVSGFSLSSYAMGWFR
QAPGKGLEWIGYIYGNYNKYYASWAKGRFTISKTSTMVDLKMTSLTTEDTATYFCA
RGGAMDVWGQGTLVTVSS (SEQ ID NO:12)
 
ATGGAGACAGGCCTGCGCTGGCTTCTCCTGGTCGCTGTGCTCAAAGGTGTCCAGT
GTCAGTCGGTGGAGGAGTCCGGGGGTCGCCTGGTCACGCCTGGGACACCCCTGA
CACTCACCTGCACAGTCTCTGGATTCTCCCTCAGTAGCTATGCAATGGGCTGGTT
CCGCCAGGCTCCAGGGAAGGGGCTGGAGTGGATCGGCTACATTTATGGTAATTA
TAATAAATACTACGCGAGCTGGGCGAAAGGCCGATTCACCATCTCCAAAACCTC
GACCATGGTGGATCTGAAGATGACTAGTCTGACAACCGAGGACACGGCCACCTA
TTTCTGTGCCAGAGGGGGTGCTATGGATGTCTGGGGCCAGGGCACCCTGGTCACC
GTCTCCAGC (SEQ ID NO:13)
Antibody D VL 
MDTRAPTQLLGLLLLWLPGARCAYDMTQTPASVEVAVGGTVTIKCQASESISTWLA
WYQQKPGQPPKLLIYRASTLASGVSSRFKGSGYGTQFTLTISGVECADAATYYCQQG
WNINNIDNIFGGGTEVVVKG (SEQ ID NO:14)
 
ATGGACACCAGGGCCCCCACTCAGCTGCTGGGGCTCCTGCTGCTCTGGCTCCCAG
GTGCCAGATGCGCCTATGATATGACCCAGACTCCAGCCTCTGTGGAGGTAGCTGT
GGGAGGCACAGTCACCATCAAGTGCCAGGCCAGTGAGAGCATTAGCACTTGGTT
AGCCTGGTATCAGCAGAAACCAGGGCAGCCTCCCAAGCTCCTGATCTACAGGGC
ATCCACTCTGGCATCTGGGGTCTCATCGCGGTTCAAAGGCAGTGGATATGGGACA
CAGTTCACTCTCACCATCAGCGGCGTGGAGTGTGCCGATGCTGCCACTTACTACT
GTCAACAGGGTTGGAATATTAATAATATTGATAATATTTTCGGCGGAGGGACCGA
GGTGGTGGTCAAAGGA (SEQ ID NO:15)
VH 
METGLRWLLLVAVLKGVQCQSVEESGGRLVTPGTPLTLTCTVSGFSLSSYSMYWVR
QAPGKGLEYIGHILSATAVSYATWAKGRFTISRTSTTVDLKITSPTTEDTATYFCARDR
DGGTTLDGFDPWGPGTLVTVSS (SEQ ID NO:16)
 
ATGGAGACAGGCCTGCGCTGGCTTCTCCTGGTCGCTGTGCTCAAAGGTGTCCAGT
GTCAGTCGGTGGAGGAGTCCGGGGGTCGCCTGGTCACGCCTGGGACACCCCTGA
CACTCACCTGCACAGTCTCTGGATTCTCCCTCAGTAGCTATTCAATGTATTGGGTC
CGCCAGGCTCCAGGGAAGGGGCTGGAATACATCGGATTTATTCTTAGTGCTACTG
CCGTATCCTACGCGACCTGGGCGAAAGGCCGATTCACCATCTCCAGAACCTCGAC
CACGGTGGATCTGAAAATCACCAGTCCGACAACCGAGGACACGGCCACCTATTT
CTGTGCCAGAGATAGAGATGGTGGTACTACACTAGATGGTTTTGATCCCTGGGGC
CCAGGCACCCTGGTCACCGTCTCCAGC (SEQ ID NO:17)
Antibody E VL 
MDIRAPTQLLGLLLLWLPGATFAQLLTQTPPSVSAAVGGTVTISCQSSQSVVNNNNL
AWYQQKPGQPPKLLIYFASTLASGVPSRFKGSGSGTQFTLTISDVQCDDAATYYCQG
TYLSDDWSDAFGGGTEVVVKG (SEQ ID NO:18)
 
ATGGACATCAGGGCCCCCACTCAGCTGCTGGGGCTCCTGCTGCTCTGGCTCCCAG
GTGCCACATTTGCCCAACTGCTGACCCAGACTCCACCCTCGGTGTCTGCAGCTGT
GGGAGGCACAGTCACCATCAGTTGCCAGTCCAGTCAGAGTGTTGTTAATAACAA
CAACTTAGCCTGGTATCAGCAGAAACCAGGGCAGCCTCCCAAGCTCCTGATCTAT
TTTGCCTCCACTCTGGCATCTGGGGTCCCATCGCGGTTCAAAGGCAGTGGATCTG
GGACACAGTTCACTCTCACCATCAGCGACGTGCAGTGTGACGATGCTGCCACTTA
CTACTGTCAAGGCACTTATCTTAGTGATGATTGGTCCGATGCTTTCGGCGGAGGG
ACCGAGGTGGTGGTCAAAGGA (SEQ ID NO:19)
VH 
METGLRWLLLVAVLKGVQCQSVEESGGRLVTPGTPLTLTCKVSGFSLSSSVMNWVR
QAPGKGLEWIGAIWSGGYTYYATWAKGRFTISRTSTTVDLKMTSLTAADTATYFCA
RGQFGASGGGDVLWGPGTLVTVSS (SEQ ID NO:20)
 
ATGGAGACAGGCCTGCGCTGGCTTCTCCTGGTCGCTGTGCTCAAAGGTGTCCAGT
GTCAGTCGGTGGAGGAGTCCGGGGGTCGCCTGGTCACGCCTGGGACACCCCTGA
 
CACTCACCTGCAAAGTCTCTGGATTCTCCCTCAGTTCCTCTGTAATGAACTGGGTC
CGCCAGGCTCCAGGGAAGGGGCTGGAGTGGATCGGAGCCATTTGGAGTGGTGGT
TACACATACTACGCGACCTGGGCAAAAGGCCGATTCACCATCTCCAGAACCTCG
ACCACGGTGGATCTGAAAATGACCAGTCTGACAGCCGCGGACACGGCCACCTAC
TTCTGTGCCAGAGGACAATTTGGTGCTAGTGGTGGTGGTGATGTTTTGTGGGGCC
CGGGCACCCTGGTCACCGTCTCCAGC (SEQ ID NO:21)
Antibody F VL 
MDIRAPTQLLGLLLLWLPGATFAQVLTQTPSSVSAAVGGTVTISCQSSPSVDNNNLLS
WFQQKPGQPPKLLIYDASTLASGVPSRFEGSGSGTQFTLAISGVQCDDAATYYCVAG
YGKRSRDIRVFGGGTGVVVKG (SEQ ID NO:22)
 
ATGGACATAAGGGCCCCCACTCAGCTGCTGGGGCTCCTGCTGCTCTGGCTCCCAG
GTGCCACATTTGCCCAAGTGCTGACCCAGACTCCATCCTCCGTGTCTGCAGCTGT
GGGAGGCACAGTCACCATCAGTTGCCAGTCCAGTCCGAGTGTTGATAATAACAA
CCTCTTATCCTGGTTTCAGCAGAAACCAGGGCAGCCTCCCAAGCTCCTGATCTAT
GATGCTTCCACTCTGGCATCTGGGGTCCCATCGCGGTTCGAAGGCAGTGGATCTG
GGACACAATTCACTCTCGCCATCAGCGGCGTTCAGTGTGACGATGCTGCCACTTA
CTACTGTGTAGCCGGTTATGGTAAAAGGAGTCGAGATATACGAGTTTTCGGCGGA
GGGACCGGGGTGGTGGTCAAAGGA (SEQ ID NO:23)
VH 
METGLRWLLLVAVLKGVQCQSVEESGGRLVTPGTPLTLTCTVSGFDISSHNMQWVR
QAPGKGLEWIGIIYPSNNAYYSNWAKGRFTISKTSTTMSLQMNSLTTEDTATYFCAR
DINSAIWGPGTLVTVSS (SEQ ID NO:24)
 
ATGGAGACAGGCCTGCGCTGGCTTCTCCTGGTCGCTGTGCTCAAAGGTGTCCAGT
GTCAGTCGGTGGAGGAGTCCGGGGGTCGCCTGGTCACGCCTGGGACACCCCTGA
CACTCACCTGCACCGTCTCTGGATTCGACATCAGTAGCCACAATATGCAATGGGT
CCGCCAGGCTCCAGGGAAGGGGCTGGAATGGATCGGAATCATTTATCCTAGTAA
TAATGCATACTATTCCAACTGGGCGAAAGGCCGATTCACCATCTCCAAAACTTCG
ACCACGATGTCTCTGCAAATGAACAGTCTGACAACCGAGGACACGGCCACCTAT
TTCTGCGCCAGAGACATAAATAGTGCTATTTGGGGCCCAGGCACCCTGGTCACCG
TCTCCAGC (SEQ ID NO:25)
Antibody G VL 
MDTRAPTQLLGLLLLWLPGVICAEVAMTQTPSSVSAAVGGTVTIDCQASESIYSNLA
WYQQKPGQPPKLLIYDASDLASGVPSRFKGSGSGTEYTLTISDLECADAATYYCQCS
WGGSTYGIAFGGGTEVVVKR (SEQ ID NO:26)
 
ATGGACACGAGGGCCCCCACTCAGCTGCTGGGGCTCCTGCTGCTCTGGCTCCCAG
GTGTCATATGTGCCGAAGTAGCGATGACCCAGACTCCATCCTCCGTGTCTGCAGC
TGTGGGAGGCACAGTCACCATCGATTGCCAGGCCAGTGAGAGCATTTATAGCAA
TTTAGCCTGGTATCAGCAGAAACCAGGGCAGCCTCCCAAGCTCCTGATCTATGAT
GCATCCGATCTGGCATCTGGGGTCCCATCGCGGTTCAAAGGCAGTGGATCTGGGA
 
CAGAGTACACTCTCACCATCAGCGACCTGGAGTGTGCCGATGCTGCCACTTACTA
CTGTCAATGTAGTTGGGGTGGTAGTACTTATGGTTTTGCTTTCGGCGGAGGGACC
GAGGTGGTGGTCAAACGT (SEQ ID NO:27)
VH 
METGLRWLLLVAVLKGVQCQSLEESGGRLVTPGTPLTLTCTVSGIDLSIYVMTWVR
QAPGKGLEWIGSIDADDSAYYATWATSRSTISRTSTTVALSITSPTTEDTATYFCARG
LYANGGPHILWGPGTLVTVSS (SEQ ID NO:28)
 
ATGGAGACTGGGCTGCGCTGGCTTCTCCTGGTCGCTGTGCTCAAAGGTGTCCAGT
GTCAGTCGCTGGAGGAGTCCGGGGGTCGCCTGGTAACGCCTGGGACACCCCTGA
CACTCACCTGCACAGTCTCTGGAATCGACCTCAGTATCTATGTAATGACTTGGGT
CCGCCAGGCTCCAGGGAAGGGGCTGGAATGGATCGGAAGCATCGATGCTGACGA
TAGCGCATACTACGCGACCTGGGCGACAAGCCGATCCACCATCTCCAGAACCTC
GACCACGGTGGCTCTGAGCATCACCAGTCCGACAACCGAGGACACGGCGACCTA
TTTCTGTGCCAGGGGACTATATGCTAATGGTGGTCCCTTTACCTTATGGGGCCCA
GGCACCCTCGTCACCGTCTCGAGC (SEQ ID NO:29)
Antibody H VL 
MDMRAPTQLLGLLLLWLPGATFAAVLTQTPSPVSAAVGGTVTIKCQSSPSVYNRNQ
LSWFQQKAGQPPKLLIFTASTLASGVPSRFKGSGSGTDFTLTISDLECADAATYYCQG
YYNTGSDIYAFGGGTEVVVKG (SEQ ID NO:30)
 
ATGGACATGAGGGCCCCCACTCAGCTGCTGGGGCTCCTGCTGCTCTGGCTCCCAG
GTGCCACATTTGCCGCCGTGCTGACCCAGACTCCATCTCCCGTGTCTGCAGCTGT
GGGAGGCACAGTCACCATCAAGTGCCAGTCCAGTCCGAGTGTTTATAATCGCAA
CCAATTATCCTGGTTTCAGCAGAAAGCAGGGCAGCCTCCCAAGCTCCTGATCTTT
ACTGCGTCCACTCTGGCATCTGGGGTCCCATCGCGGTTCAAAGGCAGTGGATCTG
GGACAGATTTCACTCTCACCATCAGCGACCTGGAGTGTGCCGATGCTGCCACTTA
CTACTGTCAAGGCTATTATAATACTGGTAGTGATACGTATGCTTTCGGCGGAGGG
ACCGAGGTGGTGGTCAAAGGA (SEQ ID NO:31)
VH 
METGLRWLLLVAVLKGVQCQSVEESGGRLVTPGTPLTLTCTVSGFSLNNYDMSWVR
QAPGKGLEYIGFINTVGYAYYASWAKGRFTISRTSTTVDLKMTSLTTEDTATYFCAS
LDNYYTWGIWGPGTLVTVSL (SEQ ID NO:32)
 
ATGGAGACAGGCCTGCGCTGGCTTCTCCTGGTCGCTGTGCTCAAAGGTGTCCAGT
GTCAGTCGGTGGAGGAGTCCGGGGGTCGCCTGGTCACGCCTGGGACACCCCTGA
CACTCACCTGCACAGTCTCTGGATTCTCCCTCAATAACTACGACATGAGCTGGGT
CCGCCAGGCTCCAGGGAAGGGGCTGGAATATATCGGATTCATTAATACTGTTGGT
TACGCATACTACGCGAGCTGGGCAAAAGGCCGATTCACCATCTCCAGAACCTCG
ACCACGGTGGATCTGAAAATGACCAGTCTGACAACCGAGGACACGGCCACCTAT
TTCTGTGCCAGCCTTGATAATTACTATACTTGGGGCATCTGGGGCCCAGGCACCC
TGGTCACCGTTTCCCTA (SEQ ID NO:33)
Antibody I VL 
MDTRAPTQLLGLLLLWLPGARCADIVMTQTPGSVSAAVGGTVTINCQAAEDIYSSLA
WYQQKSGQPPKLLIYAASILASGAPSRFSGSGSGTEYTLTISGVQCDDAATYYCQTN
YGISSYGAAFGGGTEVVVKG (SEQ ID NO:34)
 
ATGGACACGAGGGCCCCCACTCAGCTGCTGGGGCTCCTGCTGCTCTGGCTCCCAG
GTGCCAGATGTGCCGACATCGTGATGACCCAGACTCCAGGCTCCGTGTCTGCAGC
TGTGGGAGGCACAGTCACCATCAATTGCCAGGCCGCTGAGGACATTTATAGCTCT
TTGGCCTGGTATCAGCAGAAATCAGGGCAGCCTCCCAAGCTCCTGATCTATGCTG
CATCTATTCTGGCATCTGGGGCCCCATCGCGGTTCAGTGGCAGTGGATCTGGGAC
AGAGTACACTCTCACCATCAGCGGCGTGCAGTGTGACGATGCTGCCACTTACTAC
TGTCAAACCAATTATGGTATCAGTAGTTATGGTGCGGCTTTCGGCGGAGGGACCG
AGGTGGTGGTCAAAGGA (SEQ ID NO:35)
VH 
METGLRWLLLVAVLKGVQCQSVEESGGRLVTPGTPLTLTCTVSGFSLSNKPITWVRQ
APGEGLEYIGWISTTGSAYYASWAKGRFTISKTSTTVDLKITSPTTEDTATYFCARYSS
DYGHHDLWGPGTLVTVSS (SEQ ID NO:36)
 
ATGGAGACAGGCCTGCGCTGGCTTCTCCTGGTCGCTGTGCTCAAAGGTGTCCAGT
GTCAGTCGGTGGAGGAGTCCGGGGGTCGCCTGGTCACGCCTGGGACACCCCTGA
CACTCACCTGCACCGTCTCTGGGTTCTCCCTCAGTAACAAGCCAATAACATGGGT
CCGCCAGGCTCCAGGGGAGGGGCTGGAATACATCGGATGGATTAGTACTACTGG
TAGCGCATACTACGCGAGCTGGGCGAAAGGCCGATTCACCATCTCCAAAACCTC
GACCACGGTGGATCTGAAAATCACCAGTCCGACAACCGAGGACACGGCCACCTA
TTTCTGTGCCAGATATAGTAGTGATTATGGACATCATGACTTGTGGGGCCCAGGC
ACCCTGGTCACCGTCTCCAGC (SEQ ID NO:37)
Antibody J VL 
MDTRAPTQLLGLLLLWLPGATFAQVLTQTPSPVSAALGGSVTINCQASQSVYRDYLS
WFQQKPGQPPKLLINGASNLASGVPSRFSGSGSGTQFTLTISDVQCDDAATYYCLGG
FSGNINTFGGGTEVVVKG (SEQ ID NO:38)
 
ATGGACACCAGGGCCCCCACTCAGCTGCTGGGGCTCCTGCTGCTCTGGCTCCCAG
GTGCCACATTTGCGCAAGTGCTGACCCAGACTCCATCACCCGTGTCTGCAGCTCT
GGGAGGCTCAGTCACCATCAATTGCCAGGCCAGTCAGAGTGTTTATAGAGATTAC
TTATCCTGGTTTCAGCAGAAACCAGGTCAGCCTCCCAAGCTCCTGATCAATGGTG
CATCCAATCTGGCATCTGGGGTCCCATCGCGGTTCAGCGGCAGTGGATCTGGGAC
ACAGTTCACTCTCACCATCAGCGACGTGCAGTGTGACGATGCTGCCACTTACTAC
TGTCTAGGCGGTTTTAGTGGTAATATCAATACTTTCGGCGGAGGGACCGAGGTGG
TGGTCAAGGGA (SEQ ID NO:39)
VH 
METGLRWLLLVAVLKGVQCQSLEESGGRLVTPGGSLTLTCTVSGIDLNNYHMCWV
RQAPGKGLKYIGIISNTGYTYYSSWAKGRFTISKTSSTTVDLKMTSLTTEDTATYFCA
GDRLANLWGPGTLVTVSS (SEQ ID NO:40)
 
ATGGAGACAGGCCTGCGCTGGCTTCTCCTGGTCGCTGTGCTCAAAGGTGTCCAGT
GTCAGTCGCTGGAGGAGTCCGGGGGTCGCCTGGTAACGCCTGGAGGATCCCTGA
CACTCACCTGTACAGTCTCTGGAATCGACCTCAATAACTACCACATGTGCTGGGT
CCGCCAGGCTCCAGGGAAGGGGCTGAAATACATCGGAATCATTAGTAATACTGG
TTACACATACTACTCGAGCTGGGCGAAAGGCCGATTCACCATCTCCAAAACCTCG
TCGACCACGGTGGATCTGAAAATGACCAGTCTGACAACCGAGGACACGGCCACC
TATTTCTGTGCTGGAGACCGGCTTGCTAACTTGTGGGGCCCAGGCACCCTGGTCA
CTGTCTCCAGC (SEQ ID NO:41)
Antibody K VL 
MDTRAPTQLLGLLLLWLPGARCADVVMTQTPASVEAAVGGTVTIKCQASQSISTYL
KWYQQKPGQRPKRLIYSASTLTSGVPSRFKGSGSGTDFTLTISDLECGDAATYYCQS
NAGSSSSSCGYAFGGGTEVVVKG (SEQ ID NO:42)
 
ATGGACACGAGGGCCCCCACTCAGCTGCTGGGGCTCCTGCTGCTCTGGCTCCCAG
GTGCCAGATGTGCCGACGTCGTGATGACCCAGACTCCAGCCTCCGTGGAGGCAG
CTGTGGGAGGCACAGTCACCATCAAGTGCCAGGCCAGTCAGAGCATTAGTACCT
ACTTAAAGTGGTATCAGCAGAAACCAGGACAGCGTCCCAAGCGCCTGATCTATT
CTGCATCCACTCTGACATCTGGGGTCCCATCGCGGTTCAAAGGCAGTGGATCTGG
GACAGATTTCACTCTCACCATCAGCGACCTGGAGTGTGGCGATGCTGCCACTTAC
TACTGTCAAAGCAATGCTGGTAGTAGTAGTAGTAGTTGTGGTTATGCTTTCGGCG
GAGGGACCGAGGTGGTGGTCAAAGGA (SEQ ID NO:43)
VH 
METGLRWLLLVAVLKGVQCQSVEESGGRLVTPGTPLTLTCTVSGIDLSYYGMGWVR
QAPGKGLEYIGIISGIGNTYYPTWAKGRFTISKTPTTVDLRITSPTTEDTATYFCATGDF
WGPGTLVTVSS (SEQ ID NO:44)
 
ATGGAGACAGGCCTGCGCTGGCTTCTCCTGGTCGCTGTGCTCAAAGGTGTCCAGT
GTCAGTCGGTGGAGGAGTCCGGGGGTCGCCTGGTCACGCCTGGGACACCCCTGA
CACTCACCTGCACAGTCTCTGGAATTGACCTCAGTTACTATGGGATGGGCTGGGT
CCGCCAGGCTCCAGGGAAGGGGCTGGAATACATCGGGATCATTAGTGGTATTGG
TAATACATACTATCCGACCTGGGCGAAAGGCCGATTCACCATCTCCAAAACCCCG
ACCACGGTGGATCTAAGGATCACCAGTCCGACAACCGAGGACACGGCCACCTAT
TTCTGTGCCACTGGGGACTTCTGGGGCCCAGGCACCCTGGTCACCGTCTCCAGC
(SEQ ID NO:45)
Antibody L VL 
MDTRAPTQLLGLLLLWLPGARCADVVMTQTPASVSEPVGGTVTIKCQASQSISRYLK
WYQQKPGQRPKRLIYSASTLTSGVPSRFKGSGSGTDFTLTISDLECADAATYYCQSN
AGSSSSSCGYAFGGGTEVVVKG (SEQ ID NO:46)
 
ATGGACACGAGGGCCCCCACTCAGCTGCTGGGGCTCCTGCTGCTCTGGCTCCCAG
GTGCCAGATGTGCCGACGTCGTGATGACCCAGACTCCAGCCTCCGTGTCTGAACC
TGTGGGAGGCACAGTCACCATCAAGTGCCAGGCCAGTCAGAGCATTAGTAGGTA
CTTAAAGTGGTATCAGCAGAAACCAGGGCAGCGTCCCAAGCGCCTGATCTATTCT
GCATCCACTCTGACATCTGGGGTCCCATCGCGGTTCAAAGGCAGTGGATCTGGGA
CAGATTTCACTCTCACCATCAGCGACCTGGAGTGTGCCGATGCTGCCACTTACTA
CTGTCAAAGCAATGCTGGTAGTAGCAGTAGTAGTTGTGGTTATGCTTTCGGCGGA
GGGACCGAGGTGGTGGTCAAAGGA (SEQ ID NO:47)
VH 
METGLRWLLLVAVLKGVQCQSVEESGGRLVTPGTPLTLTCTVSGVDLSYYGMGWV
RQAPGKGLEYIGIISGSGNTYYASWAKGRFTISKTSTTVDLKITSPTTEDTATYFCASG
DIWGPGTLVTVSS (SEQ ID NO:48)
 
ATGGAGACAGGCCTGCGCTGGCTTCTCCTGGTCGCTGTGCTCAAAGGTGTCCAGT
GTCAGTCGGTGGAGGAGTCCGGGGGTCGCCTGGTCACGCCTGGGACACCCCTGA
CACTCACCTGCACAGTCTCTGGAGTCGACCTCAGTTACTATGGAATGGGCTGGGT
CCGCCAGGCTCCAGGGAAGGGGCTGGAATACATCGGGATCATTAGTGGTAGTGG
TAACACATACTACGCGAGCTGGGCGAAAGGCCGATTCACCATCTCCAAAACCTC
GACCACGGTGGATCTGAAAATCACCAGTCCGACAACCGAGGACACGGCCACCTA
TTTCTGTGCCAGTGGGGACTTCTGGGGCCCAGGCACCCTGGTCACCGTCTCCAGC
(SEQ ID NO:49)
Antibody M VL 
MDTRAPTQLLGLLLLWLPGARCALVMTQTASPVSAAVGGTVTISCQSSQSVYSNYLS
WFQQKPGQPPKLLIYVASSLASGVPSRFKGSGSGTQFTLTISDLECDDAATYYCGGFQ
KYIDDGGAFGGGTEVVVKG (SEQ ID NO:50)
 
ATGGACACGAGGGCCCCCACTCAGCTGCTGGGGCTCCTGCTGCTCTGGCTCCCAG
GTGCCAGATGTGCCCTTGTGATGACCCAGACTGCATCCCCCGTGTCTGCAGCTGT
GGGAGGCACAGTCACCATCAGTTGCCAGTCCAGTCAGAGTGTTTATAGTAACTAC
TTATCCTGGTTTCAGCAGAAACCAGGGCAGCCTCCCAAGCTCCTGATCTATGTTG
CATCCAGTCTGGCATCTGGGGTCCCATCGCGATTCAAAGGCAGTGGATCTGGGAC
ACAGTTCACTCTCACCATCAGTGACCTGGAGTGTGACGATGCTGCCACTTACTAC
TGTGGAGGCTTTCAGAAATATATTGATGATGGGGGGCTTTCGGCGGAGGGACC
GAGGTGGTGGTCAAAGGA (SEQ ID NO:51)
VH

METGLRWLLLVAVLKGVQCQSVEESGGRLVTPGGSLTLTCTVSGIDLSTYAMSWVR
QAPGKGLEWIGIMSSSGSAYYASWAKGRFTISKTASTTVDLKMTSLTIEDTATYFCA
RSSSIGLWGPGTLVTVSS (SEQ ID NO:52)

ATGGAGACAGGCCTGCGCTGGCTTCTCCTGGTCGCTGTGCTCAAAGGTGTCCAGT
GTCAGTCGGTGGAGGAGTCCGGGGGTCGCCTGGTCACGCCTGGAGGATCCCTGA
CACTTACCTGCACAGTCTCTGGAATCGACCTCAGTACCTATGCAATGAGTTGGGT
CCGCCAGGCTCCAGGGAAGGGGCTGGAATGGATCGGAATCATGAGTAGTAGTGG
TAGCGCATACTACGCGAGCTGGGCGAAAGGCCGATTCACCATCTCCAAAACCGC
GTCGACCACGGTGGATCTGAAAATGACCAGTCTGACAATCGAGGACACGGCCAC
CTATTTCTGTGCCAGAAGTTCTAGTTTTGGATTGTGGGGCCCAGGCACCCTGGTC
ACCGTCTCCAGC (SEQ ID NO:53)

Antibodies N-Y were generated in mice, The Kappa Constant region for all VK regions of antibodies N-Y is as follows:

TDAAPTVSIFPPSSEQLTSGGASVVCFLNNFYPKDINVKWKIDGSERQNGVLNSWTD
QDSKDSTYSMSSTLTLTKDEYERHNSYTCEATHKTSTSPIVKSFNRNEC (SEQ ID
NO:54)

The Heavy Constant Region for all VH regions of antibodies N-Y is as follows:

AKTTPPSVYPLAPGSAAQTNSMVTLGCLVKGYFPEPVTVTWNSGSLSSGVHTFPAVL
QSDLYTLSSSVTVPSSTWPSETVTCNVAHPASSTKVDKKIVPRDCGCKPCICTVPEVS
SVFIFPPKPKDVLTITLTPKVTCVVVDISKDDPEVQFSWFVDDVEVHTAQTQPREEQF
NSTFRSVSELPIMHQDWLNGKEFKCRVNSAAFPAPIEKTISKTKGRPKAPQVYTIPPPK
EQMAKDKVSLTCMITDFFPEDITVEWQWNGQPAENYKNTQPIMDTDGSYFVYSKLN
VQKSNWEAGNTFTCSVLHEGLHNHHTEKSLSHSPGK (SEQ ID NO:55).

In the following antibody amino acid sequences, the boxed-shaded amino acids
represent complement-determining regions (CDR) and the underlined amino acids represent signal peptide.
Antibody N
   VK 
MDFQVQIFSFMLISVTVILSSGEIVLTQSPALMAASPGEKVTIACSVSSSISSTNLHWSQ
QKSGTSPKLWIYGTSNLASGVPVRFSGSGSGTSYSLTISSMEAEDAATYYCQQWSTT
YTFGSGTKLELKR (SEQ ID NO:56)
 
ATGGATTTTCAAGTGCAGATTTTCAGCTTCATGCTAATCAGTGTCACAGTCATATT
GTCCAGTGGAGAAATTGTGCTCACCCAGTCTCCAGCACTCATGGCTGCATCTCCA
GGGGAGAAGGTCACCATCGCCTGCAGTGTCAGCTCAAGTATAAGTTCCACCAAC
TTACACTGGTCCCAGCAGAAGTCAGGAACCTCCCCCAAACTCTGGATTTATGGCA
CATCCAACCTTGCTTCTGGAGTCCCTGTTCGCTTCAGTGGCAGTGGATCTGGGAC
CTCTTATTCTCTCACAATCAGCAGCATGGAGGCTGAAGATGCTGCCACCTATTAC
TGTCAACAGTGGAGTACTACGTATACGTTCGGATCGGGGACCAAGCTGGAGCTG
AAACGT (SEQ ID NO:57)
   VH 
MGWNWIIFFLMAVVTGVNSEVQLQQSGADLVKPGASVKLSCTASGFNIKDYYIHWV
KQRPAQGLEWIGRIDPDNGESTYVPKFQGKATITADTSSNTAYLQLRSLTSEDTAIYY
CGREGLDYGDYYAVDYWGQGTSVTVSS (SEQ ID NO:58)
 
ATGGGATGGAACTGGATCATCTTCTTCCTGATGGCAGTGGTTACAGGGGTCAATT
CAGAGGTGCAGTTGCAGCAGTCTGGGGCAGACCTTGTGAAGCCAGGGGCCTCAG
TCAAGTTGTCCTGCACAGCTTCTGGCTTCAACATTAAAGACTACTATATACACTG
GGTGAAGCAGAGGCCTGCACAGGGCCTGGAGTGGATTGGAAGGATTGATCCTGA
TAATGGTGAAAGTACATATGTCCCGAAGTTCCAGGGCAAGGCCACTATAACAGC
AGACACATCATCCAACACAGCCTACCTACAACTCAGAAGCCTGACATCTGAGGA
CACTGCCATCTATTATTGTGGGAGAGAGGGGCTCGACTATGGTGACTACTATGCT
GTGGACTACTGGGGTCAAGGAACCTCAGTCACTGTCTCGAGC (SEQ ID NO:59)
Antibody 0
   VK 
MESDTLLLWVLLLWVPGSTGKIVLTQSPASLAVSLRQRATISCRASESVDSYGNSFM
HWYQQKPGQPPKLLIYRASNLESGVPARFSGSGSRTDFTLTIDPVEADDAATYYCQQ
NYEDPLTFGAGTKLELKR (SEQ ID NO:60)
 
ATGGAGTCAGACACACTCCTGCTATGGGTGCTACTGCTCTGGGTTCCAGGTTCCA
CAGGTAAAATTGTACTGACCCAATCTCCAGCTTCTTTGGCTGTGTCTCTAAGGCA
GAGGGCCACCATATCCTGCAGAGCCAGTGAAAGTGTTGATAGTTATGGCAATAG
TTTTATGCACTGGTACCAGCAGAAACCAGGACAGCCACCCAAACTCCTCATCTAT
CGTGCATCCAACCTAGAATCTGGGGTCCCTGCCAGGTTCAGTGGCAGTGGGTCTA
GGACAGACTTCACCCTCACCATTGATCCTGTGGAGGCTGATGATGCTGCAACCTA
TTACTGTCAGCAAAATTATGAGGATCCGCTCACGTTCGGTGCTGGGACCAAGTTG
GAGCTGAAACGT (SEQ ID NO:61)
   VH 
MGWPWVFIFLLSVTAGVHSQVQLQQSGAELVRPGTSVKVSCKASGYAFTNYLIEWI
KQRPGQGLEWIGVINPGSGITNYNEKFKIKATLTADKSSSTAYMQLSSLTSDDSAVYF
CARDWDITFYSYFREVYAMDYWGQGTSVTVSS (SEQ ID NO:62)
 
ATGGGATGGCCCTGGGTCTTTATATTTCTCCTGTCAGTAACTGCAGGTGTTCACTC
CCAGGTCCAGCTGCAGCAGTCTGGAGCTGAGCTGGTAAGGCCTGGGACCTCAGT
GAAGGTGTCCTGCAAGGCCTCTGGATACGCCTTCACTAATTACTTGATAGAGTGG
ATAAAGCAGAGGCCTGGACAGGGCCTTGAGTGGATTGGAGTGATTAATCCTGGA
AGCGGTATTATTAATTACAATGAGAAGTTCAAAATCAAGGCAACACTGACTGCA
GACAAATCCTCCAGCACTGCCTACATGCAGCTCAGCAGCCTGACATCTGATGATT
CTGCGGTCTATTTCTGTGCAAGAGACTGGGATACTTTCTATAGTTACGAGAGGGA
GGTCTATGCTATGGACTACTGGGGTCAAGGGACCTCAGTCACCGTCTCGAGC
(SEQ ID NO:63)
Antibody P
   VK 
MESQTLVLVFLLFWIPASRGDILLTQSPAILSVSPGERVSFSCRASQSIGTNIHWYQQR
TDGSPRLLIKFASESISGIPSRFSGSGSGTDFTLSINSVESEDIADYYCQQSNSWPLTFGA
GTKLELKR (SEQ ID NO:64)
 
ATGGAATCACAGACTCTGGTCCTTGTATTTTTGCTTTTCTGGATTCCAGCCTCCAG
AGGTGACATCTTGCTGACTCAGTCTCCAGCCATCCTGTCTGTGAGTCCAGGAGAA
AGAGTCAGTTTCTCCTGCAGGGCCAGTCAGAGCATTGGCACAAACATACACTGGT
ATCAGCAAAGAACAGATGGTTCTCCAAGGCTTCTCATAAAGTTTGCTTCTGAGTC
TATCTCTGGGATCCCTTCCAGGTTTAGTGGCAGTGGCTCAGGGACAGATTTTACT
CTTAGCATCAACAGTGTGGAGTCTGAAGATATTGCAGATTATTACTGTCAACAAA
GTAATAGCTGGCCACTCACGTTCGGTGCTGGGACCAAGCTGGAACTGAAACGT
(SEQ ID NO:65)
   VH 
MDWSWIIFFLMAVVIGINSEVQLQQSGAELVRSGASVKLSCTASGFNIEDYYVHWVK
QRPEQGLEWIGWIDPENGDTEYAPKFQGKATMTADTSSNTAYLQLSSLTSEDTAVY
YCNGGYGNFYFDYWGQGTTVTVSS (SEQ ID NO:66)
 
ATGGATTGGAGCTGGATCATCTTCTTCCTGATGGCAGTGGTTATAGGAATCAATT
CAGAGGTTCAGCTGCAGCAGTCTGGGGCAGAGCTTGTGAGGTCAGGGGCCTCAG
TCAAGTTGTCCTGCACAGCTTCTGGCTTCAACATTGAAGACTACTATGTGCACTG
GGTGAAGCAGAGGCCTGAACAGGGCCTGGAGTGGATTGGATGGATTGATCCTGA
GAATGGTGATACTGAATATGCCCCGAAGTTCCAGGGCAAGGCCACTATGACTGC
AGACACATCCTCCAACACAGCCTACCTGCAGCTCAGCAGCCTGACATCTGAGGA
CACTGCCGTCTATTACTGTAATGGGGGGTATGGTAACTTTTACTTTGACTACTGG
GGCCAAGGCACCACTGTCACCGTCTCGAGC (SEQ ID NO:67)
Antibody Q
   VK 
MEFQTQVFVYMLLWLSGVEGDIVMTQSHKFMSTSVGDRVSITCKASQDVGTAVAW
YQQKPGQSPKLLIYWASTRHTGVPDRFTGSGSGTDFTLTISNVQSEDLADYFCQQYSS
YPYTFGGGTKLELKR (SEQ ID NO:68)
 
ATGGAATTCCAGACTCAGGTCTTTGTATACATGTTGCTGTGGTTGTCTGGTGTTGA
AGGAGACATTGTGATGACCCAGTCTCACAAATTCATGTCCACATCAGTAGGAGA
CAGGGTCAGCATCACCTGCAAGGCCAGTCAGGATGTGGGTACTGCTGTAGCCTG
GTATCAACAGAAACCAGGGCAATCTCCTAAACTACTGATTTACTGGGCATCCACC
CGGCACACTGGAGTCCCTGATCGCTTCACAGGCAGTGGATCTGGGACAGATTTCA
CTCTCACCATTAGCAATGTGCAGTCTGAAGACTTGGCAGATTATTTCTGTCAGCA
ATATAGCAGCTATCCGTACACGTTCGGAGGGGGGACCAAGCTGGAGCTGAAACG
T (SEQ ID NO:69)
   VH 
MKCNWVIFFLMAVVIGINSEVQLQQSGAELVRSGASVKLSCTASGFNIKDYFMHWV
KQRPEQGLEWIGWIDPENGDIEYAPKFQGKATMTADTSSNTAYLQLSSLTSEDTAV
YYCNAGQATYYFDYWGQGTTVTVSS (SEQ ID NO:70)
 
ATGAAATGCAACTGGGTCATCTTCTTCCTGATGGCAGTGGTTATAGGAATCAATT
CAGAGGTTCAGCTGCAGCAGTCTGGGGCAGAGCTTGTGAGGTCAGGGGCCTCAG
TCAAGTTGTCCTGCACAGCTTCTGGCTTCAACATTAAAGACTACTTTATGCACTG
GGTGAAGCAGAGGCCTGAACAGGGCCTGGAGTGGATTGGATGGATTGATCCTGA
GAATGGTGATACTGAATATGCCCCGAAGTTCCAGGGCAAGGCCACTATGACTGC
AGACACATCCTCCAACACAGCCTACCTGCAGCTCAGCAGCCTGACATCTGAGGA
CACTGCCGTCTATTACTGTAATGCAGGGCAGGCAACTTACTACTTTGACTACTGG
GGCCAAGGCACCACGGTCACCGTCTCGAGC (SEQ ID NO:71)
Antibody R
   VK 
METDTLRAKLRSHHEAACSAFRCEMDIQMNQSPSSLSASLGDTITITCHATQNINVW
LSWYQQKPGNIPTLLIYKTSNLHTGVPSRFSGSGSGTGFTLTISSLQSEDIATYYCQQG
QSFPYTFGGGTKLELKR (SEQ ID NO:72)
 
ATGGAGACAGACACACTCCGAGCGAAGCTTCGAAGCCACCATGAAGCTGCCTGT
TCTGCTTTTAGGTGTGAGATGTGACATCCAGATGAACCAGTCTCCATCCAGTCTG
TCTGCATCCCTTGGAGACACAATTACCATCACTTGCCATGCCACTCAGAACATTA
ATGTTTGGTTAAGCTGGTACCAGCAGAAACCAGGAAATATTCCTACACTTTTGAT
CTATAAGACTTCCAACTTGCACACAGGCGTCCCATCAAGGTTTAGTGGCAGTGGA
TCTGGAACAGGTTTCACATTAACCATCAGCAGCCTGCAGTCTGAAGACATTGCCA
CTTACTACTGTCAACAGGGTCAAAGTTTCCGTACACGTTCGGAGGAGGCACCAA
GCTGGAGCTGAAACGT (SEQ ID NO:73)
   VH 
MKCNWVIFFLMAVVIGINSEVQLQQSGAELVRSGASVKLSCTASGFNIKDYIMHWV
KQRPEQGLEWIGWIDPENGDTEYAPKFQGKATMTADTSSNTAYLQLSSLTSEDTAV
YYCNAGQATYYFDYWGQGTTVTVSS (SEQ ID NO:74)
 
ATGAAATGCAACTGGGTCATCTTCTTCCTGATGGCAGTGGTTATAGGAATCAATT
CAGAGGTTCAGCTGCAGCAGTCTGGGGCAGAGCTTGTGAGGTCAGGGGCCTCAG
TCAAGTTGTCCTGCACAGCTTCTGGCTTCAACATTAAAGACTACTTTATGCACTG
GGTGAAGCAGAGGCCTGAACAGGGCCTGGAGTGGATTGGATGGATTGATCCTGA
GAATGGTGATACTGAATATGCCCCGAAGTTCCAGGGCAAGGCCACTATGACTGC
AGACACATCCTCCAACACAGCCTACCTGCAGCTCAGCAGCCTGACATCTGAGGA
CACTGCCGTCTATTACTGTAATGCAGGGCAGGCAACTTACTACTTTGACTACTGG
GGCCAAGGCACCACGGTCACCGTCTCGAGC (SEQ ID NO:75)
Antibody S
   VK 
MESQTQVLMLLLLWVSGTCGDIMMSQSPSSLAVSVGEKVTMSCKSSQSLLYSNNQK
NYLAWYQQKPGQSPKLLISWASTRESGVPDRFTGSGSGTDFTLTISSVKAEDLAVYY
CQQYYDYPWTFGGGSKLELKR (SEQ ID NO:76)
 
ATGGAATCACAGACCCAGGTTCTTATGTTACTGCTGCTATGGGTATCTGGTACCT
GTGGGGACATTATGATGTCACAGTCTCCATCCTCCCTAGCTGTGTCAGTTGGAGA
 
GAAGGTTACTATGAGCTGCAAGTCCAGTCAGAGCCTTTTATATAGTAACAATCAA
AAGAACTACTTGGCCTGGTACCAGCAGAAACCAGGGCAGTCTCCTAAACTGCTG
ATTTCCTGGGCATCCACTAGGGAATCTGGGGTCCCTGATCGCTTCACAGGCAGTG
GATCTGGGACAGATTTCACTCTCACCATCAGCAGTGTGAAGGCTGAAGACCTGGC
TGTTTATTACTGTCAGCAATATTATGACTATCCGTGGACGTTCGGTGGAGGCTCC
AAGCTGGAGCTGAAACGT (SEQ ID NO:77)
   VH 
MGWNWIIFFLMAVVIGINSEVQLQQSGAELVRSGASVKLSCTASGFNIKDYIMHWV
KQRPEQGLEWIGWIDPENGDTEYAPKFQGKATMTADTSSNTAYLQLSSLTSEDTAV
YYCNAGQQATYYFDYWGQGTTVTVSS (SEQ ID NO:78)
 
ATGGGATGGAACTGGATCATCTTCTTCCTGATGGCAGTGGTTATAGGAATCAATT
CAGAGGTTCAGCTGCAGCAGTCTGGGGCAGAGCTTGTGAGGTCAGGGGCCTCAG
TCAAGTTGTCCTGCACAGCTTCTGGCTTCAACATTAAAGACTACTTTATGCACTG
GGTGAAGCAGAGGCCTGAACAGGGCCTGGAGTGGATTGGATGGATTGATCCTGA
GAATGGTGATACTGAATATGCCCCGAAGTTCCAGGGCAAGGCCACTATGACTGC
AGACACATCCTCCAACACAGCCTACCTGCAGCTCAGCAGCCTGACATCTGAGGA
CACTGCCGTCTATTACTGTAATGCAGGGCAGGCAACTTACTACTTTGACTACTGG
GGCCAAGGCACCACGGTCACCGTCTCGAGC (SEQ ID NO:79)
Antibody T
   VK 
MDMRTPAQFLGLLLLCFQGTRCDIQMTQTSSSLSASLGDRVTISCRTSQDINNFLNW
YQQKPDGTVKFLIHHTSRLKSGVPSRFSGSGSGTDYSLTISHLEPEDIATYYCQHYYN
LPWTFGGGTKLELKR (SEQ ID NO:80)
 
ATGGACATGAGGACCCCTGCTCAGTTCCTTGGTCTCCTGTTGCTCTGTTTTCAAGG
TACCAGATGTGATATCCAGATGACACAGACTTCATCTTCCCTGTCTGCCTCTCTGG
GAGACAGAGTCACCATCAGTTGCAGGACAAGTCAGGATATTAACAATTTTTTAA
ACTGGTATCAGCAGAAACCAGATGGAACTGTTAAATTCCTGATCCACCACACATC
AAGATTAAAGTCAGGAGTCCCATCAAGGTTCAGTGGCAGTGGGTCTGGGACAGA
TTATTCTCTCACCATCAGCCACCTGGAACCTGAAGATATTGCCACTTACTATTGTC
AGCACTATTATAATCTTCCGTGGACGTTCGGTGGAGGCACCAAGTTGGAGCTGAA
ACGT (SEQ ID NO:81)
   VH 
MGWSWTFLFLLSGTAGVHSEIQLQQTGPELVKPGASVKISCKASGYSFTDNIMVWV
KQSHGKSLEWIGNINPYYGSPSYNLKFKDKATLTVDKSSSTAYMQLNSLTSEDSAVY
YCAREGGNYGSLDNWGQGTSVTVSS (SEQ ID NO:82)
 
ATGGGATGGAGCTGGACCTTTCTCTTCCTCCTGTCAGGAACTGCAGGTGTCCACT
CTGAGATCCAGCTGCAGCAGACTGGACCTGAGCTGGTGAAGCCTGGGGCTTCAG
TGAAGATATCCTGCAAGGCTTCTGGTTATTCATTCACTGACAACATCATGGTCTG
GGTGAAGCAGAGCCATGGAAAGAGCCTTGAGTGGATTGGAAACATTAATCCTTA
CTATGGTAGTCCTAGTTACAATCTGAAGTTCAAGGACAAGGCCACATTGACTGTA
GACAAATCTTCCAGCACAGCCTACATGCAGCTCAATAGTCTGACATCTGAGGACT
 
CTGCAGTCTATTACTGTGCAAGAGAGGGGGGTAATTACGGTTCTCTGGACAACTG
GGGTCAAGGAACCTCGGTCACCGTCTCGAGC (SEQ ID NO:83)
Antibody U
   VK 
MKSQTQVLMLLLLWVSGTCGDIMMSQSPSSLAVSVGEKVTMSCKSSQSLLYSNNQK
NYLAWYQQKPGQSPKLLISWASTRESGVPDRFTGSGSGTDFTLTISSVKAEDLAVYY
CQQYYDYPWTFGGGSKLEIKR (SEQ ID NO:84)
 
ATGAAGTCACAGACCCAGGTTCTTATGTTACTGCTGCTATGGGTATCTGGTACCT
GTGGGGACATTATGATGTCACAGTCTCCATCCTCCCTAGCTGTGTCAGTTGGAGA
GAAGGTTACTATGAGCTGCAAGTCCAGTCAGAGCCTTTTATATAGTAACAATCAA
AAGAACTACTTGGCCTGGTACCAGCAGAAACCAGGGCAGTCTCCTAAACTGCTG
ATTTCCTGGGCATCCACTAGGGAATCTGGGGTCCCTGATCGCTTCACAGGCAGTG
GATCTGGGACAGATTTCACTCTCACCATCAGCAGTGTGAAGGCTGAAGACCTGGC
TGTTTATTACTGTCAGCAATATTATGACTATCCGTGGACGTTCGGTGGAGGCTCC
AAGCTGGAAATAAAACGT (SEQ ID NO:85)
   VH 
MGWSWTFLFLLSGTAGVHSEIQLQQTGPELVKPGASVKISCKASGYSFTDNIMVWV
KQSHGKSLEWIGNINPYYGSPSYNLKFKDKATLTVDKSSSTAYMQLNSLTSEDSAVY
YCAREGGNYGSLDNWGQGTSVTVSS (SEQ ID NO:86)
 
ATGGGATGGAGCTGGACCTTTCTCTTCCTCCTGTCAGGAACTGCAGGTGTCCACT
CTGAGATCCAGCTGCAGCAGACTGGACCTGAGCTGGTGAAGCCTGGGGCTTCAG
TGAAGATATCCTGCAAGGCTTCTGGTTATTCATTCACTGACAACATCATGGTCTG
GGTGAAGCAGAGCCATGGAAAGAGCCTTGAGTGGATTGGAAACATTAATCCTTA
CTATGGTAGTCCTAGTTACAATCTGAAGTTCAAGGACAAGGCCACATTGACTGTA
GACAAATCTTCCAGCACAGCCTACATGCAGCTCAATAGTCTGACATCTGAGGACT
CTGCAGTCTATTACTGTGCAAGAGAGGGGGGTAATTACGGTTCTCTGGACAACTG
GGGTCAAGGAACCTCGGTCACCGTCTCGAGC (SEQ ID NO:87)
Antibody V
   VK 
MSPAQFLGLLLLCFQGTRCDIQMTQTSSSLSASLGDRVTISCRTSQDINNFLNWYQQK
PDGTVKFLIHHTSRLKSGVPSRFSGSGSGTDYSLTISHLEPEDIATYYCQHYYNLPWIF
GGGTKLEIKR (SEQ ID NO:88)
 
ATGAGTCCTGCCCAGTTCCTTGGTCTCCTGTTGCTCTGTTTTCAAGGTACCAGATG
TGATATCCAGATGACACAGACTTCATCTTCCCTGTCTGCCTCTCTGGGAGACAGA
GTCACCATCAGTTGCAGGACAAGTCAGGATATTAACAATTTTTTAAACTGGTATC
AGCAGAAACCAGATGGAACTGTTAAATTCCTGATCCACCACACATCAAGATTAA
AGTCAGGAGTCCCATCAAGGTTCAGTGGCAGTGGGTCTGGGACAGATTATTCTCT
CACCATCAGCCACCTGGAACCTGAAGATATCGCCACTTACTATTGTCAGCACTAT
TATAATCTTCCGTGGACGTTCGGTGGAGGCACAAAGTTGGAAATAAAACGT (SEQ
ID NO:89)
   VH 
MGWSWTFLFLLSGTAGVHSEVQLVESGGGLVKPGGSLKLSCAVSGFSLSYYYMSW
VRQTPEKRLEWIGTISIDGSTYYASWAKGRFTISKDSTTVYLQMSSLKSEDTAMYYC
ARGHINTGMDPWGQGTPVTVSS (SEQ ID NO:90)
 
ATGGGATGGAGCTGGACCTTTCTCTTCCTCCTGTCAGGAACTGCAGGTGTCCACT
CTGAGGTTCAGCTCGTTGAATCCGGAGGCGGACTCGTGAAGCCCGGGGGCAGTC
TTAAACTGTCCTGCGCCGTCAGCGGGTTTTCCCTCAGTTACTATTACATGTCATGG
GTGCGGCAGACCCCCGAGAAAAGGTTGGAATGGATTGGCACCATCTCAATTGAC
GGAAGCACATATTACGCATCTTGGGCCGAGGGTCGCTTCACAATTTCTAAGGACA
GTACTACCGTGTATCTGCAGATGTCCAGCCTTAAGAGCGAAGATACAGCCATGTA
CTACTGTGCTAGAGGGCACATCAACACTGGAATGGATCCTTGGGGCCAGGGCAC
CCCGGTCACAGTCTCGAGC (SEQ ID NO:91)
Antibody W
   VK 
MKLPVRLLVLMFWIPASSSDVLMTQNPLSLPVSLGDQASISCRSSQTIVHSNGNTYLE
WYLQKPGQSPKLLIYEVSNRFSGVPDRFSGSGSGTDFTLKISRVEAEDLGIYYCFQGS
HFPHTFGGGTKLELKR (SEQ ID NO:92)
 
ATGAAGTTGCCTGTTAGGCTGTTGGTGCTGATGTTCTGGATTCCTGCTTCCAGCAG
TGATGTTTTGATGACCCAAAATCCACTCTCCCTGCCTGTCAGTCTTGGAGATCAA
GCCTCCATCTCCTGCAGATCAAGTCAGACCATTGTACATAGTAATGGAAACACCT
ATTTAGAATGGTACCTGCAGAAACCAGGCCAGTCTCCAAAGCTCCTGATCTACGA
AGTTTCCAACCGATTTTCTGGGGTCCCAGACAGGTTCAGTGGCAGTGGATCAGGG
ACAGATTTCACACTCAAAATCAGCAGAGTGGAGGCTGAGGATCTGGGAATTTAT
TACTGCTTTCAAGGTTCACATTTTCCGCACACGTTCGGAGGGGGGACCAAGCTGG
AACTGAAACGT (SEQ ID NO:93)
   VH 
MEWSGVIFFLMAVVIGINSEVQLQQSGAEFVRSGASVKLSCTASGFNIKDYYMYWV
KQRPEQGLEWIGWIDPENGDTECAPKFQGKATMTADTSSNTAYLQLSSLTSEDTAVY
YCNADRYDEGAASDYGMDYWGQGSSVTVSS (SEQ ID NO:94)
 
ATGGAATGGAGCGGGGTCATCTTCTTCCTGATGGCAGTGGTTATAGGAATCAATT
CAGAGGTTCAGCTGCAGCAGTCTGGGGCAGAGTTTGTGAGGTCAGGGGCCTCAG
TCAAGTTGTCCTGCACAGCTTCTGGCTTCAACATTAAAGACTACTATATGTATTG
GGTGAAACAGAGGCCTGAACAGGGCCTGGAGTGGATTGGATGGATTGATCCTGA
GAATGGTGATACTGAATGTGCCCCGAAGTTCCAGGGCAAGGCCACTATGACTGC
AGACACATCCTCCAACACAGCCTACCTGCAACTCAGCAGCCTGACATCTGAGGA
CACTGCCGTCTATTACTGTAATGCCGATAGGTACGACGAGGGGGCCGCGTCGGA
CTATGGTATGGACTACTGGGGTCAAGGAAGTTCAGTCACAGTCTCGAGC (SEQ ID
NO:95)
Antibody X
   VK 
MKLPVRLLVLMFWIPASSSDVLMTQNPLSLPVSLGDQASISCRSSQSIVHSNGNTHLE
WYLQKPGQSPKLLIYKVSNRFSGVPDRFSGSGSGTDFTLKISRVEAEDLGVYYCFQGS
HAPHTFGGGTKLELKR (SEQ ID NO:96)
 
ATGAAGTTGCCTGTTAGGCTGTTGGTGCTGATGTTCTGGATTCCTGCTTCCAGCAG
TGATGTTTTGATGACCCAAAATCCACTCTCCCTGCCTGTCAGTCTTGGAGATCAA
GCCTCCATCTCTTGCAGATCTAGTCAGAGCATTGTACATAGTAATGGAAACACCC
ATTTAGAATGGTACCTGCAGAAACCAGGCCAGTCTCCAAAGCTCCTGATCTACAA
AGTTTCCAACCGATTTTCTGGGGTCCCAGACAGGTTCAGTGGCAGTGGATCAGGG
ACAGATTTCACACTCAAGATCAGCAGAGTGGAGGCTGAGGATCTGGGAGTTTAT
TACTGCTTTCAAGGTTCACATGCTCCGCACACGTTCGGAGGGGGGACCAAGCTGG
AACTGAAACGT (SEQ ID NO:97)
   VH 
MKCSWVIFFLMAVVIGINSEVQLQQFGAEFVRSGASVKLSCTASGFNIKDYYMYWV
KQRPEQGLEWIGWIDPGNGDTECAPKFQGKATMTADTSSNTAYLQLSSLTSEDTAV
YYCNADRYDEGAASDYAVDYWGQGSSVTVSS (SEQ ID NO:98)
 
ATGAAATGCAGCTGGGTCATCTTCTTCCTGATGGCAGTGGTTATAGGAATCAATT
CAGAGGTTCAGCTGCAGCAGTTTGGGGCAGAGTTTGTGAGGTCAGGGGCCTCAG
TCAAGTTGTCCTGCACAGCTTCTGGCTTCAACATTAAAGACTACTATATGTATTG
GGTGAAACAGAGGCCTGAACAGGGCCTGGAGTGGATTGGATGGATTGATCCTGG
GAATGGTGATACTGAATGTGCCCCGAAGTTCCAGGGCAAGGCCACTATGACTGC
AGACACATCCTCCAACACAGCCTACCTGCAGCTCAGCAGCCTGACATCTGAGGA
CACTGCCGTCTATTACTGTAATGCCGATAGATACGACGAGGGGGCCGCGTCGGA
CTATGCTGTGGACTACTGGGGTCAAGGAAGTTCGGTCACAGTCTCGAGC (SEQ ID
NO:99)
Antibody Y
   VK 
MKLPVRLLVLMFWIPASSSDVLMTQNPLSLPVSLGDQASISCRSSQTIVHSNGNTYLE
WYLQKPGQSPKLLIYLVSNRFSGVPDRFSGSGSGTDFTLKISRVGAEDLGIYYCFQGS
LFPHTFGGGTKLELKR (SEQ ID NO:100)
 
ATGAAGTTGCCTGTTAGGCTGTTGGTGCTGATGTTCTGGATTCCTGCTTCCAGCAG
TGATGTTTTGATGACCCAAAATCCACTCTCCCTGCCTGTCAGTCTTGGAGATCAA
GCCTCCATCTCTTGCAGATCTAGTCAGACCATTGTACATAGTAATGGAAACACCT
ATTTAGAATGGTACCTGCAGAAACCAGGCCAGTCTCCAAAGCTCCTGATCTACGA
AGTTTCCAACCGATTTTCTGGGGTCCCAGACAGGTTCAGTGGCAGTGGCTCAGGG
ACAGATTTCACACTCAAAATCAGCAGAGTGGGGGCTGAAGATCTGGGAATTTAT
TACTGCTTTCAAGGTTCACTTTTTCCGCACACGTTCGGAGGGGGGACCAAGTTGG
AGCTCAAACGT (SEQ ID NO:101)
   VH

MEWSCIIFFLMAVVIGINSEVQLQQSGAEFVRSGASVKLSCTASGFNIKDYYMYWVK
QRPEQGLEWIGWIDPENGDTECAPKFQGKATMTADTSSNTAYLQLSSLTSEDTAVYY
CNADRYDEGAASDYAVDYWGQGSSVTVSS (SEQ ID NO:102)

ATGGAATGGAGCTGTATCATCTTCTTCCTGATGGCAGTGGTTATAGGAATCAATT
CAGAGGTTCAGCTGCAGCAGTCTGGGGCAGAGTTTGTGAGGTCAGGGGCCTCAG
TCAAGTTGTCCTGCACAGCTTCTGGCTTCAACATTAAAGACTACTATATGTATTG
GGTGAAACAGAGGCCTGAACAGGGCCTGGAGTGGATTGGATGGATTGATCCTGA
GAATGGTGATACTGAATGTGCCCCGAAGTTCCAGGGCAAGGCCACTATGACTGC
AGACACATCCTCCAACACAGCCTACCTGCAGCTCAGCAGCCTGACATCTGAGGA
CACTGCCGTCTATTACTGTAATGCCGATAGATACGACGAGGGGGCCGCGTCGGA
CTATGCTGTGGACTACTGGGGTCAAGGAAGCTCAGTCACCGTCTCGAGC (SEQ ID
NO:103)

Table 1 shows the CDR's and sequence identifiers for Antibodies A-Y.

**Table 1**

| **Antibody** | **CDR 1** | **CDR2** | **CDR3** |
|---|---|---|---|
| A VL | QSSQRVFNNNELS | EASTLAS | LGGYDDDGDNA |
| | (SEQ ID NO:106) | (SEQ ID NO:107) | (SEQ ID NO:108) |
| AVH | NYYMT | LIYPDGSTFYANWAE G (SEQ ID NO:110) | EGGAGDNTGTEYY YGVDL (SEQ ID NO:111) |
| | (SEQ ID NO:109) | | |
| B VL | QASENVYDKSALS | LASTLAS | AGYKSSIIDGTA |
| | (SEQ ID NO:112) | (SEQ ID NO:113) | (SEQ ID NO:114) |
| B VH | NNALS | AIGAGGNTYYASWA KG (SEQ ID NO:116) | GDLPGGI |
| | (SEQ ID NO:115) | | (SEQ ID NO:117) |
| C VL | QASQSISPALA | DVSKLAS | QSYYGINSNSYGNI |
| | (SEQ ID NO:118) | (SEQ ID NO:119) | (SEQIDNO:120) |
| C VH | SYAMG | YIYGNYNKYYASWA KG (SEQ ID NO:122) | GGAMDV |
| | (SEQ ID NO:121) | | (SEQ ID NO:123) |
| D VL | QASESISTWLA | RASTLAS | QQGWNINNIDNI |
| | (SEQ ID NO:124) | (SEQ ID NO:125) | (SEQ ID NO:126) |
| D VH | SYSMY | FILSATAVSYATWAK G (SEQ ID NO:128) | DRDGGTTLDGFDP |
| | (SEQ ID NO:127) | | (SEQ ID NO:129) |
| EVL | QSSQSVVNNNNLA | FASTLAS | QGTYLSDDWSDA |
| | (SEQ ID NO:130) | (SEQ ID NO:131) | (SEQ ID NO:132) |
| E VH | SSVMN | AIWSGGYTYYATWA KG (SEQ ID NO:134) | GQFGASGGGDVL |
| | (SEQ ID NO:133) | | (SEQ ID NO:135) |
| F VL | QSSPSVDNNNLLS | DASTLAS | VAGYGKRSRDIRV |
| | (SEQ ID N4:136) | (SEQIDNO:137) | (SEQ ID NO:138) |
| F VH | SHNMQ | IIYPSNNAYYSNWAK G (SEQ ID NO:140) | DINSAI |
| | (SEQ ID NO:139) | | (SEQ ID NO:141) |
| GVL | QASESIYSNLA | DASDLAS | QCSWGGSTYGFA |
| | (SEQ ID NO.142) | (SEQ ID NO:143) | (SEQ ID NO:144) |
| G VH | IYVMT | SIDADDSAYYATWA TS (SEQ ID NO:146) | GLYANGGPFTL |
| | (SEQ ID NO:145) | | (SEQ ID NO:147) |
| H VL | QSSPSVYNRNQLS | TASTLAS | QGYYNTGSDTYA |
| | (SEQ ID NO:148) | (SEQ ID NO:149) | (SEQ ID NO:150) |
| H VH | NYDMS | FINTVGYAYYASWA KG (SEQ ID NO:152) | LDNYYTWGI |
| | (SEQ ID NO:151) | | (SEQ ID NO:153) |
| I VL | QAAEDIYSSLA | AASILAS | QTNYGISSYGAA |
| | (SEQ ID NO:154) | (SEQ ID NO:155) | (SEQ ID NO:156) |
| I VH | NKPIT | WISTTGSAYYASWA KG (SEQ ID NO:158) | YSSDYGHHDL |
| | (SEQ ID NO:157) | | (SEQ ID NO:159) |
| J VL | QASQSVYRDYLS | GASNLAS | LGGFSGNINT |
| | (SEQ ID NO:160) | (SEQ ID NO:161) | (SEQ ID NO:162) |
| J VH | NYHMC | IISNTGYTYYSSWAK G (SEQ ID NO:164) | DRLANL |
| | (SEQ ID NO:163) | | (SEQ ID NO:165) |
| K VL | QASQSISTYLK | SASTLTS | QSNAGSSSSSCGYA |
| | (SEQ ID NO:166) | (SEQ ID NO:167) | (SEQ ID NO:168) |
| K VH | YYGMG | IISGIGNTYYPTWAK G (SEQ ID NO:170) | GDF |
| | (SEQ ID NO:169) | | (SEQ ID NO:171) |
| L VL | QASQSISRYLK | SASTLTS | QSNAGSSSSSCGYA |
| | (SEQ ID NO:172) | (SEQ ID NO:173) | (SEQ ID NO:174) |
| L VH | YYGMG | IISGSGNTYYASWAK G (SEQ ID NO:176) | GDF |
| | (SEQ ID NO:175) | | (SEQ ID NO:177) |
| M VL | QSSQSVYSNYLS | VASSLAS | GGFQKYIDDGGA |
| | (SEQ ID NO:178) | (SEQ ID NO:179) | (SEQ ID NO:180) |
| M VH | TYAMS | IMSSSGSAYYASWAK G (SEQ ID NO:182) | SSSFGL |
| | (SEQ ID NO:181) | | (SEQ ID NO:183) |
| N VK | SVSSSISSTNLH | GTSNLAS | QQWSTTYT |
| | (SEQ ID NO:184) | (SEQ ID NO:185) | (SEQ ID NO:186) |
| N VH | DYYIH | RIDPDNGESTYVPKF QG (SEQ ID NO:188) | EGLDYGDYYAVDY |
| | (SEQ ID NO:187) | | (SEQ ID NO:189) |
| O VK | RASESVDSYGNSFMH | RASNLES | QQNYEDPLT |
| | (SEQ ID NO:190) | (SEQ ID NO:191) | (SEQ ID NO:192) |
| O VH | NYLIE | VINPGSGIINYNEKFK I (SEQ ID NO:194) | DWDTFYSYEREVY AMDY (SEQ ID NO:195) |
| | (SEQ ID NO:193) | | |
| P VK | RASQSIGTNIH | FASESIS | QQSNSWPLT |
| | (SEQ ID NO:196) | (SEQ ID NO:197) | (SEQ ID NO:198) |
| P VH | DYYVH | WIDPENGDTEYAPKF QG (SEQ ID NO:200) | GYGNFYFDY |
| | (SEQ ID NO:199) | | (SEQ ID NO:201) |
| Q VK | KASQDVGTAVA | WASTRHT | QQYSSYPYT |
| | (SEQ ID NO:202) | (SEQ ID NO:203) | (SEQ ID NO:204) |
| Q VH | DYFMH | WIDPENGDTEYAPKF QG (SEQ ID NO:206) | GQATYYFDY |
| | (SEQ ID NO:205) | | (SEQ ID NO:207) |
| R VK | HATQNINVWLS | KTSNLHT | QQGQSFPYT |
| | (SEQ ID NO:208) | (SEQ ID NO:209) | (SEQ ID NO:210) |
| R VH | DYFMH | WIDPENGDTEYAPKF QG (SEQ ID NO:212) | GQATYYFDY |
| | (SEQ ID NO:211) | | (SEQ ID NO:213) |
| S VK | KSSQSLLYSNNQKNYL A (SEQ ID NO:214) | WASTRES | QQYYDYPWT |
| | | (SEQ ID NO:215) | (SEQ ID NO:216) |
| S VH | DYFMH | WIDPENGDTEYAPKF | GQATYYFDY |
| | (SEQ ID NO:217) | (SEQ ID NO:218)QG | SEQ ID NO:219) |
| T VK | RTSQDINNFLN | HTSRLKS | QHYYNLPWT |
| | (SEQ ID NO:220) | (SEQ ID NO:221) | (SEQ ID NO:222) |
| T VH | DNIMV | NINPYYGSPSYNLKF KD (SEQ ID NO:224) | EGGNYGSLDN |
| | (SEQ ID NO:223) | | (SEQ ID NO:225) |
| U VK | KSSQSLLYSNNQKNYL A (SEQ ID NO:226) | WASTRES | QQYYDYPWT |
| | | (SEQ ID NO:227) | (SEQ ID NO:228) |
| U VH | DNIMV | NINPYYGSPSYNLKF KD (SEQ ID NO:230) | EGGNYGSLDN |
| | (SEQ ID NO:229) | | (SEQ ID NO:231) |
| V VK | RTSQDINNFLN | HTSRLKS | QHYYNLPWT |
| | (SEQ ID NO:232) | (SEQ ID NO:233) | (SEQ ID NO:234) |
| V VH | YYYMS | TISIDGSTYYASWAE G (SEQ ID NO:236) | GHINTGMDP |
| | (SEQ ID NO:235) | | (SEQ ID NO:237) |
| W VK | RSSQTIVHSNGNTYLE | EVSNRFS | FQGSHFPHT |
| | (SEQ ID NO:238) | (SEQ ID NO:239) | (SEQ ID NO:240) |
| W VH | DYYMY | WIDPENGDTECAPKF QG (SEQ ID NO:242) | DRYDEGAASDYGM DY (SEQ ID NO:243) |
| | (SEQ ID NO:241) | | |
| X VK | RSSQSIVHSNGNTHLE | KVSNRFS | FQGSHAPHT |
| | (SEQ ID NO:244) | (SEQ ID NO:245) | (SEQ ID NO:246) |
| X VH | DYYMY | WIDPGNGDTECAPKF QG (SEQ ID NO:248) | DRYDEGAASDYAV DY (SEQ ID NO:249) |
| | (SEQ ID NO:247) | | |
| Y VK | RSSQTIVHSNGNTYLE | EVSNRFS | FQGSLFPHT |
| | (SEQ ID NO:250) | (SEQ ID NO:251) | (SEQ ID NO:252) |
| Y VH | DYYMY | WIDPENGDTECAPKF QG (SEQ ID NO:254) : | DRYDEGAASDYAV DY (SEQ ID NO:255) |
| | (SEQ ID NO:253) | | |

The following table, Table 2, provides the SEQ ID NOs for the antibody polynucleotides and polypeptides disclosed above, without leader sequences.

**Table 2**

| Antibody | POLYPEPTIDES | | POLYNUCLEOTIDES | |
|---|---|---|---|---|
| | SEQ ID NO with leader | SEQ ID NO without leader | SEQ ID NO with leader | SEQ ID NO without leader |
| A VK | 2 | 256 | 3 | 257 |
| A VH | 4 | 258 | 5 | 259 |
| B VK | 6 | 260 | 7 | 261 |
| B VH | 8 | 262 | 9 | 263 |
| C VK | 10 | 264 | 11 | 265 |
| C VH | 12 | 266 | 13 | 267 |
| D VK | 14 | 268 | 15 | 269 |
| D VH | 16 | 270 | 17 | 271 |
| E VK | 18 | 272 | 19 | 273 |
| E VH | 20 | 274 | 21 | 275 |
| F VK | 22 | 276 | 23 | 277 |
| F VH | 24 | 278 | 25 | 279 |
| G VK | 26 | 280 | 27 | 281 |
| G VH | 28 | 282 | 29 | 283 |
| H VK | 30 | 284 | 31 | 285 |
| H VH | 32 | 286 | 33 | 287 |
| I VK | 34 | 288 | 35 | 289 |
| I VH | 36 | 290 | 37 | 291 |
| J VK | 38 | 292 | 39 | 293 |
| J VH | 40 | 294 | 41 | 295 |
| K VK | 42 | 296 | 43 | 297 |
| K VH | 44 | 298 | 45 | 299 |
| L VK | 46 | 300 | 47 | 301 |
| L VH | 48 | 302 | 49 | 303 |
| M VK | 50 | 304 | 51 | 305 |
| M VH | 52 | 306 | 53 | 307 |
| N VK | 56 | 308 | 57 | 309 |
| N VH | 58 | 310 | 59 | 311 |
| O VK | 60 | 312 | 61 | 313 |
| O VH | 62 | 314 | 63 | 315 |
| P VK | 64 | 316 | 65 | 317 |
| P VH | 66 | 318 | 67 | 319 |
| Q VK | 68 | 320 | 69 | 321 |
| Q VH | 70 | 322 | 71 | 323 |
| R VK | 72 | 324 | 73 | 325 |
| R VH | 74 | 326 | 75 | 327 |
| S VK | 76 | 328 | 77 | 329 |
| S VH | 78 | 330 | 79 | 331 |
| T VK | 80 | 332 | 81 | 333 |
| T VH | 82 | 334 | 83 | 335 |
| U VK | 84 | 336 | 85 | 337 |
| U VH | 86 | 338 | 87 | 339 |
| V VK | 88 | 340 | 89 | 341 |
| V VH | 90 | 342 | 91 | 343 |
| W VK | 92 | 344 | 93 | 345 |
| W VH | 94 | 346 | 95 | 347 |
| X VK | 96 | 348 | 97 | 349 |
| X VH | 98 | 350 | 99 | 351 |
| Y VK | 100 | 352 | 101 | 353 |
| Y VH | 102 | 354 | 103 | 355 |

An oligopeptide or polypeptide is within the scope of the invention if it has an amino acid sequence that is at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identical to least one of the CDR's of Table 2 above; and/or to a CDR of a sclerostin binding agent that cross-blocks the binding of at least one of antibodies A-Y to sclerostin; and/or is cross-blocked from binding to sclerostin by at least one of antibodies A-Y; and/or to a CDR of a sclerostin binding agent wherein the binding agent can block the inhibitory effect of sclerostin in a cell based mineralization assay (*i.e*., a sclerostin neutralizing binding agent); and/or to a CDR of a sclerostin binding agent that binds to a Loop 2 epitope.

Sclerostin binding agent polypeptides and antibodies are within the scope of the invention if they have amino acid sequences that are at least 85%, 86%, 87%, 88%, 89%, 90%, 91%, 92%, 93%, 94%%, 95%, 96%, 97%, 98% or 99% identical to a variable region of at least one of antibodies A-Y to sclerostin; and/or are cross-blocked from binding to sclerostin by at least one of antibodies A-Y; and/or can block the inhibitory effect of sclerostin in a cell based mineralization assay (*i.e.,* a sclerostin neutralizing binding agent).

Polynucleotides encoding sclerostin binding agents are within the scope of the invention if they have polynucleotide sequences that are at least 85%, 86%, 87%, 88%, 89%, 90%, 91%, 92%, 93%, 94%, 95%, 96%, 97%, 98% or 99% identical to a polynucleotide encoding a variable region of at least one of antibodies A-Y, and wherein the encoded sclerostin binding agents cross-block the binding of at least one of antibodies A-Y to sclerostin; and/or are cross-blocked from binding to sclerostin by at least one of antibodies A-Y; and/or can block the inhibitory effect of sclerostin in a cell based mineralization assay (*i.e.* a sclerostin neutralizing binding agent); and/or bind to a Loop 2 epitope.

Antibodies according to the invention may have a binding affinity for human sclerostin of less than or equal to 1 x 10⁻⁷ M, less than or equal to 1 x 10⁻⁸ M, less than or equal to 1 x 10⁻⁹ M, less than or equal to 1 x 10⁻¹⁰ M, less than or equal to 1 x 10⁻¹¹ M, or less than or equal to 1 x 10⁻¹² M.

The affinity of a binding agent such as an antibody or binding partner, as well as the extent to which a binding agent (such as an antibody) inhibits binding, can be determined by one of ordinary skill in the art using conventional techniques, for example, those described by Scatchard et al., Ann. N. Y. Acad. Sci., 51:660-672 (1949) or by surface plasmon resonance (SPR; BIAcore, Biosensor, Piscataway, NJ). For surface plasmon resonance, target molecules are immobilized on a solid phase and exposed to ligands in a mobile phase running along a flow cell. If ligand binding to the immobilized target occurs, the local refractive index changes, leading to a change in SPR angle, which can be monitored in real time by detecting changes in the intensity of the reflected light. The rates of change of the SPR signal can be analyzed to yield apparent rate constants for the association and dissociation phases of the binding reaction. The ratio of these values gives the apparent equilibrium constant (affinity) (*see, e.g.,* Wolff et al., Cancer Res., 53:2560-65 (1993)).

An antibody according to the present invention may belong to any immunoglobin class, for example IgG, IgE, IgM, IgD, or IgA. It may be obtained from or derived from an animal, for example, fowl (*e.g*., chicken) and mammals, which includes, but is not limited, to a mouse, rat, hamster, rabbit, or other rodent, cow, horse, sheep, goat, camel, human, or other primate. The antibody may be an internalizing antibody. Production of antibodies is disclosed generally in U.S. Patent Publication No. 2004/0146888 A1.

### Characterization Assays

In the methods described above to generate antibodies according to the invention, including the manipulation of the specific antibody A-Y CDRs into new frameworks and/or constant regions, appropriate assays are available to select the desired antibodies or binding agents (*i.e*., assays for determining binding affinity to sclerostin; cross-blocking assays; Biacore-based "human sclerostin peptide epitope competition binding assay;" MC3T3-E1 cell based assay; and/or *in vivo* assays).

### CROSS-BLOCKING ASSAYS

The terms "cross-block," "cross-blocked" and "cross-blocking" are used interchangeably herein to mean the ability of an antibody or other binding agent to interfere with the binding of other antibodies or binding agents to sclerostin.

The extent to which an antibody or other binding agent is able to interfere with the binding of another to sclerostin, and therefore whether it can be said to cross-block according to the invention, can be determined using competition binding assays. One particularly suitable quantitative assay uses a Biacore machine which can measure the extent of interactions using surface plasmon resonance technology, Another suitable quantitative cross-blocking assay uses an ELISA-based approach to measure competition between antibodies or other binding agents in terms of their binding to sclerostin.

### BIACORE CROSS-BLOCKING ASSAY

The following generally describes a suitable Biacore assay for determining whether an antibody or other binding agent cross-blocks or is capable of cross-blocking according to the invention. For convenience, reference is made to two antibodies, but it will be appreciated that the assay can be used with any of the sclerostin binding agents described herein. The Biacore machine (for example the Biacore 3000) is operated in line with the manufacturer's recommendations.

Thus, in one cross-blocking assay, sclerostin is coupled to a CM5 Biacore chip using standard amine coupling chemistry to generate a sclerostin-coated surface. Typically 200-800 resonance units of sclerostin would be coupled to the chip (an amount that gives easily measurable levels of binding but that is readily saturable by the concentrations of test reagent being used).

The two antibodies (termed 1* and 2*) to be assessed for their ability to cross-block each other are mixed at a one to one molar ratio of binding sites in a suitable buffer to create the test mixture. When calculating the concentrations on a binding site basis the molecular weight of an antibody is assumed to be the total molecular weight of the antibody divided by the number of sclerostin binding sites on that antibody.

The concentration of each antibody in the test mix should be high enough to readily saturate the binding sites for that antibody on the sclerostin molecules captured on the Biacore chip. The antibodies in the mixture are at the same molar concentration (on a binding basis) and that concentration would typically be between 1.00 and 1.5 micromolar (on a binding site basis).

Separate solutions containing antibody 1* alone and antibody 2* alone are also prepared. Antibody 1* and antibody 2* in these solutions should be in the same buffer and at the same concentration as in the test mix.

The test mixture is passed over the sclerostin-coated Biacore chip and the total amount of binding recorded. The chip is then treated in such a way as to remove the bound antibodies without damaging the chip-bound sclerostin. Typically this is done by treating the chip with 30 mM HCl for 60 seconds.

The solution of antibody 1* alone is then passed over the sclerostin-coated surface and the amount of binding recorded. The chip is again treated to remove all of the bound antibody without damaging the chip-bound sclerostin.

The solution of antibody 2* alone is then passed over the sclerostin-coated surface and the amount of binding recorded.

The maximum theoretical binding of the mixture of antibody 1* and antibody 2* is next calculated, and is the sum of the binding of each antibody when passed over the sclerostin surface alone. If the actual recorded binding of the mixture is less than this theoretical maximum then the two antibodies are cross-blocking each other

Thus, in general, a cross-blocking antibody or other binding agent according to the invention is one which will bind to sclerostin in the above Biacore cross-blocking assay such that during the assay and in the presence of a second antibody or other binding agent of the invention the recorded binding is between 80% and 0.1 % (*e.g.,* between 80% and 4%) of the maximum theoretical binding, specifically between 75% and 0.1% (*e.g*., between 75% and 4%) of the maximum theoretical binding, and more specifically between 70% and 0.1% (*e.g*., between 70% and 4%) of maximum theoretical binding (as just defined above) of the two antibodies or binding agents in combination.

The Biacore assay described above is a primary assay used to determine if antibodies or other binding agents cross-block each other according to the invention. On rare occasions particular antibodies or other binding agents may not bind to sclerostin coupled via amine chemistry to a CM5 Biacore chip (this usually occurs when the relevant binding site on sclerostin is masked or destroyed by the coupling to the chip). In such cases cross-blocking can be determined using a tagged version of Sclerostin, for example N-terminal His-tagged Sclerostin (R & D Systems, Minneapolis, MN, USA; 2005 cat# 1406-ST-025). In this particular format, an anti-His antibody would be coupled to the Biacore chip and then the His-tagged sclerostin would be passed over the surface of the chip and captured by the anti-His antibody. The cross blocking analysis would be carried out essentially as described above, except that after each chip regeneration cycle, new His-tagged sclerostin would be loaded back onto the anti-His antibody coated surface. In addition to the example given using N-terminal His-tagged sclerostin, C-terminal His-tagged sclerostin could alternatively be used. Furthermore, various other tags and tag binding protein combinations that are known in the art could be used for such a cross-blocking analysis (*e.g*., HA tag with anti-HA antibodies; FLAG tag with anti-FLAG antibodies; and/or biotin tag with streptavidin).

### ELISA-BASED CROSS-BLOCKING ASSAY

The following generally describes an ELISA assay for determining whether an anti-sclerostin antibody or other sclerostin binding agent cross-blocks or is capable of cross-blocking according to the invention. For convenience, reference is made to two antibodies (Ab-1 and Ab-2), but it will be appreciated that the assay can be used with any of the sclerostin binding agents described herein.

The general principle of the assay is to have an anti-sclerostin antibody coated onto the wells of an ELISA plate. An excess amount of a second, potentially cross-blocking, anti-sclerostin antibody is added in solution (*i.e*., not bound to the ELISA plate). A limited amount of sclerostin is then added to the wells. The coated antibody and the antibody in solution compete for binding of the limited number of sclerostin molecules. The plate is washed to remove sclerostin that has not been bound by the coated antibody and to also remove the second, solution phase antibody as well as any complexes formed between the second, solution phase antibody and sclerostin. The amount of bound sclerostin is then measured using an appropriate sclerostin detection reagent. An antibody in solution that is able to cross-block the coated antibody will be able to cause a decrease in the number of sclerostin molecules that the coated antibody can bind relative to the number of sclerostin molecules that the coated antibody can bind in the absence of the second, solution phase, antibody.

This assay is described below in more detail for Ab-1 and Ab-2, In the instance where Ab-1 is chosen to be the immobilized antibody, it is coated onto the wells of the ELISA plate, after which the plates are blocked with a suitable blocking solution to minimize non-specific binding of reagents that are subsequently added. An excess amount of Ab-2 is then added to the ELISA plate such that the moles of Ab-2 sclerostin binding sites per well are at least 10 fold higher than the moles of Ab-1 sclerostin binding sites that were used, per well, during the coating of the ELISA plate. Sclerostin is then added such that the moles of sclerostin added per well are at least 25-fold lower than the moles of Ab-2 sclerostin binding sites that were used for coating each well. Following a suitable incubation period, the ELISA plate is washed and a sclerostin detection reagent is added to measure the amount of sclerostin specifically bound by the coated anti-sclerostin antibody (in this case Ab-1). The background signal for the assay is defined as the signal obtained in wells with the coated antibody (in this case Ab-1), second solution phase antibody (in this case Ab-2), sclerostin buffer only (*i.e*., no sclerostin) and sclerostin detection reagents. The positive control signal for the assay is defined as the signal obtained in wells with the coated antibody (in this case Ab-1), second solution phase antibody buffer only (*i.e.,* no second solution phase antibody), sclerostin and sclerostin detection reagents. The ELISA assay needs to be run in such a manner so as to have the positive control signal be at least 6 times the background signal.

To avoid any artifacts resulting from the choice of which antibody to use as the coating antibody and which to use as the second (competitor) antibody (*e.g*., significantly different affinities between Ab-1 and Ab-2 for sclerostin), the cross-blocking assay can be run in two formats:
1) format 1 is where Ab-1 is the antibody that is coated onto the ELISA plate and Ab-2 is the competitor antibody that is in solution
   and
2) format 2 is where Ab-2 is the antibody that is coated onto the ELISA plate and Ab-1 is the competitor antibody that is in solution.

Ab-1 and Ab-2 are defined as cross-blocking if, either in format 1 or in format 2, the solution phase anti-sclerostin antibody is able to cause a reduction of between 60% and 100%, specifically between 70% and 100%, and more specifically between 80% and 100%, of the sclerostin detection signal (*i.e*., the amount of sclerostin bound by the coated antibody) as compared to the sclerostin detection signal obtained in the absence of the solution phase anti-sclerostin antibody (*i.e*., the positive control wells).

An example of such an ELISA-based cross blocking assay can be found in Example 3 ("ELISA-based cross-blocking assay").

### CELL-BASED NEUTRALIZATION ASSAY

Mineralization by osteoblast-lineage cells in culture, either primary cells or cell lines, is used as an *in vitro* model of bone formation. Mineralization takes from about one to six weeks to occur beginning with the induction of osteoblast-lineage cell differentiation by one or more differentiation agents. The overall sequence of events involves cell proliferation, differentiation, extracellular matrix production, matrix maturation, and finally deposition of mineral, which refers to crystallization and/or deposition of calcium phosphate, This sequence of events starting with cell proliferation and differentiation, and ending with deposition of mineral, is referred to herein as mineralization. Measurement of calcium (mineral) is the output of the assay.

MC3T3-E1 cells (Sudo et al. "In vitro differentiation and calcification in a new clonal osteogenic cell line derived from newborn mouse calvaria. " J. Cell Biol., 96:191-198 (1983)) and subclones of the original cell line can form mineral in culture upon growth in the presence of differentiating agents. Such subclones include MC3T3-E1-BF (Smith et al. "Glucocorticoids inhibit developmental stage-specific osteoblast cell cycle." J. Biol. Chem., 275:19992-20001 (2000)). For both the MC3T3-E1-BF subclone as well as the original MC3T3-E1 cells, sclerostin can inhibit one or more of the sequence of events leading up to and including mineral deposition (*i.e*., sclerostin inhibits mineralization). Anti-sclerostin antibodies that are able to neutralize sclerostin's inhibitory activity allow for mineralization of the culture in the presence of sclerostin such that there is a statistically significant increase in deposition of calcium phosphate (measured as calcium) as compared to the amount of calcium measured in the sclerostin-only (i.e., no antibody) treatment group.

When running the assay with the goal of determining whether a particular anti-sclerostin antibody or anti-sclerostin binding agent can neutralize sclerostin (*i.e*., is a sclerostin neutralizing antibody or derivative thereof, or is a sclerostin neutralizing binding agent), the amount of sclerostin used in the assay can be the minimum amount of sclerostin that causes at least a 70%, statistically significant, reduction in deposition of calcium phosphate (measured as calcium) in the sclerostin-only group, as compared to the amount of calcium measured in the no sclerostin group. An anti-sclerostin neutralizing antibody or an anti-sclerostin neutralizing binding agent is defined as one that causes a statistically significant increase in deposition of calcium phosphate (measured as calcium) as compared to the amount of calcium measured in the sclerostin-only (i.e., no antibody, no binding agent) treatment group. To determine whether an anti-sclerostin antibody or an anti-sclerostin binding agent is neutralizing or not, the amount of anti-sclerostin antibody or anti-sclerostin binding agent used in the assay needs to be such that there is an excess of moles of sclerostin binding sites per well as compared to the number of moles of sclerostin per well. Depending on the potency of the antibody, the fold excess that may be required can be 24, 18, 12, 6, 3, or 1.5, and one of skill is familiar with the routine practice of testing more than one concentration of binding agent. For example, a very potent anti-sclerostin neutralizing antibody or anti-sclerostin neutralizing binding agent will be able to neutralize sclerostin even when there is less than a 6-fold excess of moles of sclerostin binding sites per well as compared to the number of moles of sclerostin per well. A less potent anti-sclerostin neutralizing antibody or anti-sclerostin neutralizing binding agent will be able to neutralize sclerostin only at a 12, 18, or 24 fold excess. Sclerostin binding agents within this full range of potencies are suitable as neutralizing sclerostin binding agents.

Anti-sclerostin antibodies and derivatives thereof that can neutralize human sclerostin, and sclerostin binding agents that can neutralize human sclerostin, may be of use in the treatment of human conditions/disorders that are caused by, associated with, or result in at least one of low bone formation, low bone mineral density, low bone mineral content, low bone mass, low bone quality and low bone strength.

### IN VIVO NEUTRALIZATION ASSAY

Increases in various parameters associated with, or that result from, the stimulation of new bone formation can be measured as an output from *in vivo* testing of sclerostin binding agents in order to identify those binding agents that are able to neutralize sclerostin and thus able to cause stimulation of new bone formation. Such parameters include various serum anabolic markers (*e.g*., osteocalcin and P1NP (n-terminal propeptide of type 1 procollagen)), histomorphometric markers of bone formation (*e.g*. osteoblast surface/bone surface; bone formation rate/bone surface; and trabecular thickness), bone mineral density, bone mineral content, bone mass, bone quality, and bone strength. A sclerostin neutralizing binding agent is defined as one capable of causing a statistically significant increase, as compared to vehicle treated animals, in any parameter associated with, or that results from, the stimulation of new bone formation. Such *in vivo* testing can be performed in any suitable mammal (*e.g*. mouse, rat, and/or monkey).

Although the amino acid sequence of sclerostin is not 100% identical across mammalian species (*e.g*., mouse sclerostin is not 100% identical to human sclerostin), it will be appreciated by one skilled in the art that a sclerostin binding agent that can neutralize, *in vivo,* the sclerostin of a certain species (*e.g*., mouse) and that also can bind human sclerostin *in vitro* is very likely to be able to neutralize human sclerostin *in vivo.* Thus, such a human sclerostin binding agent (*e.g*., anti-human sclerostin antibody) may be of use in the treatment of human conditions/disorders that are caused by, associated with, or result in at least one of low bone formation, low bone mineral density, low bone mineral content, low bone mass, low bone quality, and low bone strength. Mice in which homologous recombination had been used to delete the mouse sclerostin gene and insert the human sclerostin gene in its place (i.e., human sclerostin gene knock-in mice or human SOST knock-in mice) would be an example of an additional *in vivo* system.

Pharmaceutical compositions are provided, comprising one of the above-described binding agents such as at least one of antibody A-Y to human sclerostin, along with a pharmaceutically or physiologically acceptable carrier, excipient, or diluent. Pharmaceutical compositions and methods of treatment are disclosed in copending Application Serial No. 10/868,497, filed June 16, 2004, published as U.S. 2005/0106683, which claims priority to Serial No. 60/478,977, both of which are incorporated by reference herein.

The development of suitable dosing and treatment regimens for using the particular compositions described herein in a variety of treatment regimens, including e.g., subcutaneous, oral, parenteral, intravenous, intranasal, and intramuscular administration and formulation, is well known in the art, some of which are briefly discussed below for general purposes of illustration.

In certain applications, the pharmaceutical compositions disclosed herein may be delivered via oral administration to an animal. As such, these compositions may be formulated with an inert diluent or with an assimilable edible carrier, or they may be enclosed in hard- or soft-shell gelatin capsule, or they may be compressed into tablets, or they may be incorporated directly with the food of the diet.

In certain circumstances it will be desirable to deliver the pharmaceutical compositions disclosed herein subcutaneously, parenterally, intravenously, intramuscularly, or intraperitoneally. Such approaches are well known to the skilled artisan, some of which are further described, for example, in U.S. Patent No. 5,543,158; U.S. Patent No. 5,641,515; and U.S. Patent No. 5,399,363. In certain embodiments, solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations generally will contain a preservative to prevent the growth of microorganisms.

Illustrative pharmaceutical forms suitable for injectable use include sterile aqueous solutions or dispersions and sterile powders for the extemporaneous preparation of sterile injectable solutions or dispersions (for example, see U.S. Patent No. 5,466,468). In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (e.g., glycerol, propylene glycol, liquid polyethylene glycol, and the like), suitable mixtures thereof, and/or vegetable oils. Proper fluidity may be maintained, for example, by the use of a coating, such as lecithin, by the maintenance of the required particle size in the case of dispersion, and/or by the use of surfactants. The prevention of the action of microorganisms can be facilitated by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

In one embodiment, for parenteral administration in an aqueous solution, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, a sterile aqueous medium that can be employed will be known to those of skill in the art. For example, one dosage may be dissolved in 1 ml of isotonic NaCl solution and either added to 1000 ml of hypodermoclysis fluid or injected at the proposed site of infusion (see, for example, Remington's Pharmaceutical Sciences, 15th ed., pp. 1035-1038 and 1570-1580). Some variation in dosage will necessarily occur depending on the condition of the subject being treated. Moreover, for human administration, preparations will preferably meet sterility, pyrogenicity, and the general safety and purity standards as required by FDA Office of Biologics standards.

In another embodiment of the invention, the compositions disclosed herein may be formulated in a neutral or salt form. Illustrative pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like, Salts formed with the free carboxyl groups can also be derived from inorganic bases such as, for example, sodium, potassium, ammonium, calcium, or ferric hydroxides, and such organic bases as isopropylamine, trimethylamine, histidine, procaine, and the like. Upon formulation, solutions will be administered in a manner compatible with the dosage formulation and in such amount as is therapeutically effective.

The carriers can further comprise any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions. The phrase "pharmaceutically-acceptable" refers to molecular entities and compositions that do not produce an allergic or similar untoward reaction when administered to a human.

In certain embodiments, liposomes, nanocapsules, microparticles, lipid particles, vesicles, and the like, are used for the introduction of the compositions of the present invention into suitable host cells/organisms. In particular, the compositions of the present invention may be formulated for delivery either encapsulated in a lipid particle, a liposome, a vesicle, a nanosphere, or a nanoparticle or the like. Alternatively, compositions of the present invention can be bound, either covalently or non-covalently, to the surface of such carrier vehicles.

The formation and use of liposome and liposome-like preparations as potential drug carriers is generally known to those of skill in the art (see for example, Lasic, Trends Biotechnol., 16(7):307-21 (1998); Takakura, Nippon Rinsho, 56(3):691-95 (1998); Chandran et al., Indian J. Exp. Biol., 35(8):801-09 (1997); Margalit, Crit. Rev. Ther. Drug Carrier Syst., 12(2-3):233-61 (1995); U.S. Patent No. 5,567,434; U.S. Patent No. 5,552,157; U.S. Patent No. 5,565,213; U.S. Patent No. 5,738,868, and U.S. Patent No. 5,795,587, each specifically incorporated herein by reference in its entirety). The use of liposomes does not appear to be associated with autoimmune responses or unacceptable toxicity after systemic delivery. In certain embodiments, liposomes are formed from phospholipids that are dispersed in an aqueous medium and spontaneously form multilamellar concentric bilayer vesicles (also termed multilamellar vesicles (MLVs)).

Alternatively, in other embodiments, the invention provides for pharmaceutically-acceptable nanocapsule formulations of the compositions of the present invention. Nanocapsules can generally entrap compounds in a stable and reproducible way (see, for example, Quintanar-Guerrero et al., Drug Dev. Ind. Pharm., 24(12):1113-28 (1998)). To avoid side effects due to intracellular polymeric overloading, such ultrafine particles (sized around 0.1µm) may be designed using polymers able to be degraded *in vivo.* Such particles can be made as described, for example, by Couvreur et al., Crit. Rev. Ther. Drug Carrier Syst., 5(1):1-20 (1988); zur Muhlen et al., Eur. J. Pharm. Biopharm., 45(2):149-55 (1998); Zambaux et al., J. Controlled Release, 50(1-3):31-40 (1998); and U.S. Patent No. 5,145,684.

In addition, pharmaceutical compositions of the present invention may be placed within containers, along with packaging material that provides instructions regarding the use of such pharmaceutical compositions. Generally, such instructions will include a tangible expression describing the reagent concentration, as well as within certain embodiments, relative amounts of excipient ingredients or diluents (e.g., water, saline or PBS) that may be necessary to reconstitute the pharmaceutical composition.

The dose administered may range from 0,01 mg/kg to 100 mg/kg of body weight. As will be evident to one of skill in the art, the amount and frequency of administration will depend, of course, on such factors as the nature and severity of the indication being treated, the desired response, the condition of the patient, and so forth. Typically, the compositions may be administered by a variety of techniques, as noted above.

Increases in bone mineral content and/or bone mineral density may be determined directly through the use of X-rays (e.g., Dual Energy X-ray Absorptometry or "DEXA"), or by inference through the measurement of (1) markers of bone formation and/or osteoblast activity, such as, but not limited to, osteoblast specific alkaline phosphatase, osteocalcin, type 1 procollagen C' propeptide (PICP), total alkaline phosphatase (*see* Comier, Curr. Opin, in Rheu., 7:243 (1995)) and serum procollagen 1 N-terminal propeptide (P1NP) and/or (2) markers of bone resorption and/or osteoclast activity including, but not limited to, pyridinoline, deoxypryridinoline, N-telopeptide, urinary hydroxyproline, plasma tartrate-resistant acid phosphatases, and galactosyl hydroxylysine (*see* Comier, *id*), serum TRAP 5b (tartrate-resistant acid phosphatase isoform 5b) and serum cross-linked C-telopeptide (sCTXI). The amount of bone mass may also be calculated from body weights or by using other methods (see Guinness-Hey, Metab. Bone Dis. Relat. Res., 5:177-181 (1984)). Animals and particular animal models are used in the art for testing the effect of the compositions and methods of the invention on, for example, parameters of bone loss, bone resorption, bone formation, bone strength or bone mineralization that mimic conditions of human disease such as osteoporosis and osteopenias. Examples of such models include the ovariectomized rat model (Kalu, D.N., "The ovariectomized rat model of postmenopausal bone loss." Borce and Mineral, 15:175-192 (1991); Frost, H.M. and Jee, W.S.S., "On the rat model of human osteopenias and osteoporosis." Bone and Mineral, 18:227-236 (1992); and Jee, W.S.S. and Yao, W., "Overview: animal models of osteopenia and osteoporosis." J. Musculoskel. Neuron. Interact., 1:193-207 (2001)).

Particular conditions which may be treated by the compositions of the present invention include dysplasias, wherein growth or development of bone is abnormal and a wide variety of causes of osteopenia, osteoporosis, and bone loss, Representative examples of such conditions include achondroplasia, cleidocranial dysostosis, enchondromatosis, fibrous dysplasia, Gaucher's Disease, hypophosphatemic rickets, Marfan's syndrome, multiple hereditary exotoses, neurofibromatosis, osteogenesis imperfecta, osteopetrosis, osteopoikilosis, sclerotic lesions, pseudoarthrosis, and pyogenic osteomyelitis, periodontal disease, anti-epileptic drug induced bone loss, primary and secondary hyperparathyroidism, familial hyperparathyroidism syndromes, weightlessness induced bone loss, osteoporosis (e.g., osteoporosis in men), postmenopausal bone loss, osteoarthritis, renal osteodystrophy, infiltrative disorders of bone, oral bone loss, osteonecrosis of the jaw, juvenile Paget's disease, melorheostosis, metabolic bone diseases, mastocytosis, sickle cell anemia/disease, organ transplant related bone loss, kidney transplant related bone loss, systemic lupus erythematosus, ankylosing spondylitis, epilepsy, juvenile arthritides, thalassemia, mucopolysaccharidoses, fabry disease, Turner syndrome, Down Syndrome, Klinefelter Syndrome, leprosy, Perthes' Disease, adolescent idiopathic scoliosis, infantile onset multi-system inflammatory disease, Winchester Syndrome, Menkes Disease, Wilson's Disease, ischemic bone disease (such as Legg-Calve-Perthes disease, regional migratory osteoporosis), anemic states, conditions caused by steroids, glucocorticoid-induced bone loss, heparin-induced bone loss, bone marrow disorders, scurvy, malnutrition, calcium deficiency, idiopathic osteopenia or osteoporosis, congenital osteopenia or osteoporosis, alcoholism, chronic liver disease, postmenopausal state, chronic inflammatory conditions, rheumatoid arthritis, inflammatory bowel disease, ulcerative colitis, inflammatory colitis, Crohn's disease, oligomenorrhea, amenorrhea, pregnancy, diabetes mellitus, hyperthyroidism, thyroid disorders, parathyroid disorders, Cushing's disease, acromegaly, hypogonadism, immobilization or disuse, reflex sympathetic dystrophy syndrome, regional osteoporosis, osteomalacia, bone loss associated with joint replacement, HIV associated bone loss, bone loss associated with loss of growth hormone, bone loss associated with cystic fibrosis, fibrous dysplasia, chemotherapy associated bone loss, tumor induced bone loss, cancer-related bone loss, hormone ablative bone loss, multiple myeloma, drug-induced bone loss, anorexia nervosa, disease associated facial bone loss, disease associated cranial bone loss, disease associated bone loss of the jaw, disease associated bone loss of the skull, and bone loss associated with space travel. Further conditions relate to bone loss associated with aging, including facial bone loss associated with aging, cranial bone loss associated with aging, jaw bone loss associated with aging, and skull bone loss associated with aging.

Compositions of the present invention may also be useful for improving outcomes in orthopedic procedures, dental procedures, implant surgery, joint replacement, bone grafting, bone cosmetic surgery and bone repair such as fracture healing, nonunion healing, delayed union healing and facial reconstruction. One or more compositions may be administered before, during and/or after the procedure, replacement, graft, surgery or repair.

The invention also provides a diagnostic kit comprising at least one anti-sclerostin binding agent according to the present invention, The binding agent may be an antibody. In addition, such a kit may optionally comprise one or more of the following:
(1) instructions for using the one or more binding agent(s) for screening, diagnosis, prognosis, therapeutic monitoring or any combination of these applications;
(2) a labeled binding partner to the anti-sclerostin binding agent(s);
(3) a solid phase (such as a reagent strip) upon which the anti-sclerostin binding agent(s) is immobilized; and
(4) a label or insert indicating regulatory approval for screening, diagnostic, prognostic or therapeutic use or any combination thereof.
If no labeled binding partner to the binding agent(s) is provided, the binding agent(s) itself can be labeled with one or more of a detectable marker(s), e.g., a chemiluminescent, enzymatic, fluorescent, or radioactive moiety.

The following examples are offered by way of illustration, and not by way of limitation.

### EXAMPLES

### EXAMPLE 1

### RECOMBINANT EXPRESSION OF SCLEROSTIN

Recombinant human sclerostin/SOST is commercially available from R&D Systems (Minneapolis, MN, USA; 2006 cat# 1406-ST-025). Additionally, recombinant mouse sclerostin/SOST is commercially available from R&D Systems (Minneapolis, MN, USA; 2006 cat# 1589-ST-025).

Alternatively, the different species of sclerostin can be expressed transiently in serum-free suspension adapted 293T or 293EBNA cells. Transfections can be performed as 500 mL or 1L cultures. The following reagents and materials are available from Gibco BRL (now Invitrogen, Carlsbad, CA). Catalog numbers are listed in parentheses: serum-free DMEM (21068-028); DMEM/F12 (3:1) (21068/11765); 1X Insulin-Transferrin-Selenium Supplement (51500-056); 1X Pen Strep Glut (10378-016); 2mM 1-Glutamine (25030-081); 20 mM HEPES (15630-080); 0.01% Pluronic F68 (24040-032). Briefly, the cell inoculum (5.0-10.0 X 10⁵ cells/mL X culture volume) is centrifuged at 2,500 RPM for 10 minutes at 4°C to remove the conditioned medium.

The cells are resuspended in serum-free DMEM and centrifuged again at 2,500 RPM for 10 minutes at 4°C. After aspirating the wash solution, the cells are resuspended in growth medium [DMEM/F12 (3:1) + 1X Insulin-Transferrin-Selenium Supplement + 1X Pen Strep Glut + 2mM L-Glutamine + 20 mM HEPES + 0.01% Pluronic F68] in a 1L or 3L spinner flask culture. The spinner flask culture is maintained on magnetic stir plate at 125 RPM which is placed in a humidified incubator maintained at 37°C and 5% CO₂. The mammalian expression plasmid DNA (*e.g*., pcDNA3.1, pCEP4, Invitrogen Life Technologies, Carlsbad, CA), containing the complete coding region (and stop codon) of sclerostin with a Kozak consensus sequence (*e.g*., CCACC) directly 5' of the start site ATG, is complexed to the transfection reagent in a 50 mL conical tube,

The DNA-transfection reagent complex can be prepared in 5-10% of the final culture volume in serum-free DMEM or OPTI-MEM. The transfection reagents that can be used for this purpose include X-tremeGene RO-1539 (Roche Applied Science, Indianapolis, IN), FuGene6 (Roche Applied Science, Indianapolis, IN), Lipofectamine 2000 (Invitrogen, Carlsbad, CA), and 293fectin^{™} (Invitrogen, Carlsbad, CA). 1-5 µg plasmid DNA/mL culture is first added to serum-free DMEM, followed by 1-5 µl transfection reagent/mL culture. The complexes can be incubated at room temperature for approximately 10-30 minutes and then added to the cells in the spinner flask. The transfection/expression can be performed for 4-7 days, after which the conditioned medium (CM) is harvested by centrifugation at 4,000 RPM for 60 minutes at 4°C.

### EXAMPLE 2

### PURIFICATION OF RECOMBINANT SCLEROSTIN

Recombinant sclerostin was purified from mammalian host cells as follows. All purification processes were carried out at room temperature. One purification scheme was used to purify various species of sclerostin, including murine and human sclerostin. The purification scheme used affinity chromatography followed by cation exchange chromatography.

### Heparin Chromatography

The mammalian host cell conditioned medium (CM) was centrifuged in a Beckman J6-M1 centrifuge at 4000 rpm for 1 hour at 4°C to remove cell debris. The CM supernatant was then filtered through a sterile 0.2 µm filter. (At this point the sterile filtered CM may be optionally stored frozen until purification.) If the CM was frozen, it was thawed at the following temperatures, or combination thereof: 4°C, room temperature or warm water. Following thawing, the CM was filtered through a sterile 0.2 µm filter and optionally concentrated by tangential flow ultrafiltration (TFF) using a 10 kD molecular weight cut-off membrane. The CM concentrate was filtered through a sterile 0.2 µm filter and then loaded onto a Heparin High Performance (Heparin HP) column (GE Healthcare, formerly Amersham Biosciences) equilibrated in PBS. Alternatively, the filtered CM supernatant may be loaded directly onto the Heparin HP column equilibrated in PBS.

After loading, the Heparin HP column was washed with PBS until the absorbance at 280 nm of the flow-through returned to baseline (i.e., absorbance measured before loading CM supernatant). The sclerostin was then eluted from the column using a linear gradient from 150 mM to 2M sodium chloride in PBS. The absorbance at 280 nm of the eluate was monitored and fractions containing protein were collected. The fractions were then assayed by Coomassie-stained SDS-PAGE to identify fractions containing a polypeptide that migrates at the size of glycosylated sclerostin. The appropriate fractions from the column were combined to make the Heparin HP pool.

### Cation Exchange Chromatography

The sclerostin eluted from the Heparin HP column was further purified by cation exchange chromatography using SP High Performance (SPHP) chromatography media (GE Healthcare, formerly Amersham Biosciences). The Heparin HP pool was buffer exchanged into PBS by dialysis using 10,000 MWCO membranes (Pierce Slide-A-Lyzer). The dialyzed Heparin HP pool was then loaded onto an SPHP column equilibrated in PBS. After loading, the column was washed with PBS until the absorbance at 280 nm of the flow-through returned to baseline. The sclerostin was then eluted from the SPHP column using a linear gradient from 150 mM to 1 M sodium chloride in PBS. The absorbance at 280 nm of the eluate was monitored and the eluted sclerostin was collected in fractions. The fractions were then assayed by Coomassie-stained SDS-PAGE to identify fractions containing a polypeptide that migrates at the size of glycosylated sclerostin. The appropriate fractions from the column were combined to make the SPHP pool.

### Formulation

Following purification, the SPHP pool was formulated in PBS by dialysis using 10,000 MWCO membranes (Pierce Slide-A-Lyzer). If concentration of sclerostin was necessary, a centrifugal device (Amicon Centricon or Centriprep) with a 10,000 MWCO membrane was used. Following formulation the sclerostin was filtered through a sterile 0.2 µm filter and stored at 4°C or frozen.

### EXAMPLE 3

### ELISA-BASED CROSS-BLOCKING ASSAY

An antibody is coated on an ELISA plate at 2 µg/ml. While plates are blocking, the test antibody is incubated with human sclerostin at a final concentration of 25 ng/ml for one hour at room temperature in a separate plate. This complex is then transferred to the blocked ELISA plate and incubated for a further one hour at room temperature. Plates are washed and a pool of biotinylated anti-sclerostin antibodies at 1 ug/ml is then added and incubated for one hour at room temperature. Plates are then washed and streptavidin-horseradish peroxidase conjugate added at a 1:5000 dilution. Plates are developed with TMB. Blocking antibodies are able to reduce the ELISA signal due to inhibition of sclerostin binding to the coated antibodies. Positive crossblocking wells are considered to be those wells which decrease the signal by at least 40%.

### EXAMPLE 4

### ELISA-BASED CROSS-BLOCKING ASSAY

Liquid volumes used in this example would be those typically used in 96-well plate ELISAs (e.g. 50-200 µl/well). Ab-X and Ab-Y, in this example are assumed to have molecular weights of about 145 Kd and to have 2 sclerostin binding sites per antibody molecule. An anti-sclerostin antibody (Ab-X) is coated (e.g. 50µ of 1 µg/ml) onto a 96-well ELISA plate [e.g. Coming 96 Well EIA/RIA Flat Bottom Microplate (Product # 3590), Corning Inc., Acton, MA] for at least one hour. After this coating step the antibody solution is removed, the plate is washed once or twice with wash solution (e.g., PBS and 0.05% Tween 20) and is then blocked using an appropriate blocking solution (e.g., PBS, 1% BSA, 1% goat serum and 0.5% Tween 20) and procedures known in the art. Blocking solution is then removed from the ELISA plate and a second anti-sclerostin antibody (Ab-Y), which is being tested for it's ability to cross-block the coated antibody, is added in excess (e.g. 50µl of 10µg/ml) in blocking solution to the appropriate wells of the ELISA plate. Following this, a limited amount (e.g. 50µl of 10 ng/ml) of sclerostin in blocking solution is then added to the appropriate wells and the plate is incubated for at least one hour at room temperature while shaking. The plate is then washed 2-4 times with wash solution, An appropriate amount of a sclerostin detection reagent [e.g., biotinylated anti-sclerostin polyclonal antibody that has been pre-complexed with an appropriate amount of a streptavidin-horseradish peroxidase (HRP) conjugate] in blocking solution is added to the ELISA plate and incubated for at least one hour at room temperature. The plate is then washed at least 4 times with wash solution and is developed with an appropriate reagent [e.g. HRP substrates such as TMB (colorimetric) or various HRP luminescent substrates]. The background signal for the assay is defined as the signal obtained in wells with the coated antibody (in this case Ab-X), second solution phase antibody (in this case Ab-Y), sclerostin buffer only (i.e. no sclerostin) and sclerostin detection reagents. The positive control signal for the assay is defined as the signal obtained in wells with the coated antibody (in this case Ab-X), second solution phase antibody buffer only (i.e, no second solution phase antibody), sclerostin and sclerostin detection reagents. The ELISA assay needs to be run in such a manner so as to have the positive control signal be at least 6 times the background signal.

To avoid any artifacts (e.g. significantly different affinities between Ab-X and Ab-Y for sclerostin) resulting from the choice of which antibody to use as the coating antibody and which to use as the second (competitor) antibody, the cross-blocking assay needs to be run in two formats:
1) format 1 is where Ab-X is the antibody that is coated onto the ELISA plate and Ab-Y is the competitor antibody that is in solution
   and
2) format 2 is where Ab-Y is the antibody that is coated onto the ELISA plate and Ab-X is the competitor antibody that is in solution.

Ab-X and Ab-Y are defined as cross-blocking if, either in format 1 or in format 2, the solution phase anti-sclerostin antibody is able to cause a reduction of between 60% and 100%, specifically between 70% and 100%, and more specifically between 80% and 100%, of the sclerostin detection signal (i.e. the amount of sclerostin bound by the coated antibody) as compared to the sclerostin detection signal obtained in the absence of the solution phase anti-sclerostin antibody (i.e. the positive control wells).

In the event that a tagged version of sclerostin is used in the ELISA, such as a N-terminal His-tagged Sclerostin (R&D Systems, Minneapolis, MN, USA; 2005 cat# 1406-ST-025) then an appropriate type of sclerostin detection reagent would include an HRP labeled anti-His antibody. In addition to using N-terminal His-tagged Sclerostin, one could also use C-terminal His-tagged Sclerostin. Furthermore, various other tags and tag binding protein combinations that are known in the art could be used in this ELISA-based cross-blocking assay (e.g., HA tag with anti-HA antibodies; FLAG tag with anti-FLAG antibodies; biotin tag with streptavidin).

From the foregoing, although specific embodiments of the invention have been described herein for purposes of illustration, various modifications may be made without deviating from the spirit and scope of the invention. Accordingly, the invention is not limited except as by the appended claims. All publications, published patent applications, and patent documents disclosed herein are hereby incorporated by reference.

### ASPECTS OF THE DISCLOSURE

(1) An isolated antibody selected from the group consisting of Antibody A - Antibody Y.
(2) A polypeptide comprising one or more complementarity determining regions of Antibody A, wherein the peptide comprises a binding affinity for human sclerostin of less than or equal to 1x10⁻⁷ M.
(3) A polypeptide comprising one or more complementarity determining regions of Antibody B, wherein the peptide comprises a binding affinity for human sclerostin of less than or equal to 1 x 10⁻⁷ M.
(4) A polypeptide comprising one or more complementarity determining regions of Antibody C, wherein the peptide comprises a binding affinity for human sclerostin of less than or equal to 1 x 10⁻⁷ M.
(5) A polypeptide comprising one or more complementarity determining regions of Antibody D, wherein the peptide comprises a binding affinity for human sclerostin of less than or equal to 1 x 10⁻⁷ M.
(6) A polypeptide comprising one or more complementarity determining regions of Antibody E, wherein the peptide comprises a binding affinity for human sclerostin of less than or equal to 1 x 10⁻⁷ M.
(7) A polypeptide comprising one or more complementarity determining regions of Antibody F, wherein the peptide comprises a binding affinity for human sclerostin of less than or equal to 1 x 10⁻⁷ M.
(8) A polypeptide comprising one or more complementarity determining regions of Antibody G, wherein the peptide comprises a binding affinity for human sclerostin of less than or equal to 1 x 10⁻⁷ M.
(9) A polypeptide comprising one or more complementarity determining regions of Antibody H, wherein the peptide comprises a binding affinity for human sclerostin of less than or equal to 1 x 10⁻⁷ M.
(10) A polypeptide comprising one or more complementarity determining regions of Antibody I, wherein the peptide comprises a binding affinity for human sclerostin of less than or equal to 1 x 10⁻⁷ M.
(11) A polypeptide comprising one or more complementarity determining regions of Antibody J, wherein the peptide comprises a binding affinity for human sclerostin of less than or equal to 1 x 10⁻⁷ M.
(12) A polypeptide comprising one or more complementarity determining regions of Antibody K, wherein the peptide comprises a binding affinity for human sclerostin of less than or equal to 1 x 10⁻⁷ M.
(13) A polypeptide comprising one or more complementarity determining regions of Antibody L, wherein the peptide comprises a binding affinity for human sclerostin of less than or equal to 1 x 10⁻⁷ M.
(14) A polypeptide comprising one or more complementarity determining regions of Antibody M, wherein the peptide comprises a binding affinity for human sclerostin of less than or equal to 1 x 10⁻⁷ M.
(15) A polypeptide comprising one or more complementarity determining regions of Antibody N, wherein the peptide comprises a binding affinity for human sclerostin of less than or equal to 1 x 10⁻⁷ M.
(16) A polypeptide comprising one or more complementarity determining regions of Antibody O, wherein the peptide comprises a binding affinity for human sclerostin of less than or equal to 1 x 10⁻⁷ M.
(17) A polypeptide comprising one or more complementarity determining regions of Antibody P, wherein the peptide comprises a binding affinity for human sclerostin of less than or equal to 1 x 10⁻⁷ M.
(18) A polypeptide comprising one or more complementarity determining regions of Antibody Q, wherein the peptide comprises a binding affinity for human sclerostin of less than or equal to 1 x 10⁻⁷ M.
(19) A polypeptide comprising one or more complementarity determining regions of Antibody R, wherein the peptide comprises a binding affinity for human sclerostin of less than or equal to 1 x 10⁻⁷ M.
(20) A polypeptide comprising one or more complementarity determining regions of Antibody S, wherein the peptide comprises a binding affinity for human sclerostin of less than or equal to 1 x 10⁻⁷ M.
(21) A polypeptide comprising one or more complementarity determining regions of Antibody T, wherein the peptide comprises a binding affinity for human sclerostin of less than or equal to 1 x 10⁻⁷ M.
(22) A polypeptide comprising one or more complementarity determining regions of Antibody U, wherein the peptide comprises a binding affinity for human sclerostin of less than or equal to 1 x 10⁻⁷ M.
(23) A polypeptide comprising one or more complementarity determining regions of Antibody V, wherein the peptide comprises a binding affinity for human sclerostin of less than or equal to 1 x 10⁻⁷ M.
(24) A polypeptide comprising one or more complementarity determining regions of Antibody W, wherein the peptide comprises a binding affinity for human sclerostin of less than or equal to 1 x 10⁻⁷ M.
(25) A polypeptide comprising one or more complementarity determining regions of Antibody X, wherein the peptide comprises a binding affinity for human sclerostin of less than or equal to 1 x 10⁻⁷ M.
(26) A polypeptide comprising one or more complementarity determining regions of Antibody Y, wherein the peptide comprises a binding affinity for human sclerostin of less than or equal to 1 x 10⁻⁷ M.
(27) The polypeptide of any of (2) to (26), wherein the peptide comprises a binding affinity for human sclerostin of less than or equal to 1 x 10⁻⁸ M.
(28) The polypeptide of any of (2) to (26), wherein the peptide comprises a binding affinity for human sclerostin of less than or equal to 1 x 10⁻⁹ M.
(29) The polypeptide of any of (2) to (26), wherein the peptide comprises a binding affinity for human sclerostin of less than or equal to 1 x 10⁻¹ M.
(30) The polypeptide of any of (2) to (29), wherein the peptide cross- blocks binding of any of Antibodies AA-WW to human sclerostin.
(31) The polypeptide of any of (2) to (30), wherein the polypeptide comprises one or more amino acid sequences selected from the group consisting of SEQ ID NO: 109, 110, 111, 115, 116, 117, 121, 122, 123, 127, 128, 129, 133, 134, 135, 139, 140, 141, 145, 146, 147, 151, 152, 153, 157, 158, 159, 163, 164, 165, 169, 170, 171, 175, 176, 177, 181, 182, 183, 187, 188, 189, 193, 194, 195, 199, 200, 201, 205, 206, 211, 212, 213, 217, 218, 219, 223, 224, 225, 229, 230, 231, 235, 236, 237, 241, 242, 243, 247, 248, 249, 253, 254, and 255
(32) An isolated nucleic acid molecule comprising a polynucleotide encoding the polypeptide of any of (2) to (31),
(33) A vector comprising the isolated nucleic acid molecule of (32).
(34) A host cell comprising the vector of (33).
(35) A method of making a protein comprising culturing a host cell of claim under conditions wherein the encoded protein is expressed.

## Claims

1. An isolated antibody selected from the group consisting of Antibody O, Antibody B - Antibody N, Antibody P - Antibody Y and Antibody A.

2. The antibody of claim 1, which is a monoclonal, preferably where the monoclonal is a human monoclonal, humanized monoclonal or a chimeric monoclonal.

3. The antibody of claim 1 or 2 which is an antibody fragment selected from a F(ab')₂, Fab, Fab' and Fv fragment.

4. A polypeptide comprising one or more complementarity determining regions of Antibody O, Antibody B - Antibody N, Antibody P - Antibody Y or Antibody A, wherein the peptide comprises a binding affinity for human sclerostin of less than or equal to 1 x 10⁻⁷ M.

5. The polypeptide of claim 4 wherein the peptide comprises a binding affinity for human sclerostin of less than or equal to 1 x 10⁻⁸ M, less than or equal to 1 x 10⁻⁹ M, or less than or equal to 1 x 10⁻¹⁰ M.

6. The polypeptide of claim 4 or 5, wherein the peptide cross-blocks binding of any of Antibodies O, B-N, P-Y and A to human sclerostin.

7. The polypeptide of any of claims 4 to 6, wherein the polypeptide comprises one or more amino acid sequences selected from the group consisting of SEQ ID NO: 109, 110, 111, 115, 116, 117, 121, 122, 123, 127, 128, 129, 133, 134, 135, 139, 140, 141, 145, 146, 147, 151, 152, 153, 157, 158, 159, 163, 164, 165, 169, 170, 171, 175, 176, 177, 181, 182, 183, 187, 188, 189, 193, 194, 195, 199, 200, 201, 205, 206, 211, 212, 213, 217, 218, 219, 223, 224, 225, 229, 230, 231, 235, 236, 237, 241, 242, 243, 247, 248, 249, 253, 254, and 255.

8. A polypeptide comprising the amino acid sequence of SEQ ID No: 60 and/or 62.

9. An isolated nucleic acid molecule comprising a polynucleotide encoding the polypeptide of any one of claims 4 to 8.

10. A vector comprising the isolated nucleic acid molecule of claim 9.

11. A host cell comprising the vector of claim 10.

12. A method of making a protein comprising culturing a host cell of claim 11 I under conditions wherein the encoded protein is expressed.

13. A pharmaceutical composition comprising an antibody according to any one of claims 1 to 3 or a polypeptide according to any one of claims 4 to 8 along with a pharmaceutically or physiologically acceptable carrier, excipient or diluent.

14. An antibody according to any one of claims 1 to 3 or a polypeptide according to any one of claims 4 to 8 for use in a method of bone repair or a method of treating a dysplasia or a cause of osteopenia, osteoporosis or bone loss.

15. The antibody or polypeptide of claim 14 for use in:
(a) treating osteopenia or osteoporosis;
(b) treating achondroplasia, cleidocranial dysostosis, enchondromatosis, fibrous dysplasia, Gaucher's Disease, hypophosphatemic rickets, Marfan's syndrome, multiple hereditary exotoses, neurofibromatosis, osteogenesis imperfecta, osteopetrosis, osteopoikilosis, sclerotic lesions, pseudoarthrosis, and pyogenic osteomyelitis, periodontal disease, anti-epileptic drug induced bone loss, primary and secondary hyperparathyroidism, familial hyperparathyroidism syndromes, weightlessness induced bone loss, osteoporosis in men, postmenopausal bone loss, osteoarthritis, renal osteodystrophy, infiltrative disorders of bone, oral bone loss, osteonecrosis of the jaw, juvenile Paget's disease, melorheostosis, metabolic bone diseases, mastocytosis, sickle cell anemia/disease, organ transplant related bone loss, kidney transplant related bone loss, systemic lupus erythematosus, ankylosing spondylitis, epilepsy, juvenile arthritides, thalassemia, mucopolysaccharidoses, fabry disease, Turner syndrome, Down Syndrome, Klinefelter Syndrome, leprosy, Perthes' Disease, adolescent idiopathic scoliosis, infantile onset multi-system inflammatory disease, Winchester Syndrome, Menkes Disease, Wilson's Disease, ischemic bone disease (such as Legg-Calve-Perthes disease, regional migratory osteoporosis), anemic states, conditions caused by steroids, glucocorticoid-induced bone loss, heparin-induced bone loss, bone marrow disorders, scurvy, malnutrition, calcium deficiency, idiopathic osteopenia or osteoporosis, congenital osteopenia or osteoporosis, alcoholism, chronic liver disease, postmenopausal state, chronic inflammatory conditions, rheumatoid arthritis, inflammatory bowel disease, ulcerative colitis, inflammatory colitis, Crown' disease, oligomenorrhea, amenorrhea, pregnancy, diabetes mellitus, hyperthyroidism, thyroid disorders, parathyroid disorders, Cushing's disease, acromegaly, hypogonadism, immobilization or disuse, reflex sympathetic dystrophy syndrome, regional osteoporosis, osteomalacia, bone loss associated with joint replacement, HIV associated bone loss, bone loss associated with loss of growth hormone, bone loss associated with cystic fibrosis, fibrous dysplasia, chemotherapy associated bone loss, tumor induced bone loss, cancer-related bone loss, hormone ablative bone loss, multiple myeloma, drug-induced bone loss, anorexia nervosa, disease associated facial bone loss, disease associated cranial bone loss, disease associated bone loss of the jaw, disease associated bone loss of the skull, and bone loss associated with space travel, conditions related to bone loss associated with aging, including facial bone loss associated with aging, cranial bone loss associated with aging, jaw bone loss associated with aging, and skull bone loss associated with aging; or
(c) improving outcomes in orthopedic procedures, dental procedures, implant surgery, joint replacement, bone grafting, bone cosmetic surgery and bone repair such as fracture healing, nonunion healing, delayed union healing and facial reconstruction,
